(19) Europäisches Patentamt
European Patent Office
Office européen des brevets

(11) EP 2 797 950 B1

(12) # EUROPEAN PATENT SPECIFICATION

(45) Date of publication and mention of the grant of the patent:
**18.10.2017 Bulletin 2017/42**

(21) Application number: **12824731.9**

(22) Date of filing: **22.12.2012**

(51) Int Cl.:
***C07K 14/525*** (2006.01) ***C07K 14/705*** (2006.01)
*C07K 14/005* (2006.01) *C07K 14/195* (2006.01)
*C07K 14/32* (2006.01) *C07K 14/435* (2006.01)
*C07K 14/46* (2006.01) *C07K 14/47* (2006.01)
*C07K 7/08* (2006.01) *C12N 7/00* (2006.01)
*C12N 9/16* (2006.01)

(86) International application number:
**PCT/IB2012/057657**

(87) International publication number:
**WO 2013/098755 (04.07.2013 Gazette 2013/27)**

(54) **ANTICANCER FUSION PROTEIN**
ANTIKREBS-FUSIONSPROTEIN
PROTÉINE HYBRIDE ANTICANCÉREUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.12.2011 PL 39759511**

(43) Date of publication of application:
**05.11.2014 Bulletin 2014/45**

(73) Proprietor: **Adamed sp. z o.o.**
**05-152 Czosnów k/Warszawy (PL)**

(72) Inventors:
- **PIECZYKOLAN, Jerzy Szczepan**
**PL-22-463 Radecznica (PL)**
- **PAWLAK, Sebastian Dominik**
**PL-01-420 Warszawa (PL)**
- **ZEREK, Bartlomiej Maciej**
**PL-97-200 Dabrowa (PL)**
- **RÓZGA, Piotr Kamil**
**PL-96-100 Skierniewice (PL)**

(74) Representative: **Sitkowska, Jadwiga**
**Kancelaria Patentowa**
**Al. Komisji Edukacji Narodowej 83/106**
**02-777 Warszawa (PL)**

(56) References cited:
**WO-A1-2009/094634 CN-A- 1 546 528**

- **SHIN J N ET AL: "Generation of a novel proform of tumor necrosis factor-related apoptosis-inducing ligand (TRAIL) protein that can be reactivated by matrix metalloproteinases", EXPERIMENTAL CELL RESEARCH, ACADEMIC PRESS, US, vol. 312, no. 19, 15 November 2006 (2006-11-15), pages 3892-3898, XP024945270, ISSN: 0014-4827, DOI: 10.1016/J.YEXCR.2006.08.015 [retrieved on 2006-11-15]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 797 950 B1

## Description

The invention relates to the field of therapeutic fusion proteins, especially recombinant fusion proteins. More particularly, the invention relates to fusion proteins comprising the fragment of a sequence of the soluble human TRAIL protein and a sequence of a peptide forming pores in the cell or mitochondrial membrane, pharmaceutical compositions containing them, and their use in therapy, especially as anticancer agents.

TRAIL (Tumor Necrosis Factor- Related Apoptosis Inducing Ligand) protein, a member of the cytokines family, also known as Apo2L (Apo2-ligand), is a potent activator of apoptosis in tumor cells and in cells infected by viruses. TRAIL is a ligand naturally occurring in the body. TRAIL protein, its amino acid sequence, coding DNA sequence and protein expression systems were disclosed for the first time in EP0835305A1.

TRAIL protein exerts its anticancer activity by binding to pro-apoptotic surface TRAIL receptors 1 and 2 (TRAIL-R1/R2) and subsequent activation of these receptors. These receptors, also known as DR4 and DR5 (death receptor 4 and death receptor 5), are members of the TNF receptor family and are overexpressed by different types of cancer cells. Activation of these receptors can induce external signaling pathway of suppressor gene p53-independent apoptosis, which by activated caspase-8 leads to the activation of executive caspases and thereby degradation of nucleic acids. Caspase-8 released upon TRAIL activation may also cause the release of truncated Bid protein, which is translocated to mitochondria, where it stimulates the release of cytochrome c, thus indirectly amplifying the apoptotic signal from death receptors.

TRAIL acts selectively on tumor cells, essentially without inducing apoptosis in healthy cells which show resistance to this protein. Therefore, the enormous potential of TRAIL was recognized as an anticancer agent which acts on a wide range of different types of cancers, including hematologic malignancies and solid tumors, while sparing normal cells and exerting potentially relatively little side effects.

TRAIL protein is a type II membrane protein having the length of 281 amino acids, and its extracellular region comprising amino acid residues 114-281 upon cleavage by proteases forms soluble sTRAIL molecule of 20 kDa size, which is also biologically active. Both forms, TRAIL and sTRAIL, are capable of triggering apoptosis via interaction with TRAIL receptors present on target cells. Strong antitumor activity and very low systemic toxicity of soluble part of TRAIL molecule was demonstrated using cell lines tests. Also, preliminary human clinical studies with recombinant human soluble TRAIL (rhTRAIL) having amino acid sequence corresponding to amino acids 114-281 of hTRAIL, known under the INN dulanermin, showed its good tolerance and absence of dose limiting toxicity. Toxic effects of recombinant TRAIL protein on liver cells reported up to now appear to be associated with the presence of modification, i.e. polyhistidine tags, while untagged TRAIL showed no systemic toxicity.

Fragments of TRAIL shorter than 114-281 are also able to bind with membrane death receptors and induce apoptosis via these receptors, for example, as recently reported in EP 1 688 498 for recombinant circularly permuted mutant of 122-281 hTRAIL.

However, in further clinical trials on patients the actual effectiveness of TRAIL as a monotherapy proved to be low. Also problematic was primary or acquired resistance to TRAIL shown by many cancer cells (see for example WO2007/022214). Resistance may be due to various mechanisms and may be specific for a cancer type and/or patient-dependent (Thorburn A, Behbakht K, Ford H. TRAIL receptor-targeted therapeutics: resistance mechanisms and strategies to avoid them. Drug Resist Updat 2008; 11: 17-24). This resistance limits the usefulness of TRAIL as an anticancer agent. Although the mechanism of resistance to TRAIL has not been fully understood, it is believed that it may manifest itself at different levels of TRAIL-induced apoptosis pathway, ranging from the level of cell surface receptors to the executive caspases within the signaling pathway.

To overcome this low efficiency and the resistance of tumors to TRAIL, various combination therapies with radio- and chemotherapeutic agents were designed, which resulted in synergistic apoptotic effect (WO2009/002947; A. Almasan and A. Ashkenazi, Cytokine Growth Factor Reviews 14 (2003) 337-348; RK Srivastava, Neoplasis, Vol 3, No. 6, 2001, 535-546, Soria JC et al., J. Clin. Oncology, Vol 28, No. 9 (2010), p. 1527-1533). The use of rhTRAIL for cancer treatment in combination with selected conventional chemotherapeutic agents (paclitaxel, carboplatin) and monoclonal anti-VEGF antibodies is described in WO2009/140469. However, such a combination necessarily implies well-known deficiencies of conventional chemotherapy or radiotherapy. Prior art is silent, however, about any data suggesting abolishing of cell resistance to TRAIL obtained by fusing TRAIL protein with other proteins or fragments thereof.

Moreover, the problem connected with TRAIL therapy appeared to be its low stability and rapid elimination from the body after administration.

The effect of destruction of cancer cells and inhibition of tumor proliferation as a result of disintegration (discontinuity) of the cell membrane or mitochondrial membrane is known. There are also attempts to use substances with cytolytic effect capable of membrane disintegration both as an anti-cancer therapy and adjunct anti-cancer therapy.

Many natural and synthetic peptides and proteins having cytolytic activity are known. Cytolytic peptides are also described as pore-forming peptides or cytolysins. Interactions of pore forming peptides with the surface of the membrane may be based on nonspecific electrostatic interactions of the positively charged peptide with negatively charged surface of cell membrane.

These peptides are generally of cationic character, so that they are capable of electrostatic interactions with surfaces with predominantly negatively charged particles. Upon contact and interaction of a cytolytic peptide with lipids on the cell surface, and after penetration inside the cell with the lipids on the surface of the mitochondrial membrane, interruption of the continuity of the cell membrane occurs, followed by formation of small size transmembrane pores, by which leakage of the contents of the cytoplasm, including ions, outside the cell occurs, resulting in rapid and irreversible electrolyte imbalance in the cell, cell lysis and death.

The interactions of pore-forming peptides with the surface of the membrane may also include interactions with specific receptors present on the surface.

Known naturally occurring cytolytic peptides of bacterial, plant or mammalian origin capable of forming pores in the cell membrane are often called hemolysines, because they cause lysis of red blood cells and other eukaryotic cells. These toxins include cecropin A and B, aurein 1.2, citropin 1.1, defensin (HNP-2), lactoferricin B, tachyplesin, PR-39, cytolysins of *Enterococcus faecalis,* delta hemolysin, diphtheria toxin, cytolysin of *Vibrio cholerae,* toxin from *Actinia equina,* granulysin, lytic peptides from *Streptococcus intermedius,* lentiviral lytic peptides, leukotoxin of *Actinobacillus actinomycetemcomitans,* magainin, melittin, lymphotoxin, enkephalin, paradaxin, perforin (in particular the N-terminal fragment thereof), perfringolysin O (PFO/theta toxin) from *Clostridium perfringens,* and streptolysins. Their usefulness as medicaments is limited by their ability to cause hemolysis.

Natural cytolytic peptides are described, for example, in R. Smolarczyk et al., Postępy Hig. Med. Dosw., 2009; 63: 360-368

There are also known synthetic cytolytic pore-forming peptides. They are designed to be devoid of hemolytic properties, to be less immunogenic, or to have surfaces enabling high binding specificity to cellular targets such as for example VEGFR (vascular endothelial growth factor receptor) family receptors and the receptors of the EGFR (epidermal growth factor receptor) family. They are often hybrids of natural cytolytic peptides fragments, such as a hybrid of cecropin A fragment and magainin 2 CA (1-8) MA (1-12) fragment or a hybrid of cecropin A fragment and melittin CAMEL (CA (1-7) MEL (2-9)) fragment. There are also known synthetic cytolytic peptides $D\text{-}K_4\text{-}L_2\text{-}R_9$ and $D\text{-}K_6\text{-}L_9$, consisting of amino acids lysine, arginine and leucine, part of which is in the form of D-amino acids. There are also known synthetic chimeric peptides RGD-$4C_D$(KLAKLAK)$_2$, which contains the RGD motif binding with integrin $\alpha_v\beta_3$ and an effector domain composed of D-amino acids KLAKLAKKLAKLAK, and PTD-$5_D$(KLAKLAK)$_2$ containing PTD-5 motif which allows penetration into the cells and an effector domain composed of D-amino acids KLAKLAKKLAKLAK (see, for e.g., R. Smolarczyk et al., Postępy Hig. Med. Dosw., 2009, 63: 360-368). Other well-known cytolytic synthetic peptides are described, for example, in Regen et al., Biochem. Biophys. Res. Commun. 159: 566-571, 1989.

The destruction of the membrane occurring after adhering of the peptide to the membrane may occur by the mechanism of "barrel staves" (barrel-stave model), the mechanism of a "doughnut-like shape" (toroidal-pore model) or a "carpet" mechanism (see, for e.g., R. Smolarczyk et al., Postępy Hig. Med. Dosw., 2009; 63: 360-368).

The mechanism of "barrel staves" is observed for amphipathic peptides with alpha-helical conformation having a length of at least 23 amino acids. For example, peptides which cause the destruction of the membrane by the mechanism of "barrel staves" are gramicidin A, alameticin, perforin, pilosulin, synthetic peptides with repeated KLAK motifs, cathelicidin, peptides isolated from *Entamoeba histolytica,* parasporins and cecropins. Peptides, which cause the destruction of the membrane by the "toroidal pore model" include, for example, melittin and magainin. For example, peptides which cause the destruction of the membrane by the "carpet" model are cecropins A and B.

Disintegration of cell membrane with formation of pores may be also caused by interaction of peptides of a high positive charge with negatively charged membrane components. Such properties show, among others, granulysins, analogs and derivatives of melittin, peptides comprising K(L)xR motif, tachyplesin, bombesin, magainin and viscotoxin.

The formation of pores in the membrane of the target cell may also be associated with enzymatic activity of peptides. The enzymatic activity of phospholipase A is shown, for example, by phospholipases with specific phosphodiesterase activity against phosphatidylcholine and sphingomyelin, hemolysins and cytolysins having nonspecific cytolytic activity, or hemolysins and cytolysins having cytolytic activity against biological membranes containing, for example, cholesterol. This type of enzymatic activity resulting in the formation of pores in the cell or mitochondrial membrane is exhibited by listeriolysin, equinatoxin, phospholipase PC-PLC and alpha-toxin from *Clostridium perfringens.*

There are also known conjugates and chimeras of pore-forming peptides with domains capable to target to tumor cells. For targeting, there are used antigens, carbohydrate moieties or growth factor receptors, overexpressed on the surface of tumor cells. Targeted delivery provides high levels of pore-forming peptide on the cell surface which is necessary for cytolytic activity.

The use of targeted pore forming actinoporins is described in Panchal RG. et al., Poreforming proteins and their application in biotechnology. Curr Pharm Biotechnol 2002, 3:99-115; Panchal RG: Novel therapeutic strategies to selectively kill cancer cells. Biochem Pharmacol 1998, 55:247-252 and in Hoskin DW, Ramamoorthy A: Studies on anticancer activities of antimicrobial peptides. Biochim Biophys Acta-Biomembr 2008, 1778:357-87.

It is also known that pore-forming peptides and proteins may be endowed with the ability to direct to the tumor associated antigens and receptors by means of appropriate genetic modification as well as by chemical joining to the suitable ligands or antibodies. Such modifications are described for d-endotoxin *of Bacillus thuringiensis,* equinatoxin II from *Actinia equina,* sticholysin I of *Stichodactyla helianthus* and diphtheria toxin of *Corynebacterium diphtheriae* (Soletti RC., Potentiation of anticancer-drug cytotoxicity by sea anemone pore-forming proteins in human glioblastoma cells. Anti-Cancer Drugs 2008, 19:517-525; Pederzolli C,: Biochemical and cytotoxic properties of conjugates of transferrin with equinatoxin-II, a cytolysin from a sea anemone. Bioconjugate Chem 1995, 6:166-173, van der Spek JC.: Fusion protein toxins based on diphtheria toxin: Selective targeting of growth factor receptors of eukaryotic cells. Appl Chimeric Genes Hybrid Proteins Pt B 2000, 327:239-249).

Also described is a fusion protein consisting of pore forming sticholysin toxin I and a monoclonal antibody directed against a tumor specific antigen C2, and its usefulness in the treatment in a colon cancer cell line model (Tejuca M. et al., Construction of an immunotoxin with the pore forming protein St1 and/or C5, a monoclonal antibody against a colon cancer cell line, Int. Immunopharmacol. 2004, 4:731-744). A number of fusion proteins comprising diphtheria toxin and interleukin-2 or EGF, and their potential to destroy the cell overexpressing the target receptors is also described (Murphy JR, van der Spek JC, Targeting diphtheria-toxin to growth-factor receptors, Semin Cancer Biol 1995, 6:259-267).

There is also known the use of cleavage sites recognized by specific proteases in fusion proteins molecules comprising cytolytic peptides in order to enable the release of effector proteins in the tumor environment and, consequently, their internalization into tumor cells. For example, Panchal R. et al. (Nat Biotechnol 1996, 14:852-856) disclosed alpha-hemolysins comprising in their sequence a cleavage site recognized by cathepsin B, which is activated by a protease present in the tumor environment.

There are also known modified proaerolysins (PA), inactive precursors of bacterial cytolytic pore-forming proteins, activated when cleaved by protease of prostate cancer cells (PSA) (Williams S.A. et al., JNCI J. Natl. Cancer Inst. (2007) 99 (5): 376-385).

US5817771B1 discloses conjugates, including fusion proteins, of pore-forming cytolytic peptides with an antibody or antigen as an element selectively binding on a tumor cell, and linkers enabling the selective activation of the cytolytic peptide in the tumor environment, such as, for example cleavage site recognized by enzymes such as proteases, in particular proteases overexpressed specifically in the tumor environment.

Barua et al. (Cancer Letters 293 (2010) 240-253) reported that prostate cancer cell lines resistant to TRAIL and insensitive to treatment with death receptor agonist antibodies DR4 and DR5 become sensitive to these antibodies after pretreatment of these cells with synthetic cationic amphipathic lytic peptide KLA containing KLAK sequences.

The present invention provides fusion proteins with anti-cancer properties, which contain a domain derived from TRAIL and a domain of a cytolytic effector peptide with pore-forming properties against cell and/or mitochondrial membranes of mammalian cells.

Each of the two domains of the protein of the invention has different functions. Due to the presence of a domain derived from hTRAIL, proteins according to the invention are directed selectively to cancer cells, wherein the elements of the protein exert their effects. In particular, TRAIL domain after binding with a cell may exert its activity of triggering apoptosis, and the effector peptide the activity of forming pores in cell and/or mitochondrial membrane and causing lysis of the cancer cell.

Delivery of the protein of the invention into the tumor environment allows to minimize toxicity and side effects against healthy cells in the body, as well as reduction of the frequency of administration of a medicament. In addition, targeted therapy with the use of proteins according to the invention allows to avoid the problem of low efficiency of previously known nonspecific therapies based on the pores formation in the cell or mitochondrial membrane with the use of plant or bacterial toxins, caused by high toxicity and by the necessity of administering high doses.

It turned out that in many cases fusion proteins of the invention are more potent than soluble hTRAIL and its variants including the fragment of a sequence.

Until now, effector peptides used in the fusion protein of the invention have not been used in medicine as such because of unfavorable kinetics, rapid degradation by nonspecific proteases and accumulation in the body caused by lack of proper sequence of activation of pathways, which is necessary to enable the proper action of the effector peptide at target site. Incorporation of the effector peptides into the fusion protein allows their selective delivery to the site where their action is desirable. Furthermore, the attachment of the effector peptide increases the mass of protein, which results in prolonged half-life and increased retention of protein in the tumor and its enhanced efficiency.

Novel fusion proteins have also at least reduced or limited, or even substantially eliminated haemolytic activity compared to their individual natural cytolytic peptides.

Additionally, in many cases, novel fusion proteins also overcome natural or induced resistance to TRAIL. Most likely, overcoming the resistance is due to destabilization of the cell membrane potential as a result of the fusion proteins binding to lipids of cell or mitochondrial membrane and formation of pores, which causes leakage of divalent ions outside the cell. As a consequence of binding to the lipids of mitochondrial membrane, the release of cytochrome C, SMAC/Diablo protein and AIF factor into the cytoplasm occurs, which causes proapoptotic caspase activation in the affected cell. Degradation of the mitochondrial membranes leads also to the activation of caspase-9, resulting in the induction of apoptosis.

Description of Figures.

Fig. 1 presents tumor volume changes (% of initial stage) in Cby.Cg-foxn1(nu)/J mice burdened with lung cancer A549 treated with fusion protein of the invention of Ex. 11ª compared to rhTRAIL114-281;

Fig. 2 presents tumor growth inhibition values (%TGI) in Cby.Cg-foxn1(nu)/J mice burdened with lung cancer A549 treated with fusion protein of the invention of Ex. 11ª compared to rhTRAIL114-281;

Fig. 3 presents tumor volume changes (% of initial stage) in Crl:SHO-PrkdcscidHrhr mice burdened with lung cancer A549 treated with fusion protein of the invention of Ex. 11ª compared to rhTRAIL114-281;

Fig. 4 presents tumor growth inhibition values (%TGI) in Crl:SHO-PrkdcscidHrhr mice burdened with lung cancer A549 treated with fusion protein of the invention of Ex. 11ª compared to rhTRAIL114-281;

Fig. 5 presents tumor volume changes (% of initial stage) in Crl:SHO-PrkdcscidHrhr mice nu burdened with lung cancer NCI-H460-Luc2 treated with fusion protein of the invention of Ex. 11ª compared to rhTRAIL114-281;

Fig. 6 presents tumor growth inhibition values (%TGI) in Crl:SHO-PrkdcscidHrhr mice burdened with lung cancer NCI-H460-Luc2 treated with fusion protein of the invention of Ex. 11ª compared to rhTRAIL114-281;

Fig. 7 presents tumor volume changes (% of initial stage) in Cby.Cg-foxn1(nu)/J mice burdened with prostate cancer PC3 treated with fusion protein of the invention of Ex. 11ª compared to rhTRAIL114-281;

Fig. 8 presents tumor growth inhibition values (%TGI) in Cby.Cg-foxn1(nu)/J mice burdened with prostate cancer PC3 treated with fusion protein of the invention of Ex. 11ª compared to rhTRAIL114-281;

Fig. 9 presents tumor volume changes (% of initial stage) in Crl:SHO-PrkdcscidHrhr mice burdened with pancreas cancer PANC-1 treated with fusion protein of the invention of Ex. 11ª compared to rhTRAIL114-281;

Fig. 10 presents tumor growth inhibition values (%TGI) in Crl:SHO-PrkdcscidHrhr mice burdened with pancreas cancer PANC-1 treated with fusion protein of the invention of Ex. 11ª compared to rhTRAIL114-281;

Fig. 11 presents tumor volume changes (% of initial stage) in Crl:SHO-PrkdcscidHrhr mice burdened with colon cancer HCT116 treated with fusion protein of the invention of Ex. 11ª compared to rhTRAIL114-281;

Fig. 12 presents tumor growth inhibition values (%TGI) in Crl:SHO-PrkdcscidHrhr mice burdened with colon cancer HCT116 treated with fusion protein of the invention of Ex. 11ª compared to rhTRAIL114-281;

Fig. 13 presents tumor volume changes (% of initial stage) in Crl:SHO-PrkdcscidHrhr mice burdened with colon cancer SW620 treated with fusion protein of the invention of Ex. 16ª compared to rhTRAIL114-281;

Fig. 14 presents tumor growth inhibition values (%TGI) in Crl:SHO-PrkdcscidHrhr mice burdened with colon cancer SW620 treated with fusion protein of the invention of Ex. 16ª compared to rhTRAIL114-281;

Fig. 15 presents tumor volume changes (% of initial stage) in Crl:SHO-PrkdcscidHrhr mice burdened with colon cancer Colo205 treated with fusion protein of the invention of Ex. 16ª compared to rhTRAIL114-281;

Fig. 16 presents tumor growth inhibition values (%TGI) in Crl:SHO-PrkdcscidHrhr mice burdened with colon cancer Colo205 treated with fusion protein of the invention of Ex. 16ª compared to rhTRAIL114-281;

Fig. 17 presents tumor volume changes (% of initial stage) in Crl:SHO-PrkdcscidHrhr mice burdened with uterine sarcoma MES-SA/Dx5 treated with fusion protein of the invention of Ex. 11ª compared to rhTRAIL114-281;

Fig. 18 presents tumor growth inhibition values (%TGI) in Crl:SHO-PrkdcscidHrhr mice burdened with uterine sarcoma MES-SA/Dx5 treated with fusion protein of the invention of Ex. 11[a] compared to rhTRAIL114-281;

Fig. 19 presents tumor volume changes (% of initial stage) in Crl:SHO-PrkdcscidHrhr mice burdened with pancreatic carcinoma MIA Paca-2 treated with fusion protein of the invention of Ex. 16[b] compared to rhTRAIL114-281;

Fig. 20 presents tumor growth inhibition values (%TGI) in Crl:SHO-PrkdcscidHrhr mice burdened with pancreatic carcinoma MIA Paca-2 treated with fusion protein of the invention of Ex. 16[b] compared to rhTRAIL114-281;

Fig. 21 presents tumor volume changes (% of initial stage) in Crl:SHO-PrkdcscidHrhr mice burdened with colon cancer HCT116 treated with fusion protein of the invention of Ex. 16[b] compared to rhTRAIL114-281;

Fig. 22 presents tumor growth inhibition values (%TGI) in Crl:SHO-PrkdcscidHrhr mice burdened with colon cancer HCT116 treated with fusion protein of the invention of Ex. 16[b] compared to rhTRAIL114-281;

Fig. 23 presents tumor volume changes (% of initial stage) in Crl:SHO-PrkdcscidHrhr mice burdened with hepatocellular carcinoma HepG2 treated with fusion protein of the invention of Ex. 16[b] compared to rhTRAIL114-281;

Fig. 24 presents tumor growth inhibition values (%TGI) in Crl:SHO-PrkdcscidHrhr mice burdened with hepatocellular carcinoma HepG2 treated with fusion protein of the invention of Ex. 16[b] compared to rhTRAIL114-281;

Fig. 25 presents tumor volume changes (% of initial stage) in Crl:SHO-PrkdcscidHrhr mice burdened with hepatoma PLC/PRF/5 treated with fusion protein of the invention of Ex. 16[b] compared to rhTRAIL114-281; and

Fig. 26 presents tumor growth inhibition values (%TGI) in Crl:SHO-PrkdcscidHrhr mice burdened with hepatoma PLC/PRF/5 treated with fusion protein of the invention of Ex. 16[b] compared to rhTRAIL114-281.

## Detailed Description of the Invention

The invention relates to a fusion protein comprising:

- domain (a) which is a functional fragment of the sequence of soluble hTRAIL protein, said hTRAIL protein sequence being presented as SEQ. No. 90, which fragment begins with an amino acid at a position from the range hTRAIL95 to hTRAIL121, inclusive, and ends with the amino acid hTRAIL281 or a homolog of said functional fragment having at least 70% sequence identity, preferably 85% identity, wherein said functional fragment or homolog thereof are capable of inducing apoptotic signal in mammalian cells upon binding to its receptors on the surface of the cells, and

- at least one domain (b) which is the sequence of a cytolytic effector peptide with an amphipathic alpha-helix conformation forming pores in the cell membrane,

wherein the sequence of the domain (b) is attached at the C-terminus or N-terminus of domain (a).

The term "peptide" in accordance with the invention should be understood as a molecule built from plurality of amino acids linked together by means of a peptide bond. Thus, the term "peptide" according to the invention includes oligopeptides, polypeptides and proteins.

In the present invention, the amino acid sequences of peptides will be presented in a conventional manner adopted in the art in the direction from N-terminus (N-end) of the peptide towards its C-terminus (C-end). Any sequence will thus have its N-terminus on the left side and C-terminus on the right side of its linear presentation.

The term "a functional soluble fragment of the sequence of soluble hTRAIL protein" should be understood as denoting any such fragment of soluble hTRAIL protein that is capable of inducing apoptotic signal in mammalian cells upon binding to its receptors on the surface of the cells.

It will be also appreciated by a skilled person that the existence of at least 70% or 85% homology of the TRAIL sequence is known in the art.

It should be understood that domain (b) of the effector peptide in the fusion protein of the invention is neither hTRAIL protein nor a part or fragment of hTRAIL protein.

The fusion protein of the invention incorporates at least one domain (b) of the effector peptide, attached at the C-terminus and/or or at the N-terminus of domain (a).

By sequence hTRAIL it is understood the known sequence of hTRAIL published in the GenBank database under accession number P505591 as well as in EP0835305A1 and presented in the Sequence Listing of the present invention as SEQ. No. 90.

In a particular embodiment, domain (a) is the fragment of TRAIL sequence, beginning with an amino acid from the range of hTRAIL95 to hTRAIL121, inclusive, and ending with the amino acid TRAIL 281.

In particular, domain (a) may be selected from the group consisting of sequences corresponding to hTRAIL95-281, hTRAIL114-281, hTRAIL115-281, hTRAIL119-281 and hTRAIL121-281. It will be evident to those skilled in the art that hTRAIL95-281, hTRAIL114-281, hTRAIL115-281, hTRAIL116-281, hTRAIL119-281 and hTRAIL121-281 represent a fragment of human TRAIL protein starting with amino acid denoted with the number 95, 114, 115, 116, 119 and 121, respectively, and ending with the last amino acid 281, in the known sequence of TRAIL.

In another particular embodiment, domain (a) is the homolog of a functional fragment of soluble TRAIL protein sequence beginning at amino acid position not lower than hTRAIL95 and ending at amino acid hTRAIL281, the sequence of which is at least in 70%, preferably in 85%, identical to original sequence.

In specific variants of this embodiment domain (a) is the homolog of a fragment selected from the group consisting of sequences corresponding to hTRAIL95-281, hTRAIL114-281, hTRAIL115-281, hTRAIL116-281, hTRAIL119-281 and hTRAIL121-281.

It should be understood that the homolog of a TRAIL fragment is a variation/modification of the amino acid sequence of this fragment, wherein at least one amino acid is changed, including 1 amino acid, 2 amino acids, 3 amino acids, 4 amino acids, 5 amino acids, 6 amino acids, and not more than 15% of amino acids, and wherein the fragment of a modified sequence has preserved functionality of the TRAIL sequence, i.e. the ability to bind to cell surface death receptors and induce apoptosis in mammalian cells. Modification of the amino acid sequence may include, for example, substitution, deletion and/or addition of amino acids.

Preferably, the homolog of TRAIL fragment having modified sequence shows modified affinity to the death receptors DR4 (TRAIL-R1) or DR5 (TRAIL-R2) in comparison with the native fragment of TRAIL.

The term "modified affinity" refers to increased affinity and/or affinity with altered receptor selectivity.

Preferably, the homolog of the fragment of TRAIL having modified sequence shows increased affinity to the death receptors DR4 and DR5 compared to native fragment of TRAIL.

Particularly preferably, the homolog of a fragment of TRAIL having modified sequence shows increased affinity to the death receptor DR5 in comparison with the death receptor DR4, i.e. increased selectivity DR5/DR4.

Also preferably, the homolog of a fragment of TRAIL having modified sequence shows an increased selectivity towards the death receptors DR4 and/or DR5 in relation to the affinity towards the receptors DR1 (TRAIL-R3) and/or DR2 (TRAIL-R4).

Modifications of TRAIL resulting in increased affinity and/or selectivity towards the death receptors DR4 and DR5 are known to those skilled in the art. For example, Tur V, van der Sloot AM, Reis CR, Szegezdi E, Cool RH, Samali A, Serrano L, Quax WJ. DR4-selective tumor necrosis factor-related apoptosis-inducing ligand (TRAIL) variants obtained by structure-based design. J. Biol. Chem. 2008 Jul 18;283(29):20560-8, describe D218H mutation having increased selectivity towards DR4, and Gasparian ME, Chernyak BV, Dolgikh DA, Yagolovich AV, Popova EN, Sycheva AM, Moshkovskii SA, Kirpichnikov MP. Generation of new TRAIL mutants DR5-A and DR5-B with improved selectivity to death receptor 5, Apoptosis. 2009 Jun;14(6):778-87, describe D269H mutation having reduced affinity towards DR4. hTRAIL mutants resulting in increased affinity towards one receptor selected from DR4 and DR5 compared with DR1 and DR2 receptors and increased affinity towards receptor DR5 compared with DR4 are also described in WO2009077857 and WO2009066174.

Suitable mutations are one or more mutations in the positions of native hTRAL selected from the group consisting of amino acids 131, 149, 159, 193, 199, 201, 204, 204, 212, 215, 218 and 251, in particular mutations involving substitution of an amino acid with a basic amino acid such as lysine, histidine or arginine, or an acidic amino acid such as glutamic acid or aspargic acid. In particular, one or more mutations selected from the group consisting of G131R, G131K, R149I, R149M, R149N, R149K, S159R, Q193H, Q193K, N199H, N199R, K201H, K201R, K204E, K204D, K204L, K204Y, K212R, S215E, S215H, S215K, S215D, D218Y, D218H, K251D, K251E and K251Q, as described in WO2009066174, may be mentioned.

Suitable mutations are also one or more mutations in the positions of native hTRAIL selected from the group consisting of amino acids 195, 269 and 214, particularly mutations involving substitution of an amino acid with a basic amino acid such as lysine, histidine or arginine. In particular, one or more mutations selected from the group consisting of D269H, E195R, and T214R, as described in WO2009077857, may be mentioned.

In a particular embodiment, the domain (a) which is the homolog of a fragment of hTRAIL, is selected from D218H mutant of the native TRAIL sequence, as described in WO2009066174, or the Y189N-R191K-Q193R-H264R-I266R-D269H mutant of the native TRAIL sequence, as described in Gasparian ME et al. Generation of new TRAIL mutants DR5-A and DR5-B with improved selectivity to death receptor 5, Apoptosis. 2009 Jun; 14(6): 778-87.

Domain (a), i.e. the fragment of TRAIL, is a domain responsible for binding of the construct of the fusion protein to death receptors on the surface of a cell. Furthermore, domain (a) upon binding will exert its known agonistic activity, i.e. activation of extrinsic pathway of apoptosis.

Domain (b) of the fusion protein of the invention is the domain of an effector peptide with cytolytic activity against eukaryotic cell.

In particular embodiments of the fusion protein of the invention, the effector peptide of domain (b) of the fusion protein is a peptide having pore-forming activity against cancer cells, selected from the group consisting of SEQ. No. 34 to SEQ. No. 56, and SEQ. No. 125 to SEQ. No. 132.

For the peptide with cytolytic activity it is meant a peptide having the ability of forming pores in the cell membrane, and after penetration into the cell, also in the mitochondrial membrane, thereby disrupting the continuity of the membrane. As a result of the disruption of membrane, a leakage of the contents of the cytoplasm, including ions, outside the cell occurs, which causes rapid and irreversible electrolyte imbalance in the cell, and its destruction (cell lysis).

The ability of a peptide to form pores in the cell or mitochondrial membrane and thus *cause* cell lysis can be determined by a method of testing permeabilization of cell membranes known to those skilled in the art, for example by measuring the release from the cell of intracellular substances which previously had been applied to the cell, e.g. of ATP or radiolabelled marker, or by measuring the uptake of a dye, such as trypan blue, which does not occur when the cells are intact.

The cytolytic effector peptide of the invention may be either a natural peptide or a synthetic peptide.

The effector peptide of domain (b) of the fusion protein of the invention is a pore-forming peptide possessing amphipathic alpha-helixes conformation enabling interactions with biological membranes.

Exemplary sequences of the effector peptide in this embodiment are designated as SEQ. No. 36 (pilosulin-1), SEQ. No. 37 (pilosulin-5), SEQ. No. 41 (14-amino acids synthetic lytic peptide), SEQ. No. 43 (27-amino acids peptide FF/CAP-18), SEQ. No. 44 (BAMP-28 peptide), SEQ. No. 45 (the analogue of isoform C of lytic peptide from *Entamoeba histolytica*), SEQ. No. 46 (the analogue of isoform A of lytic peptide from *Entamoeba histolytica*), SEQ. No. 47 (the analogue of isoform B of lytic peptide from *Entamoeba histolytica*), SEQ. No. 48 (the fragment of HA2 domain of influenza virus hemagglutinin), SEQ. No. 54 (the active fragment of human perforin), SEQ. No. 55 (parasporin-2 from *Bacillus thuringensis*), SEQ. No. 125 synthetic fusion peptide with KLLK motif, SEQ. No. 126, and SEQ. No. 127 (pleurocidin analogues), SEQ. No. 128 synthetic peptide with KLLK motif.

Another effector peptide of domain (b) with pore-forming activity against eukaryotic cell may be a peptide pilosulin-1, which is a cationic molecule derived from venom of the Australian ant *Myrmecia Pilosula.* Pilosulin 1 is a peptide with high content of lysine and arginine regularly repeated in a sequence. Due to the high content of these amino acids, the peptide has a strong positive charge allowing its selective interaction with membranes of cancer cells and formation of pores through the "barrel staves" mechanism (Kourie et al., Am J Physiol Cell Physiol, 278: 1063 - 1087, 2000).

In particular, such an effector peptide is 56-amino acids peptide presented in the attached sequence listing as SEQ. No. 36.

Another effector peptide of domain (b) with pore-forming activity against eukaryotic cell may be peptide pilosulin-5, responsible for ionic interactions with the cell membrane resulting in the formation of pores, and as a consequence inhibition of tumor growth. Pilosulin 5 is the peptide belonging to the pilosulins family derived from the venom of Australian ant *Myrmecia Pilosula.* This peptide has in its structure cyclically repeated pattern of amino acids lysine, alanine, and aspartic acid, imparting a positive charge, which can potentiate interactions with tumor cells surface.

In particular, such an effector peptide is 100-amino acids peptide presented in the attached sequence listing as SEQ. No. 37.

Another cytolytic effector peptide of domain (b) with activity against eukaryotic cell may be a synthetic lytic peptide. Synthetic peptides of formula $(KLAKKLA)_n$ (where n is a number of repetitions of the motif) as amphipathic and alpha-helical proteins after penetration into the cell are selectively accumulated in the negatively charged mitochondrial membrane, causing formation of pores and destabilization of electrostatic potential of mitochondria, thereby selectively eliminating cells of selected cancer cell lines (Javadpour et al, J Med Chem, 39:3107-13, 1996).

In particular, such an effector peptide is 14-amino acids peptide presented in the attached sequence listing as SEQ. No. 41.

Another effector peptide of domain (b) with cytolytic activity against eukaryotic cell may be an another synthetic lytic peptide which disintegrates the cell membrane in a detergent-like manner. (Papo N, Shai Y. New lytic peptides based on the D,L-amphipathic helix motif preferentially kill tumor cells compared to normal cells. Biochemistry. 2003 Aug 12;42(31):9346-54).

In particular, such an effector peptide is 17-amino acids peptide presented in the attached sequence listing as SEQ. No. 128.

Another effector peptide of domain (b) with pore forming activity is the peptide FF/CAP18 described by Isogai E. in "Antimicrobial and Lipopolysaccharide-Binding Activities of C-Terminal Domain of Human CAP18 Peptides to Genus Leptospira", The Journal of Applied Research, Vol. 4, No. 1, 2004, 180-185). FF/CAP18 is the analogue of 27-amino acids C-terminal sequence of human cathelicidin $hCAP18_{109-135}$, which was modified by replacement of 2 amino acid residues with phenylalanines. FF/CAP18 has strongly cationic character, increased in relation to the native sequence due to incorporated modification, and strongly binds to eukaryotic cell membranes. Once bound to the surface of the membrane, FF/CAP18 forms channels and ionic pores, leading to destabilization of electrostatic balance of cells. In addition, after penetration inside the cell, the analog builds into the mitochondrial membrane to form ion channels, thus destabilizing the electrostatic potential of mitochondria and leading to the release from mitochondria to cytosol factors such as cytochrome C, SMAC/Diablo or AIF factor, which initiates the process of apoptosis.

In particular, such an effector peptide is 27-amino acids peptide presented in the attached sequence listing as SEQ. No. 43.

Another effector peptide of domain (b) with pore forming activity is a peptide BAMP-28 with strong positive charge belonging to the cathelicidins family. This peptide is also the structural analogue of human histatins, a group of 12 peptides with a mass below 4 kDa produced by salivary glands cells and exhibiting antibacterial and antifungal properties (W. Kamysz et al., Histatyny - biatka liniowe bogate w histydynę, Nowa Stomatologia 2004). The N-terminal domain of BAMP-28 peptide is strongly positively charged and is responsible for docking to the cell membrane, whereas the C-terminal part is responsible for the cytotoxic activity. (Hugosson, M., D. et al., 1994). Antibacterial peptides and mitochondrial presequences affect mitochondrial coupling, respiration and protein import. Eur. J. Biochem. 223:1027-1033.). The mechanism of BMAP-28 peptide activity is primarily based on the formation of pores in cell and mitochondrial membranes (A. Risso et al., BMAP-28, an Antibiotic Peptide of Innate Immunity, Induces Cell Death through Opening of the Mitochondrial Permeability Transition Pore, MOLECULAR AND CELLULAR BIOLOGY, Mar. 2002, p. 1926-1935).

In particular, such an effector peptide is 27-amino acids peptide presented in the attached sequence listing as SEQ. No. 44.

Another effector peptide of domain (b) with pore forming activity is the analogue of isoform A of lytic peptide from *Entamoeba histolytica* responsible for accumulation on the cell surface resulting in pores formation, which in consequence leads to inhibition of tumor growth. Three isoforms A, B, and C of the peptides of Entamoeba histolytica have been identified, located in the granular cytoplasm of the parasite. These are 77-amino acids polypeptides stabilized by three sulfide bridges, containing in the secondary structure four amphipathic helices (Leippe, M et al EMBO J. 11, 3501-3506, 1992). These peptides have lytic properties against eukaryotic cells (Leippe, M. and Müller-Eberhard, H.J. Toxicology 87, 5-18, 1994). The third helix in the structure of these peptides has a length suitable for penetration of the cell membrane and formation of pores. Based on the amino acid sequences comprising only the third helix domain of all three isoforms, a series of analog synthetic peptides (A3, B3 and C3) has been constructed having the pore-forming and cytotoxic activity against the human cancer cell lines and characterized with low hemolytic activity (Andrä et al., FEBS Letters 385: 96-100,1996).

In particular, such an effector peptide is 24-amino acids peptide presented in the attached sequence listing as SEQ. No. 45.

Another effector peptide of domain (b) with pore forming activity against eukaryotic cell is an analogue of isoform B of lytic peptide from *Entamoeba histolytica.*

In particular, such an effector peptide is 24-amino acids peptide presented in the attached sequence listing as SEQ. No. 46.

Another effector peptide of domain (b) with pore forming activity against eukaryotic cell is an analogue of isoform C of lytic peptide from *Entamoeba histolytica.*

In particular, such an effector peptide is 24-amino acids peptide presented in the attached sequence listing as SEQ. No. 47.

Another effector peptide of domain (b) with pore forming activity against eukaryotic cell is a homologue of 20-amino acids N-terminal fragment, so called "fusion peptide", of HA2 domain of influenza virus haemagglutinin, responsible for interaction of viral capsid with host cell membrane (intercalation) resulting in formation of pores in the cell membrane of the host. Haemagglutinin (HA) of the influenza virus is a homotrimeric glycoprotein responsible for fusion of viral capsid with host cell membrane. Two domains can be distinguished in the structure of the protein, HA1 responsible for receptor binding and H2 responsible for interactions with cell membrane. In the structure of HA2 domain only N-terminal part (20 amino acids), so-called "fusion peptide", directly intercalates into the structure of the cell membrane (Dürrer P et al., J Biol Chem 271:13417-13421, 1996). Structural analysis of fusion peptide homologues showed that its activity is associated with conformational change leading to the formation of amphipathic alpha helices, which are capable of endosome membrane perforation (Takahashi S., Biochemistry 29: 6257-6264, 1990). Therefore, derivatives of "fusion peptide" can be used as effective carriers of biologically active substance providing an efficient and quick "escape" from endosomes.

In particular, such an effector peptide is 12-amino acids peptide presented in the attached sequence listing as SEQ. No. 48.

Another effector peptide of domain (b) with pore forming activity against eukaryotic cell is a peptide which is an active fragment of human perforin. The use of proteins of human origin which are "invisible" to the immune system, including human perforin, can solve the problem of limited clinical usefulness of protein chimeras containing toxins of bacterial, animal or plant origin because of generated strong immunogenicity (Frankel AE. Reducing the immune response to immunotoxin. Clin Cancer Res. 2004 Jan 1;10(1 Pt 1):13-5). N-terminal 34-amino acids fragment of human perforin forming nonspecifically pores in the cell membrane retains selective cytotoxic activity of the whole protein (Liu CC, Walsh CM, Young JD. Perforin: structure and function. Immunol Today. 1995 Apr;16(4):194-201). A perforin fragment fused to an antibody targeting to cancer cells retains selective cytotoxic activity of the whole protein (Wan L. Expression, purification, and refolding of a novel immunotoxin containing humanized single-chain fragment variable antibody against CTLA4 and the N-terminal fragment of human perforin. Protein Expr. Purif. 2006 Aug;48(2):307-13. Epub 2006 Mar 9).

In particular, such an effector peptide is 33-amino acids peptide presented in the attached sequence listing as SEQ. No. 54.

Another effector peptide of domain (b) with pore-forming activity against eukaryotic cell is parasporin-2 from *Bacillus thuringensis.* Parasporins family comprises 13 different toxins belonging to subgroups PS1, PS2- PS3, PS4 (Ohba M. Parasporin, a new anticancer protein group from Bacillus thuringiensis. Anticancer Res. 2009 Jan;29(1):427-33). Parasporin-2 exerts specificity against cancer cells (MOLT-4, Jurkat, HL60, HepG2, CACO-2) and exists in a form of 37-kDa protoxin activated by cutting off by proteinase K a portion of N and C-terminal fragments of respectively 51 and 36 amino acids. Key action of parasporin-2 consist of oligomerization within the cell membrane to form pores having a diameter of about 3 nm, resulting in increasing its permeability. Effects of parasporin-2 activity depend on the type of cell lines tested and include formation of so-called "blebbs" or bulges caused by the outflow of the cytoplasm from the cells and their lysis (HepG2 and NIH-3T3 cells) or formation of vacuole-like structures resulting in the burst of cells (MOLT-4) (Kitada S. Cytocidal actions of parasporin-2,an anti-tumor crystal toxin from Bacillus thuringiensis. J. Biol. Chem. 2006 Sep 8;281(36):26350-60). In addition, activity of parasporin-2 leads to the destruction of the structure of microtubules, actin filaments entanglement, fragmentation of mitochondria and endoplasmic reticulum (Akiba T. Crystal structure of the parasporin-2 Bacillus thuringiensis toxin that recognizes cancer cells. J. Mol. Biol. 2009 Feb 13;386(1):121-33).

In particular, such an effector peptide is 251-amino acids peptide presented in the attached sequence listing as SEQ. No. 55.

Another effector peptide of domain (b) with pore-forming activity against eukaryotic cell is a fusion protein comprising synthetic lytic peptide with KLLK motif and a peptide being antagonist of PDGF receptor on the cell surface. Binding of an inhibitor of PDGF on the cell surface allows location of the lytic peptide to the cell surface, and additionally binding affects cell proliferation and angiogenesis (Ostman A. et al., PDGF Receptors as Targets in Tumor Treatment, Adv. Cancer Res., 2007;97:247-74.)

In particular, such an effector peptide is 39-amino acids peptide presented in the attached sequence listing as SEQ. No. 125.

Fusion peptide presented in the attached sequence listing as SEQ. No. 125 and a fusion variant of PDGF antagonist and synthetic lytic peptide of SEQ. No. 125 is novel and has not been described before.

Another effector peptide of domain (b) with pore-forming activity against eukaryotic cell is a protein being analogue of pleurocidin. Pleurocidins are cationic $\alpha$-helical proteins interacting with cell membrane (Cole AM et al., Isolation and characterization of pleurocidin, an antimicrobial peptide in the skin secretions of winter flounder. J Biol Chem. 1997 May 2;272(18):12008-13*).* Pleurocidin-like peptides are active against breast carcinoma cells, including drug-resistant and slow-growing breast cancer cells. (Hilchie AL et al., Pleurocidin-family cationic antimicrobial peptides are cytolytic for breast carcinoma cells and prevent growth of tumor xenografts. Breast Cancer Res. 2011 Oct 24;13(5):R102).

In particular, such effector peptides are 25-amino acids peptide presented in the attached sequence listing as SEQ. No. 126 and 26 amino acids peptide presented in the attached sequence listing as SEQ. No. 127.

Upon binding to TRAIL receptors present on the surface of cancer cells, the fusion protein will exert a double effect. Domain (a), that is a functional fragment of TRAIL or its homolog with preserved functionality, will exert its known agonistic activity, i.e. binding to death receptors on the cell surface and activation of extrinsic pathway of apoptosis. The effector peptide of the domain (b) of the fusion protein will be able to potentially exert its action extracellularly or intracellularly in parallel to the activity of TRAIL domain.

In the fusion protein according to the invention, the antitumor TRAIL activity is potentiated by formation of pores in the cell or mitochondrial membrane resulting in disturbance of electrostatic charge of the cell, leakage of ions from the cytoplasm or destabilization of electrostatic potential mitochondria and release into the cytoplasm of factors such as cytochrome C, SMAC/DIABLO or factor AIF, which in turn activates internally induced apoptosis synergistic with the signal from the attachment of TRAIL to functional receptors of DR series.

The new fusion proteins also exhibit at least a reduced or limited, or even substantially eliminated haemolytic activity characteristic for the individual natural cytolytic peptides.

In one of the embodiments of the invention, domain (a) and domain (b) are linked by at least one domain (c) comprising the sequence of a cleavage site recognized by proteases present in the cell environment, especially in the tumor cell environment, e.g. such as metalloprotease, urokinase or furin.

Sequences recognized by protease may be selected from:

- a sequence recognized by metalloprotease MMP Pro Leu Gly Leu Ala Gly Glu Pro/ PLGLAGEP, or fragment thereof which with the last amino acid of the sequence to which is attached forms a sequence recognized by metalloprotease MMP,

- a sequence recognized by urokinase uPA Arg Val Val Arg/RVVR, or fragment thereof, which with the last amino acid of the sequence to which is attached forms a sequence recognized by urokinase,
  and combinations thereof, or

- a sequence recognized by furin Arg Gln Pro Arg/RQPR, Arg Gln Pro Arg Gly/RQPRG, Arg Lys Lys Arg/RKKR) or others atypical sequences recognized by furin disclosed by M. Gordon et al., in Inf. and Immun, 1995, 63, No. 1, p. 82-87, or native sequences recognized by furin Arg His Arg Gln Pro Arg Gly Trp Glu Gln Leu/RHRQPRGWEQL or HisArgGlnProArgGlyTrpGluGln / HRQPRGWEQ) or fragment thereof, which with the last amino acid of the sequence to which is attached forms a sequence recognized by furin.

In one of the embodiments of the invention, the protease cleavage site is a combination of the sequence recognized by metalloprotease MMP and/or a sequence recognized by urokinase uPA and/or a sequence recognized by furin located next to each other in any order.

Preferably, in one of the embodiments domain (c) is a sequence recognized by furin selected from Arg Val Val Arg Pro Leu Gly Leu Ala Gly/ RVVRPLGLAG and Pro Leu Gly Leu Ala Gly Arg Val Val Arg/PLGLAGRVVR.

Proteases metalloprotease MMP, urokinase uPA and furin are overexpressed in the tumor environment. The presence of the sequence recognized by the protease enables the cleavage of domain (a) from domain (b), i.e. the release of the functional domain (b) and thus its accelerated activation.

Activation of the effector peptide - functional domain (b) after internalization of the fusion protein into the cell may occur nonspecifically by a cleavage of domain (a) from domain (b) of the fusion protein of the invention by lisosomal enzymes (non-specific proteases).

The presence of the protease cleavage site, by allowing quick release of the effector peptide, increases the chances of transporting the peptide to the place of its action as a result of cutting off from the hTRAIL fragment by means of protease overexpressed in the tumor environment before random degradation of the fusion protein by non-specific proteases occurs.

Additionally, a transporting domain (d) may be attached to domain (b) of the effector peptide of the fusion protein of the invention.

Domain (d) may be selected from the group consisting of:

(d1) polyhistidine sequence transporting through the cell membrane, consisting of 6, 7 , 8, 9, 10 or 11 histidine residues (His/H);
and

(d2) polyarginine sequence transporting through the cell membrane, consisting of 6, 7 , 8, 9, 10 or 11 arginine residues (Arg/R),

(d3) PD4 transporting sequence (protein transduction domain 4) Tyr Ala Arg Ala Ala Ala Arg Gln Ala Arg Ala /YARAAARQARA,

(d4) a transporting sequence consisting of transferrin receptor binding sequence Thr His Arg Pro Pro Met Trp Ser Pro Val Trp Pro /THRPPMWSPVWP,

(d5) PD5 transporting sequence (protein transduction domain 5, TAT protein) Tyr Gly Arg Lys Lys Arg Arg Gln Arg Arg Arg /YGRKKRRQRRR,

or fragments thereof, which with the last amino acid of the sequence to which they are attached form sequences of transporting domains (d1) or (d2);
and

- combinations thereof.

The combination of domains, e.g. (d1) and (d2), may comprise in particular the combination (d1)/(d2) and (d2)/(d1).

Furthermore, the combination of domains, e.g. (d1) and (d2), may include domains located next to each other and connected to one end of domain (b) and/or domains linked to different ends of domain (b).

It should be understood that in the case when the fusion protein has both the transporting domain (d) attached to domain (b) and domain (c) of the cleavage site between domains (a) and (b), then domain (c) is located in such a manner that after cleavage of the construct transporting domain (d) remains attached to domain (b). In other words, if the fusion protein contains both the transporting domain (d) and the cleavage site domain (c), then domain (d) is located between domain (b) and domain (c), or is located at the end of domain (b) opposite to the place of attachment of domain (d).

The invention comprises also a variant, wherein domain (d) is located between two (c) domains, that is the variant wherein after cleavage of the construct transporting domain, preferably the translocation domain, is not attached neither to the TRAIL domain nor to the effector peptide domain.

The invention does not comprise such a variant in which domain (d) is located between domain (c) and domain (a), that is the variant wherein after cleavage of the construct transporting domain remains attached to the TRAIL domain.

In another embodiment, between domain (a) and domain (b) there is additionally located domain (e) comprising a sequence suitable for attachment of a PEG molecule to the fusion protein (pegylation linker). Such a linker may be known sequence Ala Ser Gly Cys Gly Pro Glu Gly/ASGCGPEG or fragments thereof, which with the last amino acid of the sequence to which it is attached forms a sequence suitable for attachment of a PEG molecule. The pegylation linker may be also selected from the group of the following:

Ala Ala Cys Ala Ala/AACAA,

Ser Gly Gly Cys Gly Gly Ser/SGGCGGS, and

Ser Gly Cys Gly Ser/SGCGS,

or fragment thereof, which with the last amino acid of the sequence to which it is attached forms a sequence suitable for attachment of a PEG molecule Preferably, the sequence of pegylation linker is Ala Ser Gly Cys Gly Pro Glu Gly/ ASGCGPEG.

Apart from main functional elements of the fusion protein and the cleavage site domain(s), the fusion proteins of the invention may contain a neutral sequence/sequences of a flexible steric linker. Such steric linkers are well known and described in the literature. Their incorporation into the sequence of the fusion protein is intended to provide the correct folding of proteins produced by the process of its overexpression in the host cells. In particular, steric linker may be a glycine, glycine-serine or glycine-cysteine-alanine linker.

In particular, steric linker may be a combination of glycine and serine residues such as Gly Gly Gly Gly Ser/GGGGS or any fragment thereof acting as steric linker, for example a fragment Gly Gly Gly Ser/GGGS, Gly Gly Gly/GGG or Gly Gly Gly Gly/GGGG, Gly Gly Ser Gly Gly, Gly Gly Ser Gly/GGSG, Gly Ser Gly/GSG or Ser Gly Gly/SGG, or combinations thereof.

In other embodiment, the steric linker may be any combination of glycine, serine and alanine residues such as Ala Ser Gly Gly/ASGG or any fragment thereof acting as steric linker, for example Ala Ser Gly/ASG. It is also possible to use the combination of steric linkers, for example the sequence Gly Gly Gly Ser Gly / GGGGS or any fragment thereof acting as steric linker, for example the fragment Gly Gly Gly/GGG, with another fragment acting as steric linker. In such a case the steric linker may be a combination of glycine, serine and alanine residues such as Gly Gly Gly Ser Ala Ser Gly Gly/GGGSASGG, Gly Gly Ser Gly Gly Gly Ser Gly Gly Gly /GGSGGGSGGG, Gly Gly Ser Gly Gly Gly Gly Gly Ser/GGSGGGGGS or Gly Gly Gly Gly Gly Gly Ser/ GGGGGGS. In still another embodiment, steric linker may be a combination of serine and histidine residues Ser His His Ser/SHHS or Ser His His Ala Ser/SHHAS.

In still another embodiment, the steric linker may be also selected from single amino acid residues such as single glycine or cysteine residue, in particular one or two up to four glycine or cysteine residues.

In another embodiment, the linker may also be formed by a fragment of steric linkers described above, which with the terminal amino acid of the sequence to which it is attached forms a steric linker sequence.

In another embodiment, the steric linker may promote the formation and stabilization of the structure of the trimer of the fusion protein of the invention, thus increasing its half-life in the blood circulation system and preventing from deasociation which may affect activity of the protein after administration into the blood circulation system. In this case the linker is a combination of cysteine and alanine, for example, a fragment Cys Cys Ala Ala Ala Ala Ala Cys/ CAAACAAC or Cys Cys Ala Ala Ala Ala Ala Cys / CAACAAAC or fragments thereof, which is the terminal amino acid sequence to which it is attached and forms a steric linker sequence stabilising the trimer structure.

In addition, the steric linker may also be useful for activation of functional domain (b), occurring in a non-specific manner. Activation of domain (b) in a non-specific manner may be performed by cutting off the domain (a) from the domain (b) of the fusion protein according to the invention due to pH-dependent hydrolysis of the steric linker.

Particular embodiments of the fusion protein of the invention are fusion proteins comprising a pore-forming peptide selected from the group of peptides represented by:

SEQ. No. 36, SEQ. No. 37, SEQ. No. 41, SEQ. No. 43, SEQ. No. 44, SEQ. No. 45, SEQ. No. 46, SEQ. No. 47, SEQ. No. 48, SEQ. No. 54, SEQ. No. 55, SEQ. No. 125, SEQ. No. 126, SEQ. No. 127, and SEQ. No. 128.

A detailed description of the structure of representative fusion proteins mentioned above are shown in the Examples presented below.
In accordance with the present invention, by the fusion protein it is meant a single protein molecule containing two or more proteins or fragments thereof, covalently linked via peptide bond within their respective peptide chains, without additional chemical linkers.
The fusion protein can also be alternatively described as a protein construct or a chimeric protein. According to the present invention, the terms "construct" or "chimeric protein", if used, should be understood as referring to the fusion protein as defined above.
For a person skilled in the art it will be apparent that the fusion protein thus defined can be synthesized by known methods of chemical synthesis of peptides and proteins.
The fusion protein can be synthesized by methods of chemical peptide synthesis, especially using the techniques of peptide synthesis in solid phase using suitable resins as carriers. Such techniques are conventional and known in the art, and described inter alia in the monographs, such as for example Bodanszky and Bodanszky, The Practice of Peptide Synthesis, 1984, Springer- Verlag, New York, Stewart et al., Solid Phase Peptide Synthesis, 2nd Edition, 1984, Pierce Chemical Company.
The fusion protein can be synthesized by the methods of chemical synthesis of peptides as a continuous protein. Alternatively, the individual fragments (domains) of protein may be synthesized separately and then combined together in one continuous peptide via a peptide bond, by condensation of the amino terminus of one peptide fragment from the carboxyl terminus of the second peptide. Such techniques are conventional and well known.
Preferably, however, the fusion protein of the invention is a recombinant protein, generated by methods of gene expression of a polynucleotide sequence encoding the fusion protein in host cells.
For verification of the structure of the resulting peptide known methods of the analysis of amino acid composition of peptides may be used, such as high resolution mass spectrometry technique to determine the molecular weight of the peptide. To confirm the peptide sequence, protein sequencers can also be used, which sequentially degrade the peptide and identify the sequence of amino acids.
The invention discloses a polynucleotide sequence, particularly DNA sequence, encoding the fusion protein as defined above.
Preferably, the polynucleotide sequence, particularly DNA, according to the invention, encoding the fusion protein as defined above, is a sequence optimized for expression in E. coli.
The invention discloses also an expression vector containing the polynucleotide sequence, particularly DNA sequence of the invention as defined above.
The invention discloses also a host cell comprising an expression vector as defined above.
Methods for generation of recombinant proteins, including fusion proteins, are well known. In brief, this technique consists in generation of polynucleotide molecule, for example DNA molecule encoding the amino acid sequence of the target protein and directing the expression of the target protein in the host. Then, the target protein encoding polynucleotide molecule is incorporated into an appropriate expression vector, which ensures an efficient expression of the polypeptide. Recombinant expression vector is then introduced into host cells for transfection/transformation, and as a result a transformed host cell is produced. This is followed by a culture of transformed cells to overexpress the target protein, purification of obtained proteins, and optionally cutting off by cleavage the tag sequences used for expression or purification of the protein.
Suitable techniques of expression and purification are described, for example in the monograph Goeddel, Gene Expression Technology, Methods in Enzymology 185, Academic Press, San Diego, CA (1990), and A. Staron et al., Advances Mikrobiol., 2008, 47, 2, 1983-1995.
Cosmids, plasmids or modified viruses can be used as expression vectors for the introduction and replication of DNA sequences in host cells. Typically plasmids are used as expression vectors. Suitable plasmids are well known and commercially available.
Expression vector comprises a polynucleotide molecule encoding the fusion protein of the invention and the necessary regulatory sequences for transcription and translation of the coding sequence incorporated into a suitable host cell. Selection of regulatory sequences is dependent on the type of host cells and can be easily carried out by a person skilled in the art. Examples of such regulatory sequences are transcriptional promoter and enhancer or RNA polymerase binding sequence, ribosome binding sequence, containing the transcription initiation signal, inserted before the coding sequence, and transcription terminator sequence, inserted after the coding sequence. Moreover, depending on the host cell and the vector used, other sequences may be introduced into the expression vector, such as the origin of replication, additional DNA restriction sites, enhancers, and sequences allowing induction of transcription.

The expression vector will also comprise a marker gene sequence, which confers defined phenotype to the transformed cell and enables specific selection of transformed cells. Furthermore, the vector may also contain a second marker sequence which allows to distinguish cells transformed with recombinant plasmid containing inserted coding sequence of the target protein from those which have taken up the plasmid without insert. Most often, typical antibiotic resistance markers are used, however, any other reporter genes known in the field may be used, whose presence in a cell (in vivo) can be easily determined using autoradiography techniques, spectrophotometry or bio- and chemi-luminescence. For example, depending on the host cell, reporter genes such as ß-galactosidase, ß-glucuronidase, luciferase, chloramphenicol acetyltransferase or green fluorescent protein may be used.

Furthermore, the expression vector may contain signal sequence, transporting proteins to the appropriate cell compartment, e.g. periplasma, where folding is facilitated. Additionally a sequence encoding a label/tag, such as HisTag attached to the N-terminus or GST attached to the C-terminus, may be present, which facilitates subsequent purification of the protein produced using the principle of affinity, via affinity chromatography on a nickel column. Additional sequences that protect the protein against proteolytic degradation in the host cells, as well as sequences that increase its solubility may also be present.

Auxiliary element attached to the sequence of the target protein may block its activity, or be detrimental for another reason, such as for example due to toxicity. Such element must be removed, which may be accomplished by enzymatic or chemical cleavage. In particular, a six-histidine tag HisTag or other markers of this type attached to allow protein purification by affinity chromatography should be removed, because of its described effect on the liver toxicity of soluble TRAIL protein. Heterologous expression systems based on various well-known host cells may be used, including prokaryotic cells: bacterial, such as *Escherichia coli* or *Bacillus subtilis,* yeasts such as *Saccharomyces cervisiae* or *Pichia pastoris,* and eukaryotic cell lines (insect, mammalian, plant).

Preferably, due to the ease of culturing and genetic manipulation, and a large amount of obtained product, the *E. coli* expression system is used. Accordingly, the polynucleotide sequence containing the target sequence encoding the fusion protein of the invention will be optimized for expression in *E. coli,* i.e. it will contain in the coding sequence codons optimal for expression in *E. coli,* selected from the possible sequence variants known in the state of art. Furthermore, the expression vector will contain the above described elements suitable for *E. coli* attached to the coding sequence.

Accordingly, in a preferred embodiment, a polynucleotide sequence comprising a sequence encoding a fusion protein of the invention, optimized for expression in *E. coli* is selected from the group of polynucleotide sequences consisting of:

SEQ. No. 60; SEQ. No. 61; SEQ. No. 62; SEQ. No. 67; SEQ. No. 68; SEQ. No. 69; SEQ. No. 70; SEQ. No. 71; SEQ. No. 72; SEQ. No. 74; SEQ. No. 75; SEQ. No. 76; SEQ. No. 77; SEQ. No. 78; SEQ. No. 79; SEQ. No. 86; SEQ. No. 87; SEQ. No. 88; SEQ. No. 108; SEQ. No. 109; SEQ. No. 110; SEQ. No. 111; SEQ. No. 112; SEQ. No. 113; SEQ. No. 114, SEQ. No. 115; and SEQ. No. 119;, which encode fusion proteins having amino acid sequences corresponding to amino acid sequences selected from the group consisting of amino acid sequences, respectively:

SEQ. No. 4; SEQ. No. 5; SEQ. No. 6; SEQ. No. 11; SEQ. No. 12; SEQ. No. 13; SEQ. No. 14; SEQ. No. 15; SEQ. No. 16; SEQ. No. 18; SEQ. No. 19; SEQ. No. 20; SEQ. No. 21; SEQ. No. 22; SEQ. No. 23; SEQ. No. 30; SEQ. No. 31; SEQ. No. 32; SEQ. No. 91; SEQ. No. 92; SEQ. No. 93; SEQ. No. 94; SEQ. No. 95; SEQ. No. 96; SEQ. No. 97, SEQ. No. 98; and SEQ. No. 102.

In a preferred embodiment, the invention provides also an expression vector suitable for transformation of E. coli, comprising the polynucleotide sequence selected from the group of polynucleotide sequences SEQ. Nos 60-62, 67-72, 74-79, 86-88, 108-115, and 119_ indicated above, as well as E. coli cell transformed with such an expression vector.

Transformation, i.e. introduction of a DNA sequence into bacterial host cells, particularly *E. coli,* is usually performed on the competent cells, prepared to take up the DNA for example by treatment with calcium ions at low temperature (4°C), and then subjecting to the heat-shock (at 37-42°C) or by electroporation. Such techniques are well known and are usually determined by the manufacturer of the expression system or are described in the literature and manuals for laboratory work, such as Maniatis et al., Molecular Cloning. Cold Spring Harbor, N.Y., 1982).

The procedure of overexpression of fusion proteins of the invention in *E. coli* expression system will be further described below.

The invention also provides a pharmaceutical composition containing the fusion protein of the invention as defined above as an active ingredient and a suitable pharmaceutically acceptable carrier, diluent and conventional auxiliary components. The pharmaceutical composition will contain an effective amount of the fusion protein of the invention and pharmaceutically acceptable auxiliary components dissolved or dispersed in a carrier or diluent, and preferably will be in the form of a pharmaceutical composition formulated in a unit dosage form or formulation containing a plurality of doses. Pharmaceutical forms and methods of their formulation as well as other components, carriers and diluents are known to the skilled person and described in the literature. For example, they are described in the monograph Remington's Pharmaceutical Sciences, ed. 20, 2000, Mack Publishing Company, Easton, USA.

The terms "pharmaceutically acceptable carrier, diluent, and auxiliary ingredient" comprise any solvents, dispersion media, surfactants, antioxidants, stabilizers, preservatives (e.g. antibacterial agents, antifungal agents), isotonizing agents, known in the art. The pharmaceutical composition of the invention may contain various types of carriers, diluents and excipients, depending on the chosen route of administration and desired dosage form, such as liquid, solid and aerosol forms for oral, parenteral, inhaled, topical, and whether that selected form must be sterile for administration route such as by injection. The preferred route of administration of the pharmaceutical composition according to the invention is parenteral, including injection routes such as intravenous, intramuscular, subcutaneous, intraperitoneal, intratumoral, or by single or continuous intravenous infusions.

In one embodiment, the pharmaceutical composition of the invention may be administered by injection directly to the tumor. In another embodiment, the pharmaceutical composition of the invention may be administered intravenously. In yet another embodiment, the pharmaceutical composition of the invention can be administered subcutaneously or intraperitoneally. A pharmaceutical composition for parenteral administration may be a solution or dispersion in a pharmaceutically acceptable aqueous or non-aqueous medium, buffered to an appropriate pH and isoosmotic with body fluids, if necessary, and may also contain antioxidants, buffers, bacteriostatic agents and soluble substances, which make the composition compatible with the tissues or blood of recipient. Other components, which may be included in the composition, are for example water, alcohols such as ethanol, polyols such as glycerol, propylene glycol, liquid polyethylene glycol, lipids such as triglycerides, vegetable oils, liposomes. Proper fluidity and the particles size of the substance may be provided by coating substances, such as lecithin, and surfactants, such as hydroxypropyl-celulose, polysorbates, and the like.

Suitable isotonizing agents for liquid parenteral compositions are, for example, sugars such as glucose, and sodium chloride, and combinations thereof.

Alternatively, the pharmaceutical composition for administration by injection or infusion may be in a powder form, such as a lyophilized powder for reconstitution immediately prior to use in a suitable carrier such as, for example, sterile pyrogen-free water.

The pharmaceutical composition of the invention for parenteral administration may also have the form of nasal administration, including solutions, sprays or aerosols. Preferably, the form for intranasal administration will be an aqueous solution and will be isotonic or buffered o maintain the pH from about 5.5 to about 6.5, so as to maintain a character similar to nasal secretions. Moreover, it will contain preservatives or stabilizers, such as in the well-known intranasal preparations.

The composition may contain various antioxidants which delay oxidation of one or more components. Furthermore, in order to prevent the action of microorganisms, the composition may contain various antibacterial and antifungal agents, including, for example, and not limited to, parabens, chlorobutanol, thimerosal, sorbic acid, and similar known substances of this type. In general, the pharmaceutical composition of the invention can include, for example at least about 0.01 wt. % of active ingredient. More particularly, the composition may contain the active ingredient in the amount from 1% to 75% by weight of the composition unit, or for example from 25% to 60% by weight, but not limited to the indicated values. The actual amount of the dose of the composition according to the present invention administered to patients, including man, will be determined by physical and physiological factors, such as body weight, severity of the condition, type of disease being treated, previous or concomitant therapeutic interventions, the patient and the route of administration. A suitable unit dose, the total dose and the concentration of active ingredient in the composition is to be determined by the treating physician.

The composition may for example be administered at a dose of about 1 microgram/kg of body weight to about 1000 mg/kg of body weight of the patient, for example in the range of 5 mg/kg of body weight to 100 mg/kg of body weight or in the range of 5 mg/kg of body weight to 500 mg/kg of body weight. The fusion protein and the compositions containing it exhibit anticancer or antitumor and can be used for the treatment of cancer diseases. The invention also provides the use of the fusion protein of the invention as defined above for treating cancer diseases in mammals, including humans. The invention also provides a method of treating neoplastic/cancer diseases in mammals, including humans, comprising administering to a subject in need of such treatment an *anti*-neoplastic/anticancer effective amount of the fusion protein of the invention as defined above, optionally in the form of appropriate pharmaceutical composition.

The fusion protein of the invention can be used for the treatment of hematologic malignancies such as leukaemia, granulomatosis, myeloma and other hematologic malignancies. The fusion protein can also be used for the treatment of solid tumors such as breast cancer, lung cancer, including non-small cell lung cancer, colon cancer, pancreatic cancer, ovarian cancer, bladder cancer, prostate cancer, kidney cancer, brain cancer, and the like. Appropriate route of administration of the fusion protein in the treatment of cancer will be in particular parenteral route, which consists in administering the fusion protein of the invention in the form of injections or infusions, in the composition and form appropriate for this administration route. The invention will be described in more detail in the following general procedures and examples of specific fusion proteins.

General procedure for overexpression of the fusion protein

Preparation of a plasmid

Amino acid sequence of a target fusion protein was used as a template to generate a DNA sequence encoding it, comprising codons optimized for expression in *Escherichia coli.* Such a procedure allows to increase the efficiency of further step of target protein synthesis in *Escherichia coli.* Resulting nucleotide sequence was then automatically synthesized. Additionally, the cleavage sites of restriction enzymes NdeI (at the 5'-end of leading strand) and XhoI (at the 3'-end of leading strand) were added to the resulting gene encoding the target protein. These were used to clone the gene into the vector pET28a (Novagen). They may be also be used for cloning the gene encoding the protein to other vectors. Target protein expressed from this construct can be optionally equipped at the N-terminus with a polyhistidine tag (six histidines), preceded by a site recognized by thrombin, which subsequently serves to its purification via affinity chromatography. Some targets were expressed without any tag, in particular without histidine tag, and those were subsequently purified on SP Sepharose. The correctness of the resulting construct was confirmed firstly by restriction analysis of isolated plasmids using the enzymes NdeI and XhoI, followed by automatic sequencing of the entire reading frame of the target protein. The primers used for sequencing were complementary to the sequences of T7 promoter (5'-TAATACGACTCACTATAGG-3') and T7 terminator (5'-GCTAGTTATTGCTCAGCGG-3') present in the vector. Resulting plasmid was used for overexpression of the target fusion protein in a commercial *E. coli* strain, which was transformed according to the manufacturer's recommendations. Colonies obtained on the selection medium (LB agar, kanamycin 50 μg/ml, 1% glucose) were used for preparing an overnight culture in LB liquid medium supplemented with kanamycin (50 μg/ml) and 1% glucose. After about 15h of growth in shaking incubator, the cultures were used to inoculate the appropriate culture.

Overexpression and purification of fusion proteins - general procedure A

LB medium with kanamycin (30 μg/ml) and 100 μM zinc sulfate was inoculated with overnight culture. The culture was incubated at 37°C until the optical density (OD) at 600 nm reached 0.60-0.80. Then IPTG was added to the final concentration in the range of 0.25 -1 mM. After incubation (3.5 - 20h) with shaking at 25°C the culture was centrifuged for 25 min at 6,000 g. Bacterial pellets were resuspended in a buffer containing 50 mM $KH_2PO_4$, 0.5 M NaCl, 10 mM imidazole, pH 7.4. The suspension was sonicated on ice for 8 minutes (40% amplitude, 15-second pulse, 10 s interval). The resulting extract was clarified by centrifugation for 40 minutes at 20000 g, 4°C. Ni-Sepharose (GE Healthcare) resin was pre-treated by equilibration with buffer, which was used for preparation of the bacterial cells extract. The resin was then incubated overnight at 4°C with the supernatant obtained after centrifugation of the extract. Then it was loaded into chromatography column and washed with 15 to 50 volumes of buffer 50 mM $KH_2PO_4$, 0.5 M NaCl, 20 mM imidazole, pH 7.4. The obtained protein was eluted from the column using imidazole gradient in 50 mM $KH_2PO_4$ buffer with 0.5 M NaCl, pH 7.4. Obtained fractions were analyzed by SDS-PAGE. Appropriate fractions were combined and dialyzed overnight at 4°C against 50 mM Tris buffer, pH 7.2, 150 mM NaCl, 500 mM L-arginine, 0.1 mM $ZnSO_4$, 0.01% Tween 20, and at the same time Histag, if present, was cleaved with thrombin (1:50). After the cleavage, thrombin was separated from the target fusion protein expressed with His tag by purification using Benzamidine SepharoseTM resin. Purification of target fusion proteins expressed without Histag was performed on SP Sepharose. The purity of the product was analyzed by SDS-PAGE electrophoresis (Maniatis et al, Molecular Cloning. Cold Spring Harbor, NY, 1982).

Overexpression and purification of fusion proteins - general procedure B

LB medium with kanamycin (30 μg/ml) and 100 μM zinc sulfate was inoculated with overnight culture. Cultures were incubated at 37°C until optical density (OD) at 600 nm reached 0.60-0.80. Then IPTG was added to the final concentration in the range 0.5 -1 mM. After 20h incubation with shaking at 25° C the culture was centrifuged for 25 min at 6000 g. Bacterial cells after overexpression were disrupted in a French Press in a buffer containing 50 mM $KH_2PO_4$, 0.5 M NaCl, 10 mM imidazole, 5mM beta-mercaptoethanol, 0.5mM PMSF (phenylmethylsulphonyl fluoride), pH 7.8. Resulting extract was clarified by centrifugation for 50 minutes at 8000 g. The Ni-Sepharose resin was incubated overnight with the obtained supernatant. Then the resin with bound protein was packed into the chromatography column. To wash-out the fractions containing non-binding proteins, the column was washed with 15 to 50 volumes of buffer 50 mM $KH_2PO_4$, 0.5 M NaCl, 10 mM imidazole, 5mM beta-mercaptoethanol, 0.5mM PMSF (phenylmethylsulphonyl fluoride), pH 7.8. Then, to wash-out the majority of proteins binding specifically with the bed, the column was washed with a buffer containing 50 mM $KH_2PO_4$, 0.5 M NaCl, 500 mM imidazole, 10% glycerol, 0.5 mM PMSF, pH 7.5. Obtained fractions were analyzed by SDS-PAGE (Maniatis et al, Molecular Cloning. Cold Spring Harbor, NY, 1982). The fractions containing the target protein were combined and, if the protein was expressed with histidine tag, cleaved with thrombin (1U per 4 mg of protein, 8h at 16°C) to remove polyhistidine tag. Then the fractions were dialyzed against formulation buffer (500 mM L-arginine, 50 mM Tris, 2.5 mM $ZnSO_4$, pH 7.4).

In this description Examples of proteins originally expressed with histidine tag that was subsequently removed are designated with superscript a) next to the Example number. Proteins that were originally expressed without histidine tag are designated with superscript b) next to the Example number.

Characterization of fusion proteins by 2-D electrophoresis

In order to further characterize obtained proteins and to select precisely chromatographic conditions, isoelectric points of the proteins were determined. For this purpose, two-dimensional electrophoresis (2-D) method was used, in two stages according to the following schedule.

Step 1. Isoelectrofocusing of proteins in a pH gradient and denaturing conditions.

Protein preparations at concentrations of 1 - 2 mg/ml were precipitated by mixing in a 1:1 ratio with a precipitation solution containing 10% trichloroacetic acid and 0.07% beta-mercaptoethanol in acetone. The mixture was incubated for 30 min at -20°C and then centrifuged for 25 min at 15,000 g and 4°C. The supernatant was removed and the pellet was washed twice with cold acetone with 0.07% beta-mercaptoethanol. Then the residues of acetone were evaporated until no detectable odour. The protein pellet was suspended in 250 ml of rehydration buffer 8M urea, 1% CHAPS, 15 mM DTT, 0.5% ampholyte (GE Healthcare) with a profile of pH 3-11 or 6-11, depending on the strip subsequently used. The protein solution was placed in a ceramic chamber for isoelectrofocusing, followed by 13 cm DryStrip (GE Healthcare) with appropriate pH profile (3-11 or 6-11). The whole was covered with a layer of mineral oil. The chambers were placed in the Ettan IPGphor III apparatus, where isoelectrofocusing was conducted according to the following program assigned to the dimensions of the strip and the pH profile:

16h dehydration at 20° C.

Focusing in the electric field at a fixed pH gradient

| Time | Voltage |
|---|---|
| 1h | 500 V |
| 1h | gradient 500 - 1000 V |
| 2h 30min | gradient 1000 - 8000 V |
| 30 min | 8000 V |

Then, the strip containing the focused proteins was washed for 1 min in deionised water, stained with Coomassie Brilliant and then decolorized and archived as an image to mark the location of proteins. Discoloured strip was equilibrated 2 x 15 min with a buffer of the following composition: 50mM Tris-HCl pH 8.8, 6M urea, 1% DTT, 2% SDS, 30% glycerol.

Step 2. Separation in a second direction by SDS-PAGE.

The strip was placed over the 12.5% polyacrylamide gel containing a single well per standard size and then separation was performed in an apparatus for SDS-PAGE, at a voltage of 200V for 3 hours. The gel was stained with Coomassie Brilliant then archived with the applied scale. Proteins were identified by determining its weight on the basis of the standard of size, and its IPI was read for the scale of 6-11 on the basis of the curves provided by the manufacturer (GE Healthcare) (ratio of pH to % of length of the strip from the end marked as anode) or a scale of 3-11 on the basis of the curve determined experimentally by means of isoelectrofocusing calibration kit (GE Healthcare).

Examples

The representative examples of the fusion proteins of the invention are shown in the following Examples.

In the examples the amino acids sequences of fusion proteins are written from N-terminus to C-terminus of the protein. In the Examples, by TRAIL is always meant hTRAIL.

The following designations of amino acids sequences components are used, wherein next to the three-letter designation, the equivalent single-letter designation is given.

LINKER1: steric linker Gly Gly /GG

LINKER2: steric linker Gly Gly Gly / GGG

LINKER3: steric linker Gly Ser Gly /GSG

LINKER4: steric linker Gly Gly Gly Gly Ser/GGGGS

LINKER5: steric linker Gly Gly Gly Gly Gly Ser/GGGGGS

LINKER6: steric linker Gly Gly Ser Gly Gly/GGSGG

LINKER7: steric linker Gly Gly Gly Ser Gly Gly Gly/ GGGSGGG

LINKER8: steric linker Gly Gly Gly Gly Ser Gly /GGGGSG

LINKER9: steric linker Gly Gly Gly Ser Gly Gly Gly Gly Gly Ser/ GGGSGGGGGS

LINKER10: steric linker Gly Gly Gly Gly Ser Gly Gly Gly Gly / GGGGSGGGG

LINKER11: steric linker Gly Ser Gly Gly Gly Ser Gly Gly Gly/ GSGGGSGGG

LINKER12: steric linker Cys Ala Ala Cys Ala Ala Ala Cys/ CAACAAAC

LINKER13: steric linker Cys Ala Ala Ala Cys Ala Ala Cys/ CAAACAAC

LINKER 14: steric linker Cys/C

LINKER 15: steric linker Gly/G

LINKER16: steric linker Ser Gly Gly/SGG

FURIN: sequence cleaved by furin Arg Lys Lys Arg / RKKR

FURIN.NAT: native sequence cleaved by furin His Arg Gln Pro Arg Gly Trp Glu Gln / HRQPRGWEQ

UROKIN: sequence cleaved by urokinase Arg Val Val Arg / RVVR

MMP: sequence cleaved by metalloprotease Pro Leu Gly Leu Ala Gly/ PLGLAG

PEG1: pegylation linker Ala Ser Gly Cys Gly Pro Glu/ASGCGPE

PEG2: pegylation linker Ala Ser Gly Cys Gly Pro Glu Gly/ ASGCGPEG

TRANS1: transporting sequence His His His His His His /HHHHHH

TRANS2: transporting sequence Arg Arg Arg Arg Arg Arg Arg/RRRRRRR

TRANS3: transporting sequence Arg Arg Arg Arg Arg Arg Arg Arg/RRRRRRRR

TRANS4: transporting sequence Tyr Ala Arg Ala Ala Ala Arg Gln Ala Arg Ala /YARAAARQARA

TRANS5: transporting sequence Thr His Arg Pro Pro Met Trp Ser Pro Val Trp Pro /THRPPMWSPVWP

TRANS6: transporting sequence Tyr Gly Arg Lys Lys Arg Arg Gln Arg Arg Arg /YGRKKRRQRRR

Example 4. Fusion protein of SEQ. No. 4

The protein of SEQ. No. 4 is a fusion protein having the length of 227 amino acids and the mass of 25.7 kDa, wherein domain (a) is the sequence of TRAIL121-281, and domain (b) of the effector peptide is the 56-amino acids pilosulin-1 (SEQ. No. 36) attached at the N-terminus of domain (a).
Additionally, between domain (b) and domain (a) there are sequentially incorporated urokinase cleavage site (RVVR) and metalloprotease cleavage site (PLGLAG). Thus, the structure of the fusion protein of the invention is as follows:

(SEQ. No. 36)-UROKIN-MMP-(TRAIL 121-281)

The amino acid sequence and the DNA encoding sequence comprising codons optimized for expression in E. coli are, respectively SEQ. No. 4 and SEQ. No. 60, as shown in the attached Sequence Listing.
The amino acid sequence SEQ. No. 4 of the structure described above was used as a template to generate its coding DNA sequence SEQ. No. 60. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed with histidine tag.

Example 5. Fusion protein of SEQ. No. 5

The protein of SEQ. No. 5 is a fusion protein having the length of 264 amino acids and the mass of 29.5kDa, wherein domain (a) is the sequence of TRAIL95-281, and domain (b) of the effector peptide is the 56-amino acids pilosulin-1 (SEQ. No. 36), and is attached at the C-terminus of domain (a).
Additionally, between domain (a) and domain (b) there are sequentially incorporated steric linker (CAACAAC), steric linker (GGG), metalloprotease cleavage site (PLGLAG) and urokinase cleavage site (RVVR). Thus, the structure of the fusion protein of the invention is as follows:

(TRAIL95-281)-LINKER12-LINKER2-MMP-UROKIN-(SEQ. No. 36)

The amino acid sequence and the DNA encoding sequence comprising codons optimized for expression in E. coli are, respectively SEQ. No. 5 and SEQ. No. 61 as shown in the attached Sequence Listing.
The amino acid sequence SEQ. No. 5 of the structure described above was used as a template to generate its coding DNA sequence SEQ. No. 61. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed with histidine tag.

Example 6. Fusion protein of SEQ. No. 6

The protein of SEQ. No. 6 is a fusion protein having the length of 299 amino acids and the mass of 33.2 kDa, wherein domain (a) is the sequence of TRAIL95-281, and domain (b) of the effector peptide is the 90-amino acids peptide pilosulin 5 (SEQ. No. 37) attached at the C-terminus of domain (a).
Additionally, between domain (a) and domain (b) there are sequentially incorporated steric linker (GSG), steric linker (CAACAAAC), metalloprotease cleavage site (PLGLAG), urokinase cleavage site (RWR) and steric linker (G). Thus, the structure of the fusion protein of the invention is as follows:

(TRAIL95-281)-LINKER3-LINKER12-MMP-UROKIN-LINKER15-(SEQ. No. 37)

The amino acid sequence and the DNA encoding sequence comprising codons optimized for expression in E. coli are, respectively SEQ. No. 6 and SEQ. No. 62 as shown in the attached Sequence Listing.
The amino acid sequence SEQ. No. 6 of the structure described above was used as a template to generate its coding DNA sequence SEQ. No. 62. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed both with histidine tag (Ex. 6[a]) and without histidine tag (Ex. 6[b]).

Example 11. Fusion protein of SEQ. No. 11

The protein of SEQ. No. 11 is a fusion protein having the length of 202 amino acids and the mass of 23 kDa, wherein domain (a) is the sequence of TRAIL 121-281, and domain (b) of the effector peptide is the synthetic 14-amino acids lytic peptide (SEQ. No. 41) attached at the C-terminus of domain (a).

Additionally, between domain (a) and domain (b) there are sequentially incorporated steric linker (GGGGSGGGG), urokinase cleavage site (RWR), metalloprotease cleavage site (PLGLAG) and 8-arginine transporting sequence (RRRRRRRR). Thus, the structure of the fusion protein of the invention is as follows:

(TRAIL121-281)-LINKER10-UROKIN-MMP-TRANS3-(SEQ. No. 41)

The amino acid sequence and the DNA encoding sequence comprising codons optimized for expression in E. coli are, respectively SEQ. No. 11 and SEQ. No. 67 as shown in the attached Sequence Listing.

The amino acid sequence SEQ. No. 11 of the structure described above was used as a template to generate its coding DNA sequence SEQ. No. 67. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed both with histidine tag (Ex. 11[a]) and without histidine tag (Ex. 11[b]).

Example 12. Fusion protein of SEQ. No. 12

The protein of SEQ. No. 12 is a fusion protein having the length of 205 amino acids and the mass of 23,2 kDa, wherein domain (a) is the sequence of TRAIL 121-281, and domain (b) of the effector peptide is the synthetic 14-amino acids lytic peptide (SEQ. No. 41) attached at the C-terminus of domain (a).

Additionally, between domain (a) and domain (b) there are sequentially incorporated steric linker (GG), sequence of pegylation linker (ASGCGPEG), steric linker sequence (GGG), metalloprotease cleavage site (PLGLAG), urokinase cleavage site (RWR) and polyarginine transporting sequence (RRRRRRR). Thus, the structure of the fusion protein of the invention is as follows:

(TRAIL 121-281)-LINKER1-PEG2-LINKER2-MMP-UROKIN-TRANS2-(SEQ. No. 41)

The amino acid sequence and the DNA encoding sequence comprising codons optimized for expression in E. coli are, respectively SEQ. No. 12 and SEQ. No. 68 as shown in the attached Sequence Listing.

The amino acid sequence SEQ. No. 12 of the structure described above was used as a template to generate its coding DNA sequence SEQ. No. 68. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed with histidine tag.

Example 13. Fusion protein of SEQ. No. 13

The protein of SEQ. No. 13 is a fusion protein having the length of 228 amino acids and the mass of 25.9 kDa, wherein domain (a) is the sequence of TRAIL 95-281, and domain (b) of the effector peptide is the 14-amino acids lytic peptide (SEQ. No. 41) attached at the C-terminus of domain (a).

Additionally, between domain (a) and domain (b) there are sequentially incorporated steric linker (GGGGSGGGG), urokinase cleavage site (RVVR), metalloprotease cleavage site (PLGLAG) and 8-arginine transporting sequence (RRRRRRRR). Thus, the structure of the fusion protein of the invention is as follows:

(TRAIL 95-281)-LINKER10-UROKIN-MMP-TRANS3-(SEQ. No. 41)

The amino acid sequence and the DNA encoding sequence comprising codons optimized for expression in E. coli are, respectively SEQ. No. 13 and SEQ. No. 69 as shown in the attached Sequence Listing.

The amino acid sequence SEQ. No. 13 of the structure described above was used as a template to generate its coding DNA sequence SEQ. No. 69. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed both with histidine tag (Ex. 13[a]) and without histidine tag (Ex. 13[b]).

Example 14. Fusion protein of SEQ. No. 14

The protein of SEQ. No. 14 is a fusion protein having the length of 192 amino acids and the mass of 22.1 kDa, wherein domain (a) is the sequence of TRAIL 121-281, and domain (b) of the effector peptide is the synthetic 14-amino acids lytic peptide (SEQ. No. 41) attached at the N-terminus of domain (a).

Additionally, between domain (b) and domain (a) there are sequentially incorporated steric linker (C), urokinase cleavage site (RWR) and metalloprotease cleavage site (PLGLAG). Additionally at the N-terminus of effector peptide is attached polyhistidine transporting domain (HHHHHH). Thus, the structure of the fusion protein of the invention is as follows:

TRANS1-(SEQ. No. 41)-LINKER14-UROKIN-MMP-(TRAIL 121-281)

The amino acid sequence and the DNA encoding sequence comprising codons optimized for expression in E. coli are, respectively SEQ. No. 14 and SEQ. No. 70 as shown in the attached Sequence Listing.

The amino acid sequence SEQ. No. 14 of the structure described above was used as a template to generate its coding DNA sequence SEQ. No. 70. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed with histidine tag.

Example 15. Fusion protein of SEQ. No. 15

The protein of SEQ. No. 15 is a fusion protein having the length of 200 amino acids and the mass of 23.3 kDa, wherein domain (a) is the sequence of TRAIL 121-281, and domain (b) of the effector peptide is the 14-amino acids lytic peptide (SEQ. No. 41) is attached at the N-terminus of domain (a).

Additionally, between domain (b) and domain (a) there are sequentially incorporated steric linker (C), 8-arginine transporting sequence (RRRRRRRR), urokinase cleavage site (RWR) and metalloprotease cleavage site (PLGLAG). Additionally, histidine transporting sequence (HHHHHH) is attached at the N-terminus of the effector peptide. Thus, the structure of the fusion protein of the invention is as follows:

TRANS1-(SEQ. No. 41)-LINKER14-TRANS3-UROKIN-MMP-(TRAIL 121-281)

The amino acid sequence and the DNA encoding sequence comprising codons optimized for expression in E. coli are, respectively SEQ. No. 15 and SEQ. No. 71 as shown in the attached Sequence Listing.

The amino acid sequence SEQ. No. 15 of the structure described above was used as a template to generate its coding DNA sequence SEQ. No. 71. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed with histidine tag.

Example 16. Fusion protein of SEQ. No. 16

The protein of SEQ. No. 16 is a fusion protein having the length of 202 amino acids and the mass of 23,1 kDa, wherein domain (a) is the sequence of TRAIL 121-281, and domain (b) of the effector peptide is the synthetic 14-amino acids lytic peptide (SEQ. No. 41) attached at the C-terminus of domain (a).

Additionally, between domain (a) and domain (b) there are sequentially incorporated steric linker (GGGGSGGGG), metalloprotease cleavage site (PLGLAG), urokinase cleavage site (RWR) and 8-arginine transporting sequence (RRRRRRRR). Thus, the structure of the fusion protein of the invention is as follows:

(TRAIL 121-281)-LINKER10-MMP-UROKIN TRANS3-(SEQ. No. 41)

The amino acid sequence and the DNA encoding sequence comprising codons optimized for expression in E. coli are, respectively SEQ. No. 16 and SEQ. No. 72 as shown in the attached Sequence Listing.
The amino acid sequence SEQ. No. 16 of the structure described above was used as a template to generate its coding DNA sequence SEQ. No. 72. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed both with histidine tag (Ex. 16[a]) and without histidine tag (Ex. 16[b]).

Example 18. Fusion protein of SEQ. No. 18

The protein of SEQ. No. 18 is a fusion protein having the length of 203 amino acids and the mass of 23.6 kDa, wherein domain (a) is the sequence of TRAIL 116-281, and domain (b) of the effector peptide is the 27-amino acids peptide hCAP-18/LL-37 (SEQ. No. 43) attached at the N-terminus of domain (a).
Additionally between domain (b) and domain (a) there are sequentially incorporated urokinase cleavage site (RWR) and metalloprotease cleavage site (PLGLAG). Thus, the structure of the fusion protein of the invention is as follows:

(SEQ. No. 43)-UROKIN-MMP-(TRAIL116-281)

The amino acid sequence and the DNA encoding sequence comprising codons optimized for expression in E. coli are, respectively SEQ. No. 18 and SEQ. No. 74 as shown in the attached Sequence Listing.
The amino acid sequence SEQ. No. 18 of the structure described above was used as a template to generate its coding DNA sequence SEQ. No. 74. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed with histidine tag.

Example 19. Fusion protein of SEQ. No. 19

The protein of SEQ. No. 19 is a fusion protein having the length of 203 amino acids and the mass of 23,3 kDa, wherein domain (a) is the sequence of TRAIL 116-281, and domain (b) of the effector peptide is the 27-amino acids peptide BAMP-28 (SEQ. No. 44) attached at the N-terminus of domain (a).
Additionally, between domain (b) and domain (a) there are sequentially incorporated urokinase cleavage site (RWR) and metalloprotease cleavage site (PLGLAG). Thus, the structure of the fusion protein of the invention is as follows:

(SEQ. No. 44)-UROKIN-MMP-(TRAIL116-281)

The amino acid sequence and the DNA encoding sequence comprising codons optimized for expression in E. coli are, respectively SEQ. No. 19 and SEQ. No. 75 as shown in the attached Sequence Listing.
The amino acid sequence SEQ. No. 19 of the structure described above was used as a template to generate its coding DNA sequence SEQ. No. 75. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed with histidine tag.

Example 20. Fusion protein of SEQ. No. 20

The protein of SEQ. No. 20 is a fusion protein having the length of 200 amino acids and the mass of 22.8 kDa, wherein domain (a) is the sequence of TRAIL 121-281, and domain (b) of the effector peptide is the 24-amino acids analogue of isoform C of the lytic peptide from *Entamoeba histolytica* (SEQ. No. 45) attached at the C-terminus of domain (a).
Additionally, between domain (a) and domain (b) there are sequentially incorporated steric linker (GGSGG), metalloprotease cleavage site (PLGLAG) and urokinase cleavage site (RWR). Thus, the structure of the fusion protein of the invention is as follows:

(TRAIL121-281)-LINKER6-MMP-UROKIN-(SEQ. No. 45)

The amino acid sequence and the DNA encoding sequence comprising codons optimized for expression in E. coli are, respectively SEQ. No. 20 and SEQ. No. 76 as shown in the attached Sequence Listing.

The amino acid sequence SEQ. No. 20 of the structure described above was used as a template to generate its coding DNA sequence SEQ. No. 76. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed with histidine tag.

Example 21. Fusion protein of SEQ. No. 20

The protein of SEQ. No. 20 is a fusion protein having the length of 200 amino acids and the mass of 22.8 kDa, wherein domain (a) is the sequence of TRAIL 121-281, and domain (b) of the effector peptide is the 24-amino acids analogue of isoform A of the lytic peptide from *Entamoeba histolytica* (SEQ. No. 46) attached at the C-terminus of domain (a).

Additionally, between domain (a) and domain (b) there are sequentially incorporated steric linker (GGSGG), metalloprotease cleavage site (PLGLAG) and urokinase cleavage site (RVVR). Thus, the structure of the fusion protein of the invention is as follows:

(TRAIL121-281)-LINKER6-MMP-UROKIN-(SEQ. No. 46)

The amino acid sequence and the DNA encoding sequence comprising codons optimized for expression in E. coli are, respectively SEQ. No. 21 and SEQ. No. 77 as shown in the attached Sequence Listing.

The amino acid sequence SEQ. No. 21 of the structure described above was used as a template to generate its coding DNA sequence SEQ. No. 77. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed with histidine tag.

Example 22. Fusion protein of SEQ. No. 22

The protein of SEQ. No. 22 is a fusion protein having the length of 200 amino acids and the mass of 22.8 kDa, wherein domain (a) is the sequence of TRAIL 121-281, and domain (b) of the effector peptide is the 24-amino acids analogue of isoform B of a lytic peptide from *Entamoeba histolytica* (SEQ. No. 47) attached at the C-terminus of domain (a).

Additionally, between domain (a) and domain (b) there are sequentially incorporated steric linker (GGSGG), metalloprotease cleavage site (PLGLAG) and urokinase cleavage site (RVVR). Thus, the structure of the fusion protein of the invention is as follows:

(TRAIL121-281)-LINKER6-MMP-UROKIN-(SEQ. No. 47)

The amino acid sequence and the DNA encoding sequence comprising codons optimized for expression in E. coli are, respectively SEQ. No. 22 and SEQ. No. 78 as shown in the attached Sequence Listing.

The amino acid sequence SEQ. No. 22 of the structure described above was used as a template to generate its coding DNA sequence SEQ. No. 78. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed with histidine tag.

Example 23. Fusion protein of SEQ. No. 23

The protein of SEQ. No. 23 is a fusion protein having the length of 190 amino acids and the mass of 22.1 kDa, wherein domain (a) is the sequence of TRAIL 121-281, and domain (b) of the effector peptide is the 12-amino acids fragment of HA2 domain of influenza virus haemagglutinin (SEQ. No. 48) attached at the N-terminus of domain (a).

Additionally, between domain (b) and domain (a) there are sequentially incorporated 7-arginine transporting sequence (RRRRRRR), urokinase cleavage site (RWR) and metalloprotease cleavage site (PLGLAG). Thus, the structure of the fusion protein of the invention is as follows:

(SEQ. No. 48)-TRANS2-UROKIN-MMP-(TRAIL121-281)

The amino acid sequence and the DNA encoding sequence comprising codons optimized for expression in E. coli are, respectively SEQ. No. 23 and SEQ. No. 79 as shown in the attached Sequence Listing.

The amino acid sequence SEQ. No. 23 of the structure described above was used as a template to generate its coding DNA sequence SEQ. No. 79. A plasmid containing the coding sequence of DNA was generated and overexpression of the fusion protein was carried out in accordance with the general procedures described above. Overexpression was performed according to the general procedure B, using *E. coli BL21 (DE3)* or *Tuner (DE3)* strain from Novagen. The protein was separated by electrophoresis in accordance with the general procedure described above. The protein was expressed both with histidine tag (Ex. 23[a]) and without histidine tag (Ex. 23[b]).

Example 30. Fusion protein of SEQ. No. 30

The protein of SEQ. No. 30 is a fusion protein having the length of 200 amino acids and the mass of 22.5 kDa, wherein domain (a) is the sequence of TRAIL 121-281, and domain (b) of the effector peptide is the 33-amino acids active fragment of human perforin (SEQ. No. 54) attached at the C-terminus of domain (a).

Additionally, between domain (a) and domain (b) is incorporated steric linker sequence (GGGGSG). Thus, the structure of the fusion protein of the invention is as follows:

(TRAIL121-281)-LINKER8-(SEQ. No. 54)

The amino acid sequence and the DNA encoding sequence comprising codons optimized for expression in E. coli are, respectively SEQ. No. 30 and SEQ. No. 86 as shown in the attached Sequence Listing.

The amino acid sequence SEQ. No. 30 of the structure described above was used as a template to generate its coding DNA sequence SEQ. No. 86. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed with histidine tag.

Example 31. Fusion protein of SEQ. No. 31

The protein of SEQ. No. 31 is a fusion protein having the length of 210 amino acids and the mass of 23.5 kDa, wherein domain (a) is the sequence of TRAIL 121-281, and domain (b) of the effector peptide is the 33-amino acids active fragment of human perforin (SEQ. No. 54) attached at the C-terminus of domain (a).

Additionally, between domain (a) and domain (b) there are sequentially incorporated steric linker (GGGGSG), urokinase cleavage site (RVVR), AND metalloprotease cleavage site (PLGLAG). Thus, the structure of the fusion protein of the invention is as follows:

(TRAIL121-281)-LINKER8-UROKIN-MMP-(SEQ. No.54)

The amino acid sequence and the DNA encoding sequence comprising codons optimized for expression in E. coli are, respectively SEQ. No. 31 and SEQ. No. 87 as shown in the attached Sequence Listing.

The amino acid sequence SEQ. No. 31 of the structure described above was used as a template to generate its coding DNA sequence SEQ. No. 87. A plasmid containing the coding sequence of DNA was generated and overexpression of the fusion protein was carried out in accordance with the general procedures described above. Overexpression was performed according to the general procedure B, using *E. coli BL21 (DE3)* or *Tuner (DE3)* strain from Novagen. The protein was separated by electrophoresis in accordance with the general procedure described above. The protein was expressed with histidine tag.

Example 32. Fusion protein of SEQ. No. 32

The protein of SEQ. No. 32 is a fusion protein having the length of 436 amino acids and the mass of 48 kDa, wherein domain (a) is the sequence of TRAIL 116-281, and domain (b) of the effector peptide is the 251-amino acids parasporin-2 from *Bacillus thuringensis* (SEQ. No. 55) attached at the N-terminus of domain (a).

Additionally, between domain (b) and domain (a) there are sequentially incorporated urokinase cleavage site (RVVR), metalloprotease cleavage site (PLGLAG) and steric linker (GSGGGSGGG). Thus, the structure of the fusion protein of the invention is as follows:

(SEQ. No. 55)-UROKIN-MMP-LINKER11-(TRAIL116-281)

The amino acid sequence and the DNA encoding sequence comprising codons optimized for expression in E. coli are, respectively SEQ. No. 32 and SEQ. No. 88 as shown in the attached Sequence Listing.

The amino acid sequence SEQ. No. 32 of the structure described above was used as a template to generate its coding DNA sequence SEQ. No. 88. A plasmid containing the coding sequence of DNA was generated and overexpression of the fusion protein was carried out in accordance with the general procedures described above. Overexpression was performed according to the general procedure B, using *E. coli BL21 (DE3)* or *Tuner (DE3)* strain from Novagen. The protein was separated by electrophoresis in accordance with the general procedure described above. The protein was expressed with histidine tag.

Example 34. Fusion protein of SEQ. No. 91

The protein of SEQ. No. 91 is a fusion protein having the length of 223 amino acids and the mass of 25.2 kDa, wherein domain (a) is the sequence of TRAIL 121-281, and domain (b) of the effector peptide is the 39-amino acids fusion peptide comprising PDGFR inhibitor and synthetic lytic peptide (SEQ. No. 125), attached at the N-terminus of domain (a).

Additionally, between domain (b) and domain (a) there are sequentially incorporated urokinase cleavage site (RVVR) and metalloprotease cleavage site (PLGLAG), steric linker (GG), steric linker (CAAACAAC) and steric linker (SGG). Thus, the structure of the fusion protein of the invention is as follows:

(SEQ. No. 125)-UROKIN-MMP- LINKER1-LINKER13-LINKER16-(TRAIL121-281)

The amino acid sequence and the DNA encoding sequence comprising codons optimized for expression in E. coli are, respectively SEQ. No. 91 and SEQ. No. 108 as shown in the attached Sequence Listing.

The amino acid sequence SEQ. No. 91 of the structure described above was used as a template to generate its coding DNA sequence SEQ. No. 108. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed without histidine tag.

Example 35. Fusion protein of SEQ. No. 92

The protein of SEQ. No. 92 is a fusion protein having the length of 223 amino acids and the mass of 25.6 kDa, wherein domain (a) is the sequence of TRAIL 95-281, and domain (b) of the effector peptide is the 14-amino acids fusion synthetic lytic peptide (SEQ. No. 41), attached at the N-terminus of domain (a).

Additionally, between domain (b) and domain (a) there are sequentially incorporated polyarginine transporting domain (RRRRRRR), furin cleavage site (RKKR), steric linker (GGG),and steric linker (CAAACAAC). Thus, the structure of the fusion protein of the invention is as follows:

(SEQ. No. 41)-TRANS2-FURIN-LINKER2-LINKER13-(TRAIL95-281)

The amino acid sequence and the DNA encoding sequence comprising codons optimized for expression in E. coli are, respectively SEQ. No. 92 and SEQ. No. 109 as shown in the attached Sequence Listing.

The amino acid sequence SEQ. No. 92 of the structure described above was used as a template to generate its coding DNA sequence SEQ. No. 109. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above.

The protein was expressed without histidine tag.

Example 36. Fusion protein of SEQ. No. 93

The protein of SEQ. No. 93 is a fusion protein having the length of 232 amino acids and the mass of 26.7 kDa, wherein domain (a) is the sequence of TRAIL 95-281, and domain (b) of the effector peptide is the 14-amino acids fusion synthetic lytic peptide (SEQ. No. 41), attached at the N-terminus of domain (a). Additionally, between domain (b) and domain (a) there are sequentially incorporated polyarginine transporting domain (RRRRRRR), furin cleavage site (RKKR), native furin cleavage site (HRQPRGWEQ) steric linker (GGG),and steric linker (CAAACAAC). Thus, the structure of the fusion protein of the invention is as follows:

(SEQ. No. 41)-TRANS2-FURIN-FURN.NAT-LINKER2-LINKER13- (TRAIL95-281)

The amino acid sequence and the DNA encoding sequence comprising codons optimized for expression in E. coli are, respectively SEQ. No. 93 and SEQ. No. 110 as shown in the attached Sequence Listing.

The amino acid sequence SEQ. No. 93 of the structure described above was used as a template to generate its coding DNA sequence SEQ. No. 110. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed without histidine tag.

Example 37. Fusion protein of SEQ. No. 94

The protein of SEQ. No. 94 is a fusion protein having the length of 207 amino acids and the mass of 23 kDa, wherein domain (a) is the sequence of TRAIL 121-281, and domain (b) of the effector peptide is the 14-amino acids fusion synthetic lytic peptide (SEQ. No. 41), attached at the C-terminus of domain (a).

Additionally, between domain (a) and domain (b) there are sequentially incorporated steric linker (GGGGSGGGG), metalloprotease cleavage site (PLGLAG), urokinase cleavage site (RWR), transporting domain (YARAAARQARA) and steric linker (GG). Thus, the structure of the fusion protein of the invention is as follows:

(TRAIL121-281)-LINKER10-MMP-UROKIN-TRANS4-LINKER1-(SEQ. No. 41)

The amino acid sequence and the DNA encoding sequence comprising codons optimized for expression in E. coli are, respectively SEQ. No. 94 and SEQ. No. 111 as shown in the attached Sequence Listing.

The amino acid sequence SEQ. No. 94 of the structure described above was used as a template to generate its coding DNA sequence SEQ. No. 111. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed without histidine tag.

Example 38. Fusion protein of SEQ. No. 95

The protein of SEQ. No. 95 is a fusion protein having the length of 218 amino acids and the mass of 24.4 kDa, wherein domain (a) is the sequence of TRAIL 121-281, and domain (b) of the effector peptide is the 25-amino acids pleurocidine analogue (SEQ. No. 126), attached at the C-terminus of domain (a).

Additionally, between domain (a) and domain (b) there are sequentially incorporated steric linker (GGGGSGGGG), metalloprotease cleavage site (PLGLAG), urokinase cleavage site (RWR), transporting domain (YARAAARQARA) and steric linker (GG). Thus, the structure of the fusion protein of the invention is as follows:

(TRAIL121-281)-LINKER10-MMP-UROKIN-TRANS4-LINKER1-(SEQ. No. 126)

The amino acid sequence and the DNA encoding sequence comprising codons optimized for expression in E. coli are, respectively SEQ. No. 95 and SEQ. No. 112 as shown in the attached Sequence Listing.

The amino acid sequence SEQ. No. 95 of the structure described above was used as a template to generate its coding DNA sequence SEQ. No. 112. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed without histidine tag.

Example 39. Fusion protein of SEQ. No. 96

The protein of SEQ. No. 96 is a fusion protein having the length of 219 amino acids and the mass of 24.5 kDa, wherein domain (a) is the sequence of TRAIL 121-281, and domain (b) of the effector peptide is the 26-amino acids pleurocidine analogue (SEQ. No. 127), attached at the C-terminus of domain (a).

Additionally, between domain (a) and domain (b) there are sequentially incorporated steric linker (GGGGSGGGG), metalloprotease cleavage site (PLGLAG), urokinase cleavage site (RWR), transporting domain (YARAAARQARA) and steric linker (GG). Thus, the structure of the fusion protein of the invention is as follows:

(TRAIL121-281)-LINKER10-MMP-UROKIN-TRANS4-LINKER1-(SEQ. No. 127)

The amino acid sequence and the DNA encoding sequence comprising codons optimized for expression in E. coli are, respectively SEQ. No. 96 and SEQ. No. 113 as shown in the attached Sequence Listing.

The amino acid sequence SEQ. No. 96 of the structure described above was used as a template to generate its coding DNA sequence SEQ. No. 113. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed without histidine tag.

Example 40. Fusion protein of SEQ. No. 97

The protein of SEQ. No. 97 is a fusion protein having the length of 212 amino acids and the mass of 23.9 kDa, wherein domain (a) is the sequence of TRAIL 121-281, and domain (b) of the effector peptide is the 17-amino acids synthetic lytic peptide (SEQ. No. 128), attached at the C-terminus of domain (a).

Additionally, between domain (a) and domain (b) there are sequentially incorporated steric linker (GGGGSGGGG), metalloprotease cleavage site (PLGLAG), urokinase cleavage site (RVVR), transporting domain (THRPPMWSPVWP) and steric linker (GGG). Thus, the structure of the fusion protein of the invention is as follows:

(TRAIL121-281)-LINKER10-MMP-UROKIN-TRANS5-LINKER2-(SEQ. No. 127)

The amino acid sequence and the DNA encoding sequence comprising codons optimized for expression in E. coli are, respectively SEQ. No. 97 and SEQ. No. 114 as shown in the attached Sequence Listing.

The amino acid sequence SEQ. No. 97 of the structure described above was used as a template to generate its coding DNA sequence SEQ. No. 114. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed without histidine tag.

Example 41. Fusion protein of SEQ. No. 98

The protein of SEQ. No. 98 is a fusion protein having the length of 207 amino acids and the mass of 23.3 kDa, wherein domain (a) is the sequence of TRAIL 121-281, and domain (b) of the effector peptide is the 14-amino acids synthetic lytic peptide (SEQ. No. 41), attached at the C-terminus of domain (a).

Additionally, between domain (a) and domain (b) there are sequentially incorporated steric linker (GGGGSGGGG), metalloprotease cleavage site (PLGLAG), urokinase cleavage site (RVVR), transporting domain (YGRKKRRQRRR) and steric linker (GG). Thus, the structure of the fusion protein of the invention is as follows:

(TRAIL121-281)-LINKER10-MMP-UROKIN-TRANS6-LINKER1-(SEQ. No. 41)

The amino acid sequence and the DNA encoding sequence comprising codons optimized for expression in E. coli are, respectively SEQ. No. 98 and SEQ. No. 115 as shown in the attached Sequence Listing.

The amino acid sequence SEQ. No. 98 of the structure described above was used as a template to generate its coding DNA sequence SEQ. No. 115. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed without histidine tag.

Example 45. Fusion protein of SEQ. No. 102

The protein of SEQ. No. 102 is a fusion protein having the length of 234 amino acids and the mass of 26.2 kDa, wherein domain (a) is the sequence of TRAIL 121-281, and two domains (b) of the effector peptide are the 25-amino acids melittin peptide (SEQ. No. 132) and 14-amino acids synthetic lytic peptide (SEQ. No. 41), attached sequentially at the C-terminus of domain (a).
Additionally, between domain (a) and domain (b) there are sequentially incorporated steric linker (GGGGSGGGG), metalloprotease cleavage site (PLGLAG), and urokinase cleavage site (RVVR) and steric linker (GG). Additionally a steric linker (GGGGS) is incorporated between two effector domains.
Thus, the structure of the fusion protein of the invention is as follows:

(TRAIL121-281)-LINKER10-MMP-UROKIN-LINKER1-(SEQ.No.132)-LINKER4-(SEQ.No. 41)

The amino acid sequence and the DNA encoding sequence comprising codons optimized for expression in E. coli are, respectively SEQ. No. 102 and SEQ. No. 119 as shown in the attached Sequence Listing.
The amino acid sequence SEQ. No. 102 of the structure described above was used as a template to generate its coding DNA sequence SEQ. No. 119. A plasmid containing the coding sequence of DNA was generated and overexpression of the fusion protein was carried out in accordance with the general procedures described above. Overexpression was performed according to the general procedure A, using *E. coli Tuner (DE3)* strain from Novagen. The protein was separated by electrophoresis in accordance with the general procedure described above. The protein was expressed without histidine tag.

Example 51. Examination of anti-tumor activity of the fusion proteins

Examination of anti-tumor activity of the fusion proteins was carried out *in vitro* in a cytotoxicity assay on tumor cell lines and *in vivo* in mice. For comparison purposes, rhTRAIL114-281 protein and placebo were used.

1. <u>Measurement of circular dichroism:</u> determination of secondary structures composition of the obtained proteins

Quality of the preparations of fusion proteins in terms of their structures was determined by circular dichroism for the fusion proteins of Ex. 23, Ex. 11, and Ex. 13.
Circular dichroism is used for determination of secondary structures and conformation of proteins. CD method uses optical activity of the protein structures, manifested in rotating the plane of polarization of light and the appearance of elliptical polarization. CD spectrum of proteins in far ultraviolet (UV) provides precise data on the conformation of the main polypeptide chain.

<u>Dialysis</u>

Samples of the protein to be analysed, after formulation into a buffer consisting of 50 mM Tris-HCl pH 8.0, 100 mM NaCl, 10% glycerol, 0.1 mM $ZnCl_2$, 80 mM saccharose, 5mM DTT, were dialysed in dialysis bags (Sigma-Aldrich) with cut-off 12 kDa. Dialysis was performed against 100 fold excess (v/v) of buffer with respect to protein preparations, with stirring for several hours at 4°C. After dialysis was completed, each preparation was centrifuged (25 000 rpm., 10 min., 4°C) and supernatants were collected. Protein concentration in the samples thus obtained was determined by Bradford method.
Measurement of circular dichroism for proteins in the concentration range of 0.1-2.7 mg/ml was performed on Jasco J-710 spectropolarimeter, in a quartz cuvette with optical way 0.2 mm or 1 mm. The measurement was performed under the flow of nitrogen at 7 l/min, which allowed to perform the measurement in the wavelength range from 195 to 250 nm. Parameters of the measurement: spectral resolution of - 1 nm; half width of the light beam 1 nm; sensitivity 20 mdeg, the averaging time for one wavelength - 8 s, scan speed 10 nm/min.
Obtained spectra were analyzed numerically in the range of 193-250 nm using CDPro software. Points for which the voltage at the photomultiplier exceeded 700 V were omitted, due to too low signal to noise ratio in this wavelength range.
The data obtained served for calculations of particular secondary structures content in the analyzed proteins with use of CDPro software (Table 1).

Table 1. Content of secondary structures in the analyzed proteins..

| Protein | NRMSD (Exp-Cal) | α-helix | β-sheet | Schift | Disorder |
|---|---|---|---|---|---|
| Ex. 23 | 0.149 | 3.7% | 42.0% | 21.1% | 33.2% |
| Ex. 11 | 0.079 | 25.1% | 22.7% | 21.2% | 30.9% |
| Ex. 13 | 0.047 | 15.0% | 32.2% | 20.6% | 32.2% |
| hTRAIL* | | 1.94% | 50.97% | 7.74% | 39.35% |
| hTRAIL | 0.389 | 4.9% | 33.7% | 23.1% | 38.3% |
| * value obtained on the basis of crystalline structure 1 D4V<br>** values obtained on the basis of crystalline structures 1IKQ, 1 R4Q, 1ABR, 3PX8 | | | | | |

The control molecule (rhTRAIL114-281) shows CD spectrum characteristic for the proteins with predominantly type β-sheet structures (sharply outlined ellipticity minimum at the wavelength of 220 nm). This confirms the calculation of secondary structure components, suggesting a marginal number of α-helix elements.

The result obtained is also consistent with the data from the crystal structure of hTRAIL protein, and is characteristic for fusion protein of the invention of Ex. 23, wherein beta elements constitute 42% of their structure. For proteins of Ex. 11 and Ex. 13 higher alpha-helix content was observed (additional minimum of the spectrum at wavelength 208 nm). This is due to the presence in constructs of KLAKLAK motifs which have strong amphipathic character and form alpha-helical- like structures. Unfortunately, due to low stability of proteins from Ex. 23, Ex. 11 and Ex. 13 in the buffer for CD measurements and low concentrations of analyzed preparations their spectra are characterized by a high noise level and with low resolution. Therefore, they may not fully reflect the actual situation, and only suggest the result.

2. Tests on cell lines *in vitro*

Cell lines

Cell lines were obtained from ATCC and CLS, and then propagated and deposited in the Adamed's Laboratory of Biology Cell Line Bank. During the experiment, cells were routinely checked for the presence of Mycoplasma by PCR technique using the kit Venor®GeM Mycoplasma PCR Detection Kit (Minerva Biolabs, Berlin, Germany). The cultures were maintained at standard conditions: 37°C, 5% $CO_2$ (in the case of DMEM - 10% $CO_2$), and 85% relative humidity. Particular cell lines were cultured in appropriate media as recommended by ATCC.

Table 2. Adherent cells

| Cell line | Cancer type | Medium | number of cells per well (thousands) |
|---|---|---|---|
| Colo 205 ATCC #CCL-222 | *human colorectal cancer* | RPMI + 10% FBS + penicillin + streptomycin | 5 |
| HT-29 ATCC # CCL-2 | *human colorectal cancer* | McCoy's + 10% FBS + penicillin + streptomycin | 5 |
| DU-145 ATCC # HTB-81 | *human prostate cancer* | RPMI + 10% FBS + penicillin + streptomycin | 3 |
| PC-3 ATCC # CRL-1435 | *human prostate cancer* | RPMI + 10% FBS + penicillin + streptomycin | 4 |
| MCF-7 ATCC #HTB-22 | *human breast cancer* | MEM + 10% FBS + penicillin + streptomycin | 4.5 |
| MDA-MB-231 ATCC # HTB-26 | *human breast cancer* | DMEM + 10% FBS + penicillin + streptomycin | 4.5 |

(continued)

| Cell line | Cancer type | Medium | number of cells per well (thousands) |
|---|---|---|---|
| MDA-MB-435s ATCC# HTB-129 | *human breast cancer* | DMEM + 10% FBS + penicillin + streptomycin | 4 |
| UM-UC-3 ATCC # CLR-1749 | *human bladder cancer* | MEM + 10% FBS + penicillin + streptomycin | 3.5 |
| SW780 ATCC #CRL-2169 | *human bladder cancer* | DMEM + 10% FBS + penicillin + streptomycin | 3 |
| SW620 ATCC #CCL-227 | *human colorectal cancer* | DMEM + 10% FBS + penicillin + streptomycin | 5 |
| BxPC-3 ATCC #CRL-1687 | *human pancreatic cancer* | RPMI + 10% FBS + penicillin + streptomycin | 4.5 |
| SK-OV-3 ATCC # HTB-77 | *human ovarian cancer* | McCoy's + 10% FBS + penicillin + streptomycin | 4 |
| NIH: OVCAR-3 ATCC #HTB-161 | *human ovarian cancer* | RPMI + 20% FBS + 0,01 mg/ml insulina + penicillin + streptomycin | 7 |
| HepG2 ATCC # HB-8065 | *human liver hepatoma* | MEM + 10% FBS + penicillin + streptomycin | 7 |
| 293 ATCC # CLR-1573 | *Human embrional kidney cells* | MEM + 10% FBS + penicillin + streptomycin | 4 |
| ACHN ATCC #CCL-222 | *human kidney cancer* | MEM + 10% FBS + penicillin + streptomycin | 4 |
| CAKI 1 ATCC #HTB-46 | *human kidney cancer* | McCoy's + 10% FBS + penicillin + streptomycin | 3.5 |
| CAKI 2 ATCC # HTB-47 | *human kidney cancer* | McCoy's + 10% FBS + penicillin + streptomycin | 3.5 |
| NCI- H69AR ATCC #CRL-11351 | *human small cell lung cancer* | RPMI + 10% FBS + penicillin + streptomycin | 10 |
| HT144 ATCC # HTB-63 | *human melanoma cells* | McCoy's + 10% FBS + penicillin + streptomycin | 7 |
| NCI-H460 ATCC #HTB-177 | *human lung cancer* | RPMI + 10% FBS + penicillin + streptomycin | 2.5 |
| A549 ATCC # CCL-185 | *human lung cancer* | RPMI + 10% FBS + penicillin + streptomycin | 2.5 |
| MES-SA ATCC # CRL-1976 | human uterine sarcoma | McCoy's + 10% FBS + penicillin + streptomycin | 3.5 |
| MES-SA/Dx5 ATCC #CRL-1977 | multidrug-resistant human uterine sarcoma | McCoy's + 10% FBS + penicillin + streptomycin | 4 |

(continued)

| Cell line | Cancer type | Medium | number of cells per well (thousands) |
|---|---|---|---|
| MES-SA/Mx2 ATCC #CRL-2274 | human uterine sarcoma | Waymouth's MB 752/1 + McCoy's (1 : 1) + 10% FBS + penicillin + streptomycin | 4 |
| SK-MES-1 ATCC # HTB-58 | *human lung cancer* | MEM + 10% FBS + penicillin + streptomycin | 5 |
| HCT-116 ATCC # CCL-247 | *human colorectal cancer* | McCoy's + 10% FBS + penicillin + streptomycin | 3 |
| MCF10A ATCC # CRL-10317 | mammary epithelial cells | DMEM:F12 + 5% horse plasma + 0.5 μg/ml hydrocortisone + 10 μg/ml insuline + 20 ng/ml growth factor EGF | 5 |
| Panc-1 CLS 330228 | *human pancreatic cancer* | DMEM + 10% FBS + penicillin + streptomycin | 5 |
| Panc03.27 ATCC # CRL-2549 | *human pancreatic cancer* | RPMI + 10% FBS + penicillin + streptomycin | 5 |
| PLC/PRF/5 CLS 330315 | *human liver hepatoma* | DMEM + 10% FBS + penicillin + streptomycin | 5 |
| LNCaP ATCC # CRL-1740 | *human prostate cancer* | RPMI + 10% FBS + penicillin + streptomycin | 4.5 |
| SK-Hep-1 CLS300334 | *human liver hepatoma* | RPMI + 10% FBS + penicillin + streptomycin | 10 |
| A498 CLS 300113 | *human kidney cancer* | MEM + 10% FBS + penicillin + streptomycin | 3 |
| HT1080 ATCC #CCL-121 | Human fibrosarcoma | MEM + 10% FBS + penicillin + streptomycin | 3 |
| HUV-EC-C ATCC # CRL-1730 | human umbilical vein endothelial cells | M199 + 20% FBS + penicillin + 0,05 mg/ml ECGS + 0,1 mg/ml heparine + penicillin + streptomycin | 8.5 |

Table 3. Nonadherent cells:

| Cell line | Cancer type | Medium | number of cells per well (thousands) |
|---|---|---|---|
| NCI-H69 ATCC # HTB-119 | human small cell lung cancer | RPMI + 10% FBS + penicillin + streptomycin | 22 |
| Jurkat A3 ATCC #CRL-2570 | *human leukaemia* | RPMI + 10% FBS + penicillin + streptomycin | 10 |
| HL60 ATCC # CCL-240 | *human leukaemia* | RPMI + 20% FBS + penicillin + streptomycin | 10 |
| CCRF-CEM ATCC # CCL-119 | *human leukaemia* | RPMI + 20% FBS + penicillin + streptomycin | 10 |

MTT cytotoxicity test

MTT assay is a colorimetric assay used to measure proliferation, viability and cytotoxicity of cells. It consists in decomposition of a yellow tetrazolium salt MTT (4,5-dimethyl-2-thiazolyl)-2,5-diphenyltetrazolium bromide) to the water-insoluble purple dye formazan by mitochondrial enzyme succinate-tetrazolium reductase 1. MTT reduction occurs only in living cells. Data analysis consists in determining $IC_{50}$ concentration of the protein (in ng/ml), at which the 50% reduction in the number of cells occurs in the population treated compared to control cells. Results were analyzed using GraphPad Prism 5.0 software. The test was performed according to the literature descriptions (Celis, JE, (1998). Cell Biology, a Laboratory Handbook, second edition, Academic Press, San Diego; Yang, Y., Koh, LW, Tsai, JH., (2004); Involvement of viral and chemical factors with oral cancer in Taiwan, Jpn J Clin Oncol, 34 (4), 176-183).

Cell culture medium was diluted to a defined density ($10^4$ - $10^5$ cells per 100 μl). Then 100 μl of appropriately diluted cell suspension was applied to a 96-well plate in triplicates. Thus prepared cells were incubated for 24 h at 37°C in 5% or 10% $CO_2$, depending on the medium used, and then to the cells (in 100 μl of medium) further 100 μl of the medium containing various concentrations of tested proteins were added. After incubation of the cells with tested proteins over the period of next 72 hours, which is equivalent to 3-4 times of cell division, the medium with the test protein was added with 20 ml of MTT working solution [5 mg/ml], and incubation was continued for 3 h at 37°C in 5% $CO_2$. Then the medium with MTT solution was removed, and formazan crystals were dissolved by adding 100 μl of DMSO. After stirring, the absorbance was measured at 570 nm (reference filter 690 nm).

EZ4U cytotoxicity test

EZ4U (Biomedica) test was used for testing cytotoxic activity of the proteins in nonadherent cell lines. The test is a modification of the MTT method, wherein formazan formed in the reduction of tetrazolium salt is water-soluble. Cell viability study was carried out after continuous 72-hour incubation of the cells with protein (seven concentrations of protein, each in triplicates). On this basis $IC_{50}$ values were determined (as an average of two independent experiments) using the GraphPad Prism 5 software. Control cells were incubated with the solvent only.

The results of *in vitro* cytotoxicity tests are summarized as $IC_{50}$ values (ng/ml), which corresponds to the protein concentration at which the cytotoxic effect of fusion proteins is observed at the level of 50% with respect to control cells treated only with solvent. Each experiment represents the average value of at least two independent experiments performed in triplicates. As a criterion of lack of activity of protein preparations the $IC_{50}$ limit of 2000 ng/ml was adopted. Fusion proteins with an $IC_{50}$ value above 2000 were considered inactive.

Cells selected for this test included tumor cell lines that are naturally resistant to TRAIL protein (the criterion of natural resistance to TRAIL: $IC_{50}$ for TRAIL protein > 2000), as well as tumor cell lines sensitive to TRAIL protein and resistant to doxorubicin line MES-SA/DX5 as a cancer line resistant to conventional anticancer medicaments.

Undifferentiated HUVEC cell line was used as a healthy control cell line for assessment of the effect/toxicity of the fusion proteins in non-cancer cells.

The results obtained confirm the possibility of overcoming the resistance of the cell lines to TRAIL by administration of certain fusion proteins of the invention to cells naturally resistant to TRAIL. When fusion proteins of the invention were administered to the cells sensitive to TRAIL, in some cases a clear and strong potentiation of the potency of action was observed, which was manifested in reduced $IC_{50}$ values of the fusion protein compared with $IC_{50}$ for the TRAIL alone. Furthermore, cytotoxic activity of the fusion protein of the invention in the cells resistant to classical anticancer medicament doxorubicin was obtained, and in some cases it was stronger than activity of TRAIL alone.

The $IC_{50}$ values above 2000 obtained for the non-cancer cell lines show the absence of toxic effects associated with the use of proteins of the invention for healthy cells, which indicates potential low systemic toxicity of the protein.

Determination of cytotoxic activity of selected protein preparations against extended panel of tumor cell lines

Table 4 presents the results of the tests of cytotoxic activity *in vitro* for selected fusion proteins of the invention against a broad panel of tumor cells from different organs, corresponding to the broad range of most common cancers.

The experimental results are presented as a mean value ± standard deviation (SD). All calculations and graphs were prepared using the GraphPad Prism 5.0 software.

Obtained $IC_{50}$ values confirm high cytotoxic activity of fusion proteins and thus their potential utility in the treatment of cancer.

Table 4. Cytotoxic activity of the fusion proteins of the invention

| Protein | Continuous incubation of preparations with cells over 72h (test MTT, ng/ml) | | | | | | | | | | | |
|---|---|---|---|---|---|---|---|---|---|---|---|---|
| | A549 | | HCT116 | | MCF10A | | MES-SA | | MES-SA/Dx5 | | SK-MES-1 | |
| | $IC_{50}$ | ±SD | $IC_{50}$ | ±SD | $IC_{50}$ | ±SD | $IC_{50}$ | ±SD | $IC_{50}$ | ±SD | $IC_{50}$ | ±SD |
| rhTRAIL95-281 | >10000 | | 7557 | 3454 | >10000 | | >10000 | | 29.15 | 12.66 | 39.35 | 8.13 |
| Ex. 11[a] | 115.5 | 60.1 | 6.81 | 4.13 | 103.02 | 18.07 | 7.3 | 1.67 | 1.46 | 0.46 | 1.93 | 0.37 |
| Ex. 13[a] | 909.35 | 169.21 | 112 | | 750.5 | 156.27 | 110.85 | 9.69 | | | 29.04 | 0.65 |
| Ex. 6[a] | 915.2 | | 205.8 | | 995.7 | | 126.1 | | | | | |
| Ex. 23[a] | 1054.7 | 406.3 | 1054.7 | 406.3 | 245.45 | 25.67 | | | 48.06 | 1.75 | 22.1 | 0.18 |
| | NCI-H460 | | | | | | | | | | | |
| rhTRAIL95-281 | 5889 | 111 | | | | | | | | | | |
| Ex. 6[a] | 96.85 | | | | | | | | | | | |

Table 4a. Cytotoxic activity of the fusion proteins of the invention

| Protein | Continuous incubation of preparations with cells over 72h (test MTT, ng/ml) | | | | | | | | | | | |
|---|---|---|---|---|---|---|---|---|---|---|---|---|
| | COLO 205 | | DU 145 | | MDA-MB-231 | | PC 3 | | SW 620 | | SW 780 | |
| | $IC_{50}$ | ±SD | $IC_{50}$ | ±SD | $IC_{50}$ | ±SD | $IC_{50}$ | ±SD | $IC_{50}$ | ±SD | $IC_{50}$ | ±SD |
| rhTRAIL95-281 | | | | | | | | | | | | |
| Ex. 11[a] | 0.42 | 0.57 | 4.74 | 0.104 | 12.54 | 0.74 | 948 | 42.43 | 735.25 | 45.89 | 0.79 | 0.41 |
| | UM-UC-3 | | HT 29 | | 293 | | ACHN | | CAKI 2 | | BxPC3 | |
| TRAIL 95-281 | 2242 | 1367 | >10000 | | >10000 | | >10000 | | >10000 | | 64.71 | 31.81 |
| Ex. 11[a] | 0.64 | 0.04 | 4185.5 | 981 | 1152 | 77.78 | 4.86 | 1.06 | 25.42 | 3.22 | 0.43 | 0.114 |
| | HepG2 | | HT 144 | | NCI-H460 | | LNCaP | | OV-CAR-3 | | JURKAT A3 | |
| rhTRAIL 95-281 | >10000 | | 1730 | 218.5 | 5889 | 111 | 2052 | 466 | 963.00 | 144.25 | >10000 | |
| Ex. 11[a] | 5.63 | 0.45 | 0.26 | 0.065 | 1.8 | 0.34 | 408.15 | 11.8 | 0.114 | 0.07 | 0.29 | 0.24 |
| | PLC/PRF/5 | | PANC-1 | | NCI-H460 | | | | | | | |
| rhTRAIL95-281 | >9000 | | >10000 | | 5889 | 111 | | | | | | |
| Ex. 11[a] | 436.8 | | 142.25 | 56.78 | | | | | | | | |

Table 4b. Cytotoxic activity of the fusion proteins of the invention

| Protein | Continuous incubation of preparations with cells over 72h (test MTT, ng/ml) | | | | | | | | | | | |
|---|---|---|---|---|---|---|---|---|---|---|---|---|
| | A549 | | MCF10A | | HCT116 | | MES-SA | | MES-SA/Dx5 | | SK-MES-1 | |
| | $IC_{50}$ | ±SD | $IC_{50}$ | ±SD | $IC_{50}$ | ±SD | $IC_{50}$ | ±SD | $IC_{50}$ | ±SD | $IC_{50}$ | ±SD |
| TRAIL 95-281 | >2000 | | >2000 | | >2000 | | >2000 | | 27.59 | 13.34 | 100.7 | 26.4 |
| Ex. 6[b] | 915 | | 996 | | 206 | | 126 | | 56.2 | | 53.3 | |
| Ex. 23[b] | 550 | | 1342 | | 245 | 26 | 99 | | 48.1 | 1.8 | 22.11 | 0.18 |
| Ex. 16[b] | 10.96 | 2.14 | 4.71 | 1.26 | 1,5 | 0.19 | 0.08 | 0.07 | 0.0001 | | 0.06 | 0.03 |
| Ex. 11[b] | 89.2 | 11.1 | | | 13.73 | 1.34 | | | | | | |
| Ex. 13[b] | 405 | | | | | | | | | | | |

Table 4c. Cytotoxic activity of the fusion proteins of the invention

| Protein | Continuous incubation of preparations with cells over 72h (test MTT, ng/ml) | | | | | | | | | | | |
|---|---|---|---|---|---|---|---|---|---|---|---|---|
| | SW620 | | Panc-1 | | PLC/PRF/5 | | HT-29 | | Caki-1 | | SK-HEP-1 | |
| | $IC_{50}$ | ±SD | $IC_{50}$ | ±SD | $IC_{50}$ | ±SD | $IC_{50}$ | ±SD | $IC_{50}$ | ±SD | $IC_{50}$ | ±SD |
| TRAIL 95-281 | >2000 | | >2000 | | >2000 | | >2000 | | 13.42 | 2.16 | >2000 | |
| Ex. 11[b] | 325 | 24 | 10.87 | 1.8 | 46.4 | 20 | 893 | | 11.57 | | 75.1 | 11.3 |
| Ex. 16[b] | 1688 | 917 | 0.68 | 0.93 | 2.89 | 2.02 | 1063 | 480 | 3.29 | 1.44 | 4.27 | 2.36 |
| Ex. 13[b] | 4.42 | | 26 | | 5,8 | | | | | | | |
| | Caki-2 | | SK-OV-3 | | BxPC-3 | | HT-144 | | OV-CAR-3 | | HT-1080 | |
| | $IC_{50}$ | ±SD | $IC_{50}$ | ±SD | $IC_{50}$ | ±SD | $IC_{50}$ | ±SD | $IC_{50}$ | ±SD | $IC_{50}$ | ±SD |
| TRAIL 95-281 | >2000 | | >2000 | | 60.6 | 22.8 | 1134 | 375 | 963 | 144 | >2000 | |
| Ex. 16[b] | 3.54 | 0.52 | 161.2 | 1.8 | 0.55 | 0.12 | 0.13 | 0.05 | 0.12 | | 1025 | 395 |
| | MES-SA/Mx2 | | Colo205 | | MCF-7 | | MDA-MB-231 | | MDA-MB-B-435S | | ACHN | |
| | $IC_{50}$ | ±SD | $IC_{50}$ | ±SD | $IC_{50}$ | ±SD | $IC_{50}$ | ±SD | $IC_{50}$ | ±SD | $IC_{50}$ | ±SD |
| TRAIL 95-281 | 38.95 | 6.14 | 59.02 | 21.16 | >2000 | | >2000 | | >2000 | | >2000 | |
| Ex. 16[b] | 0.0001 | | 0.48 | 0.65 | 1.74 | 0.51 | 1.71 | 1.19 | 0.86 | 1.08 | 0.38 | 0.32 |

Table 4d. Cytotoxic activity of the fusion proteins of the invention

| Protein | Continuous incubation of preparations with cells over 72h (test MTT, ng/ml) | | | | | | | | | | | |
|---|---|---|---|---|---|---|---|---|---|---|---|---|
| | NCI-H460 | | HepG2 | | Panc03.27 | | A498 | | HUV-EC-C | | | |
| | $IC_{50}$ | ±SD | $IC_{50}$ | ±SD | $IC_{50}$ | ±SD | $IC_{50}$ | ±SD | $IC_{50}$ | ±SD | $IC_{50}$ | ±SD |
| TRAIL 95-281 | 438 | 77 | >2000 | | 315 | | 1611 | 103 | >2000 | | | |
| Ex. 16[b] | 0.47 | 0.22 | 11.27 | 1.3 | 11.3 | 0.4 | 0.06 | 0.07 | >2000 | | | |
| Ex. 13[b] | 6,78 | | 13 | | | | | | | | | |
| Ex. 11[b] | | | | | 42.1 | 17.4 | 2.6 | 0.15 | | | | |

3. Antitumor effectiveness of fusion proteins *in vivo* on xenografts

Antitumor activity of protein preparations was tested in a mouse model of human colon cancer Colo 205 and HCT-116, human lung cancer A549 and NCI-H460-Luc, human prostate cancer PC-3, human pancreas cancer Panc-1 and MIA PaCa-2, human liver cancer PCL/PRF/5 and HepG2, and human multidrug resistant MES uterine sarcoma -SA.Dx5.

Cells

The cells of human lung cancer A549 and NCI-H460-Luc2 and human prostate cancer PC3 were maintained in RPMI1640 medium (HyClone, Logan, UT, USA) supplemented with 10% fetal calf serum and 2 mM glutamine.

The cells of human colon cancer Colo 205 were maintained in RPMI1640 medium (HyClone, Logan, UT, USA) (optionally mixed in the ratio of 1:1 with Opto-MEM (Invitrogen, Cat. No. 22600-134) supplemented with 10% fetal calf serum and 2 mM glutamine.

The cells of human pancreas cancer PANC-1, human liver cancer PLC/PRF/5, pancreas cancer MIA PaCa-2 and human colon cancer SW-620 were maintained in DMEM medium (HyClone, Logan, UT, USA) supplemented with 10% fetal calf serum and 2 mM glutamine.

The cells of human colon cancer HCT-116 were maintained in McCoy's medium (HyClone, Logan, UT, USA) supplemented with 10% fetal calf serum and 2 mM glutamine.

The cells of multidrug resistant human uterine sarcoma MES-SA.Dx5 were maintained in McCoy's medium (HyClone, Logan, UT, USA) supplemented with 10% fetal calf serum and 2 mM glutamine, and 1 IJM doxorubicin hydrochloride (Sigma, Cat. No. D1515-10MG). Three days before the cells implantation, the cells were cultured in medium without doxorubicin.

The cells of human liver cancer HepG2 were maintained in MEM medium (HyClone, Logan, UT, USA) supplemented with 10% fetal calf serum and 2 mM glutamine. On the day of mice grafting, the cells were detached from the support by washing the cells with trypsin (Invitrogen), then the cells were centrifuged at 1300 rpm, 4°C, 8 min., suspended in HBSS buffer (Hanks medium).

On the day of mice grafting, the cells were detached from the support by washing the cells with trypsin (Invitrogen), then the cells were centrifuged at 1300 rpm, 4°C, 8 min., suspended in HBSS buffer (Hanks medium).

Mice

Examination of antitumor activity of proteins of the invention was conducted on 4-6 week-old (lung cancer model) Cby.Cg-foxn1(nu)/J) mice or 9-10 week old (prostate cancer model) obtained from Centrum Medycyny Doświadczalnej in Biatystok, 4-5 week-old female Crl:SHO-Prkdc$^{scid}$Hr$^{h}$ mice obtained from Charles River Germany. Mice were kept under specific pathogen-free conditions with free access to food and demineralised water (*ad libitum*). All experiments on animals were carried in accordance with the guidelines: "Interdisciplinary Principles and Guidelines for the Use of Animals in Research, Marketing and Education" issued by the New York Academy of Sciences' Ad Hoc Committee on Animal Research and were approved by the IV Local Ethics Committee on Animal Experimentation in Warsaw (No. 71/2009).

The course and evaluation of the experiments

Tumor size was measured using electronic calliper, tumor volume was calculated using the formula: $(a^2 \times b)/2$,

where a = shorter diagonal of the tumor (mm) and b = longer diagonal of the tumor (mm). Inhibition of tumor growth was calculated using the formula:

$$TGI\ [\%]\ (\text{Tumor growth inhibition}) = (WT/WC) \times 100 - 100\%$$

wherein WT is the average tumor volume in the treatment group, and WC is the average tumor volume in the control group.

The experimental results are presented as a mean value ± standard deviation (SD). All calculations and graphs were prepared using the program GraphPad Prism 5.0.

Lung cancer model c

Experiment A.

On day 0 Cby.Cg-foxn1(nu)/J mice were grafted subcutaneously (s.c.) in the right side with $5x10^6$ of A549 cells suspended in 0.1 ml of the mixture HBSS:Matrigel 4:1 using syringe with a needle 0.5 x 25 mm (Bogmark). On the 19 day of the experiment mice were randomized to obtain the average size of tumors in the group of ~75 mm3 and assigned to treatment groups. The treatment groups were administered with the preparations of fusion protein of the invention of Ex. $11^a$ (15 mg/kg), and rhTRAIL114-281 (20 mg/kg) as a comparison and water for injections as a control. The preparations were administered intravenously (i.v.) 6 times once daily every second day. On the 35 day of the experiment mice were sacrificed by disruption of the spinal cord.

The experimental results are shown on Fig. 1 and Fig. 2 as a diagram of changes of the tumor volume and tumor growth inhibition (%TGI) as the percentage of control in mice treated with fusion protein of the invention of Ex. $11^a$ and comparatively with rhTRAIL114-281.

The experimental results presented in Fig. 1 and 2 show that administration of the fusion protein of the invention of Ex.$11^a$ caused A549 tumor growth inhibition, with TGI 28% relative to the control on 35 day of experiment. For rhTRAIL114-281 used as the comparative reference, a slight inhibitory effect on tumor cell growth was obtained relative to the control, with TGI at the level of 0%. Thus, fusion proteins of the invention exerted much stronger effect compared to TRAIL alone.

Experiment B.On day 0 Crl:SHO-Prkdc$^{scid}$Hr$^{hr}$ mice were grafted subcutaneously (s.c.) in the right side with $7x10^6$ of A549 cells suspended in 0.1 ml of the mixture HBSS:Matrigel 3:1 using syringe with a needle 0.5 x 25 mm (Bogmark). On the 17 day of the experiment mice were randomized to obtain the average size of tumors in the group of ~100-120 $mm^3$ and assigned to treatment groups. The treatment groups were administered with the preparations of fusion protein of the invention of Ex. $11^a$ (40 mg/kg), and rhTRAIL114-281 (20 mg/kg) as a comparison against formulation buffer (19 mM $NaH_2PO_4$ 81 mM $Na_2HPO_4$, 50 mM NaCl, 5 mM glutathione, 0.1 mM $ZnCl_2$, 10% glycerol, pH 7.4) as a control. The preparations were administered intravenously (i.v.) 6 times once daily every second day. On the 34 day of the experiment mice were sacrificed by disruption of the spinal cord.

The experimental results are shown on Fig. 3 and Fig. 4 as a diagram of changes of the tumor volume and tumor growth inhibition (%TGI) as the percentage of control in mice treated with fusion protein of the invention of Ex.$11^a$ and comparatively rhTRAIL114-281.

The experimental results presented in Fig. 3 and 4 show that administration of the fusion protein of the invention of Ex.$11^a$ caused A549 tumor growth inhibition, with TGI 45% relative to the control on 34 day of experiment. For rhTRAIL114-281 used as the comparative reference, a slight inhibitory effect on tumor cell growth was obtained relative to the control, with TGI at the level of 21,8%. Thus, fusion protein of the invention exerted much stronger effect compared to TRAIL alone.

Experiment C.

On day 0 Crl:SHO-Prkdc$^{scid}$Hr$^{hr}$ mice were grafted subcutaneously (s.c.) in the right side with $5x10^6$ of NCI-H460-Luc2 cells suspended in 0.1 ml of HBSS buffer using syringe with a needle 0.5 x 25 mm (Bogmark). On the 11 day of the experiment mice were randomized to obtain the average size of tumors in the group of ~100-120 $mm^3$ and assigned to treatment groups. The treatment groups were administered with the preparations of fusion protein of the invention of Ex.$11^a$ (40 mg/kg and 30 mg/kg), and rhTRAIL114-281 (20 mg/kg) as a comparison against formulation buffer (19 mM $NaH_2PO_4$ 81 mM $Na_2HPO_4$, 50 mM NaCl, 5 mM glutathione, 0.1 mM $ZnCl_2$, 10% glycerol, pH 7.4) as a control. The preparations were administered intravenously (i.v.) 6 times once daily every second day (in case of fusion protein of the invention of Ex.$11^a$ first administration at a dose 40 mg/kg and subsequent at 30 mg/kg. On the 29 day of the experiment mice were sacrificed by disruption of the spinal cord.

The experimental results are shown on Fig. 5 and Fig. 6 as a diagram of changes of the tumor volume and tumor growth inhibition (%TGI) as the percentage of control in mice treated with fusion protein of the invention of Ex. 11[a] and comparatively with rhTRAIL114-281.

The experimental results presented in Fig. 5 and 6 show that administration of the fusion protein of the invention of Ex.11[a] caused tumor NCI-H460-Luc2 growth inhibition, with TGI 93% relative to the control on 29 day of experiment. For rhTRAIL114-281 used as the comparative reference, an inhibitory effect on tumor cell growth was obtained relative to the control, with TGI at the level of 76%. Thus, fusion protein of the invention exerted much stronger effect compared to TRAIL alone.

The tested fusion protein did not cause significant side effects manifested by a decrease in body weight of mice (i.e. less than 10% of the baseline body weight). This shows low systemic toxicity of the protein of the invention.

Prostate *cancer* model

On day 0 Cby.Cg-foxn1(nu)/J mice were grafted subcutaneously (s.c.) in the right side with $5x10^6$ of PC3 cells suspended in 0.18 ml of HBSS buffer and 0.02 ml of Matrigel using syringe with a needle 0.5 x 25 mm (Bogmark). On the 29 day of the experiment mice were randomized to obtain the average size of tumors in the group of ~90 $mm^3$ and assigned to treatment groups. The treatment groups were administered with the preparations of fusion protein of the invention of Ex. 11[a] (15 mg/kg) and 0.9% NaCl as a control. The preparations were administered intravenously (i.v.) once daily for 6 days. On the 60 day of the experiment mice were sacrificed by disruption of the spinal cord.

The experimental results are shown on Fig. 7 and Fig. 8 as a diagram of changes of the tumor volume and tumor growth inhibition (%TGI) as the percentage of control in mice treated with fusion protein of the invention of Ex. 11[a].

The experimental results presented in Fig. 7 and 8 show that administration of the fusion protein of the invention of Ex.11[a] caused PC3 tumor growth inhibition, with TGI 33% relative to the control on the 60 day of experiment.

The tested fusion protein did not cause significant side effects manifested by a decrease in body weight of mice (i.e. less than 10% of the baseline body weight). This shows low systemic toxicity of the protein of the invention.

Pancreas *cancer* model

Experiment A on PANC-1 cells

On day 0 Crl:SHO-$Prkdc^{scid}Hr^{hr}$ mice were grafted subcutaneously (s.c.) in the right side with $5x10^6$ of PANC-1 cells suspended in 0.1 ml of the mixture HBSS:Matrigel 3:1 using syringe with a needle 0.5 x 25 mm (Bogmark). On the 31 day of the experiment mice were randomized to obtain the average size of tumors in the group of ~110 $mm^3$ and assigned to treatment groups. The treatment groups were administered with the preparations of fusion protein of the invention of Ex. 11[a] (40 mg/kg), and rhTRAIL114-281 (20 mg/kg) as a comparison against formulation buffer (19 mM $NaH_2PO_4$ 81 mM $Na_2HPO_4$, 50 mM NaCl, 5 mM glutathione, 0.1 mM $ZnCl_2$, 10% glycerol, pH 7.4) as a control. The preparations were administered intravenously (i.v.) 6 times once daily every second day. On the 42 day of the experiment mice were sacrificed by disruption of the spinal cord.

The experimental results are shown on Fig. 9 and Fig. 10 as a diagram of changes of the tumor volume and tumor growth inhibition (%TGI) as the percentage of control in mice treated with fusion protein of the invention of Ex.11[a] and comparatively with rhTRAIL114-281.

The experimental results presented in Fig. 9 and 10 show that administration of the fusion protein of the invention of Ex. 11[a] caused PANC-1 tumor growth inhibition, with TGI 32.6% relative to the control on 42 day of experiment. For rhTRAIL114-281 used as the comparative reference, a slight inhibitory effect on tumor cell growth was obtained relative to the control, with TGI at the level of 26%. Thus, fusion protein of the invention exerted much stronger effect compared to TRAIL alone.

The tested fusion protein did not cause significant side effects manifested by a decrease in body weight of mice (i.e. less than 10% of the baseline body weight). This shows low systemic toxicity of the protein of the invention.

Experiment B on MIA PaCa-2 cells

On day 0 Crl:SHO-$Prkdc^{scid}Hr^{hr}$ mice were grafted subcutaneously (s.c.) in the right flank region with $5x10^6$ of MIA PaCa-2 cells suspended in 0.1 ml of 3:1 mixture HBSS buffer:Matrigel using syringe with a 0.5 x 25 mm needle (Bogmark). When tumors reached the size of 60-398 $mm^3$ (day 20), mice were randomized to obtain the average size of tumors in the group of ~ 170 $mm^3$ and assigned to treatment groups. The treatment groups were administered with the preparations of fusion protein of the invention of Ex. 16[b] (50 mg/kg) and rhTRAIL114-281 (50 mg/kg) as a comparison and reference compound gemcitabine (Gemzar, Eli Lilly) (50 mg/kg). The preparations were administered intravenously (*i.v.*) six times every second day, gemcitabine was applied intraperitoneally (*i.p.*) in the same schedule. The control group received formulation buffer.

When a therapeutic group reached the average tumor size of ~ 1000 $mm^3$, mice were sacrificed by the cervical dislocation.

The experimental results obtained in mice Crl:SHO-*Prkdc^scid^Hr^hr^* burdened with MIA PaCa-2 pancreatic carcinoma treated with fusion protein of the invention of Ex. 16[b] and comparatively with rhTRAIL114-281 and gemcitabine are shown in Fig. 19 as a diagram of changes of the tumor volume and in Figure 20 which shows tumor growth inhibition (%TGI) as a percentage of control.

The results of the experiment presented in Figures 19 and 20 show that administration of the fusion protein of the invention of Ex. 16[b] caused MIA PaCa-2 tumor growth inhibition, with TGI 93% relative to the control on 61$^{th}$ day of the experiment. For rhTRAIL114-281 and gemcitabine as comparative references, a slight inhibitory effect on tumor cell growth was obtained relative to the control, with TGI at the level of 68% and 42,6%, respectively. Thus, fusion proteins of the invention exerted much stronger effect compared to TRAIL alone and standard chemotherapy.

Colon cancer model

Experiment A on HCT116 cellsOn day 0 Crl:SHO-$Prkdc^{scid}Hr^{hr}$ mice were grafted subcutaneously (s.c.) in the right side with $5x10^6$ of HCT116 cells suspended in 0.1 ml of HBSS buffer using syringe with a needle 0.5 x 25 mm (Bogmark). On the18 day of the experiment mice were randomized to obtain the average size of tumors in the group of ~400$mm^3$ and assigned to treatment groups. The treatment groups were administered with the preparations of fusion protein of the invention of Ex. 11[a] (35 mg/kg), and rhTRAIL114-281 (20 mg/kg) as a comparison against formulation buffer (5 mM $NaH_2PO_4$, 95 mM $Na_2HPO_4$, 200 mM NaCl, 5 mM glutathione, 0.1 mM $ZnCl_2$, 10% glycerol, 80 mM saccharose, pH 8.0) as a control. The preparations were administered intravenously (i.v.) 6 times once daily every second day. On the 32 day of the experiment mice were sacrificed by disruption of the spinal cord.

The experimental results are shown on Fig. 11 and Fig. 12 as a diagram of changes of the tumor volume and tumor growth inhibition (%TGI) as a percentage of control in mice treated with fusion protein of the invention of Ex. 11[a] and comparatively with rhTRAIL114-281.

The experimental results presented in Fig. 11 and 12 show that administration of the fusion protein of the invention of Ex.11[a] caused tumor HCT116 growth inhibition, with TGI 33.5% relative to the control on 32 day of experiment. For rhTRAIL114-281 used as the comparative reference, a slight inhibitory effect on tumor cell growth was obtained relative to the control, with TGI at the level of 5.6%. Thus, fusion protein of the invention exerted much stronger effect compared to TRAIL alone.

The tested fusion protein did not cause significant side effects manifested by a decrease in body weight of mice (i.e. less than 10% of the baseline body weight). This shows low systemic toxicity of the protein of the invention.

Experiment A1 on HCT116 cells

On day 0 Crl:SHO-*$Prkdc^{scid}Hr^{hr}$* mice were grafted subcutaneously (s.c.) in the right flank region with $5x10^6$ of HCT116 cells suspended in 0.1 ml of the 3:1 mixture HBSS buffer:Matrigel using syringe with a 0.5 x 25 mm needle (Bogmark). When tumors reached the size of 71-432 $mm^3$ (day 13), mice were randomized to obtain the average size of tumors in the group of ~ 180 $mm^3$ and assigned to treatment groups. The treatment groups were administered with the preparations of fusion protein of the invention of Ex. 16[b] (90 mg/kg) and rhTRAIL114-281 (65 mg/kg) as a comparison. The preparations were administered intravenously (*i.v.*) six times every second day. The control group received formulation buffer.

When an experimental group reached the average tumor size of ~ 1000 $mm^3$, mice were sacrificed by cervical dislocation.

The experimental results obtained in mice CrLSHO-*$Prkdc^{scid}Hr^{hr}$* burdened with HCT116 colon cancer treated with fusion proteins of the invention of Ex. 16[b] and comparatively with rhTRAIL114-281 are shown in Fig. 21 as a diagram of changes of the tumor volume and in Figure 22 which shows tumor growth inhibition (%TGI) as a percentage of control.

The results of experiments presented in Figures 21 and 22 show that administration of the fusion protein of the invention of Ex. 16[b] caused HCT116 tumor growth inhibition, with TGI 65.8% relative to the control on 24$^{th}$ day of the experiment. For rhTRAIL114-281 used as the comparative reference, a slight inhibitory effect on tumor cell growth was obtained relative to the control, with TGI at the level of 37.9%. Thus, fusion proteins of the invention exerted much stronger effect compared to TRAIL alone.

Experiment B on SW620 cells

On day 0 Crl:SHO-$Prkdc^{scid}Hr^{hr}$ mice were grafted subcutaneously (s.c.) in the right side with $5x10^6$ of SW620 cells suspended in 0.1 ml of the mixture HBSS:Matrigel 3:1 using syringe with a needle 0.5 x 25 mm (Bogmark). On the 17 day of the experiment mice were randomized to obtain the average size of tumors in the group of ~320 $mm^3$ and assigned to treatment groups. The treatment groups were administered with the preparation of fusion protein of the invention of Ex. 16[a] (20 mg/kg) and rhTRAIL114-281 (30 mg/kg) as a comparison against formulation buffer (19 mM $NaH_2PO_4$ 81 mM $Na_2HPO_4$, 50 mM NaCl, 5 mM glutathione, 0.1 mM $ZnCl_2$, 10% glycerol, pH 7.4) as a control. The preparations were administered intravenously (i.v.) 6 times once daily every second day. On the 31 day of the experiment mice were sacrificed by disruption of the spinal cord.

The experimental results in mice Crl:SHO-Prkdc$^{scid}$Hr$^{hr}$ burdened with SW620 treated with fusion protein of the invention of Ex.16[a] and comparatively with rhTRAIL114-281 are shown on Fig. 13 and Fig. 14 as a diagram of changes of the tumor volume and tumor growth inhibition (%TGI) as a percentage of control.

The experimental results presented in Fig. 13 and 14 show that administration of the fusion protein of the invention of Ex.16[a] caused tumor SW620 growth inhibition, with TGI equal to 25% comparing to control on the 31 day of experiment. For rhTRAIL114-281 used as the comparative reference, no inhibitory effect on tumor cell growth was obtained relative to the control, with TGI at the level of -9%. Thus, fusion proteins of the invention exerted much stronger effect compared to TRAIL alone.

The tested fusion protein did not cause significant side effects manifested by a decrease in body weight of mice (i.e. less than 10% of the baseline body weight). This shows low systemic toxicity of the protein of the invention.

<u>Experiment C on Colo205 cells</u>On day 0 Crl:SHO-Prkdc$^{scid}$Hr$^{hr}$ mice were grafted subcutaneously (s.c.) in the right side with 5x10$^6$ of Colo205 cells suspended in 0.1 ml of HBSS buffer using syringe with a needle 0.5 x 25 mm (Bogmark). On the 13 day of the experiment mice were randomized to obtain the average size of tumors in the group of ~115 $mm^3$ and assigned to treatment groups.

The treatment groups were administered with the preparations of fusion protein of the invention of Ex.16[a] (30 mg/kg), and rhTRAIL114-281 (30 mg/kg) as a comparison against formulation buffer (19 mM $NaH_2PO_4$ 81 mM $Na_2HPO_4$, 50 mM NaCl, 5 mM glutathione, 0.1 mM $ZnCl_2$, 10% glycerol, pH 7.4) as a control. The preparations were administered intravenously (i.v.) 6 times once daily every second day. On the 33 day of the experiment mice were sacrificed by disruption of spinal cord.

The experimental results are shown on Fig. 15 and Fig. 16 as a diagram of changes of the tumor volume and tumor growth inhibition (%TGI) as the percentage of control.

The experimental results presented in Fig. 15 and 16 show that administration of the fusion protein of the invention of Ex.16[a] caused Colo205 tumor growth inhibition, with TGI equal to 80% relative to the control on 33 day of experiment. For rhTRAIL114-281 used as the comparative reference, a slight inhibitory effect on tumor cell growth was obtained relative to the control, with TGI at the level of 18.8%. Thus, fusion proteins of the invention exerted much stronger effect compared to TRAIL alone.

The tested fusion proteins did not cause significant side effects manifested by a decrease in body weight of mice (i.e. less than 10% of the baseline body weight). This shows low systemic toxicity of the protein of the invention.

<u>Multidrug resistant uterine sarcoma model</u>

On day 0 Crl:SHO-Prkdc$^{scid}$Hr$^{hr}$ mice were grafted subcutaneously (s.c.) in the right side with 7x10$^6$ of MES-SA/Dx5 cells suspended in 0.1 ml of the mixture HBSS:Matrigel 10:1 using syringe with a needle 0.5 x 25 mm (Bogmark). On the 19 day of the experiment mice were randomized to obtain the average size of tumors in the group of ~180 $mm^3$ and assigned to treatment groups. The treatment groups were administered with the preparation of fusion protein of the invention of Ex.11[a] (30 mg/kg) and rhTRAIL114-281 (10 mg/kg) as a comparison against formulation buffer (19 mM $NaH_2PO_4$ 81 mM $Na_2HPO_4$, 50 mM NaCl, 5 mM glutathione, 0,1 mM $ZnCl_2$, 10% glycerol, pH 7.4) as a control. Preparations were administered intravenously (*i.v.*) 6 times once daily every second day. On the 35 day of the experiment mice were sacrificed by disruption of spinal cord.

The experimental results are shown on Fig. 17 and Fig. 18 as a diagram of changes of the tumor volume and tumor growth inhibition (%TGI) as a percentage of control.

The experimental results presented in Fig. 17 and 18 show that administration of the fusion protein of the invention of Ex.11[a] caused MES-SA/Dx5 tumor growth inhibition, with TGI equal to 81% relative to the control on 35 day of experiment. For rhTRAIL114-281 used as the comparative reference, a slight inhibitory effect on tumor cell growth was obtained relative to the control, with TGI at the level of 29%. Thus, fusion protein of the invention exerted much stronger effect compared to TRAIL alone.

The tested fusion protein did not cause significant side effects manifested by a decrease in body weight of mice (i.e. less than 10% of the baseline body weight). This shows low systemic toxicity of the protein of the invention.

Liver cancer model

Experiment A on HepG2 cells

On day 0 Crl:SHO-$Prkdc^{scid}Hr^{hr}$ mice were grafted subcutaneously (s.c.) in the right flank region with $7x10^6$ of HepG2 cells suspended in 0.1 ml of the 3:1 mixture HBSS buffer:Matrigel using syringe with a 0.5 x 25 mm needle (Bogmark). When tumors reached the size of 64-529 $mm^3$ (day 25), mice were randomized to obtain the average size of tumors in the group of ~ 230 $mm^3$ and assigned to treatment groups. The treatment groups were administered with the preparations of fusion protein of the invention of Ex. $16^b$ (80 mg/kg) and rhTRAIL114-281 (50 mg/kg) as a comparison and reference compound 5-FU (5-Fluorouracil, Sigma-Aldrich) (20 mg/kg). The preparations were administered intravenously (*i.v.*) six times every second day, 5-FU was applied intraperitoneally (*i.p.*). The control group received formulation buffer.

When the therapeutic group reached the average tumor size of ~ 1000 $mm^3$, mice were sacrificed by cervical dislocation.

The experimental results obtained are shown in Fig. 23 as a diagram of changes of the tumor volume and in Figure 24 which shows tumor growth inhibition (%TGI) as a percentage of control.

The results of the experiment presented in Figures 23 and 24 show that administration of the fusion protein of the invention of Ex. $16^b$ caused HepG2 tumor growth inhibition, with TGI 94.6% relative to the control on $42^{th}$ day of the experiment. For rhTRAIL114-281 as the comparative reference, a slight inhibitory effect on tumor cell growth was obtained relative to the control, with TGI at the level of 23.2%. Reference compound, 5-FU, didn't show any efficacy against HepG2 tumors. Thus, fusion protein of the invention exerted much stronger effect compared to TRAIL alone and standard chemotherapy.

Experiment B on PLC/PRF/5 cells

On day 0 Crl:SHO-$Prkdc^{scid}Hr^{hr}$ mice were grafted subcutaneously (s.c.) in the right flank region with $7x10^6$ of PLC/PRF/5 cells suspended in 0.1 ml of the 3:1 1 mixture HBSS buffer:Matrigel using syringe with a 0.5 x 25 mm needle (Bogmark). When tumors reached the size of 72-536 $mm^3$ (day 29), mice were randomized to obtain the average size of tumors in the group of ~ 205 $mm^3$ and assigned to treatment groups. The treatment groups were administered with the preparations of fusion protein of the invention of Ex. $16^b$ (50 mg/kg) and rhTRAIL114-281 (50 mg/kg) as a comparison and reference compound 5-FU (5-Fluorouracil, Sigma-Aldrich) (30 mg/kg). The preparations were administered intravenously (*i.v.*) six times every second day, except 5-FU, which was applied intraperitoneally (i.p.) in the schedule (q1dx5)x2. The control group received formulation buffer.

When an experimental group reached the average tumor size of ~ 1000 $mm^3$, mice were sacrificed by cervical dislocation.

The experimental results obtained are shown in Fig. 25 as a diagram of changes of the tumor volume and in Figure 26 which shows tumor growth inhibition (%TGI) as a percentage of control.

The results of the experiment presented in Figures 25 and 26 show that administration of the fusion protein of the invention of Ex. $16^b$ caused PLC/PRF/5 tumor growth inhibition, with TGI 53% relative to the control on $43^{th}$ day of the experiment. For rhTRAIL114-281 and 5-FU as comparative references, a slight inhibitory effect on tumor cell growth was obtained relative to the control, with TGI at the level of 25.2% and 32.2%, respectively. Thus, fusion protein of the invention exerted much stronger effect compared to TRAIL alone and standard chemotherapy.

## Claims

**1.** A fusion protein comprising:

- domain (a) which is a functional fragment of the sequence of soluble hTRAIL protein, said hTRAIL protein sequence being presented as SEQ. No. 90, which fragment begins with an amino acid at a position from the range hTRAIL95 to hTRAIL121, inclusive, and ends with the amino acid at the position hTRAIL281, or a homolog of said functional fragment having at least 70% sequence identity, preferably 85% identity, wherein said functional fragment or homolog thereof are capable of inducing apoptotic signal in mammalian cells upon binding to its receptors on the surface of the cells, and
- at least one domain (b) which is the sequence of a cytolytic effector peptide with an amphipathic alpha-helix conformation forming pores in the cell membrane,

wherein the sequence of the domain (b) is attached at the C-terminus or N-terminus of domain (a),

**2.** The fusion protein according to claim 1, wherein domain (a) is selected from the group consisting of hTRAIL95-281, hTRAIL114-281, hTRAIL115-281, hTRAIL116-281, hTRAIL119-281, and hTRAIL121-281.

**3.** The fusion protein according to claim 1 or 2, in which domain (b) is selected from the group consisting of:

- pilosulin-1 of SEQ. No. 36,
- pilosulin-5 of SEQ. No. 37,
- 14-amino acids synthetic lytic peptide of SEQ. No. 41,
- 27-amino acids peptide FFhCAP18 of SEQ. No. 43,
- BAMP-28 peptide of SEQ. No. 44,
- analogue of isoform C of lytic peptide from Entamoeba histolytica of SEQ. No. 45,
- analogue of isoform A of lytic peptide from Entamoeba histolytica SEQ. No. 46,
- analogue of isoform B of lytic peptide from Entamoeba histolytica of SEQ. No. 47,
- fragment of HA2 domain of influenza virus haemagglutinin of SEQ. No. 48,
- active fragment of human perforin of SEQ. No. 54,
- parasporin-2 z Bacillus thuringensis of SEQ. No. 55,
- fusion protein comprising synthetic lytic peptide with KLLK motif and a peptide being antagonist of PDGF receptor of SEQ. No. 125,
- pleurocidin analogue of SEQ. No. 126,
- pleurocidin analogue of SEQ. No. 127, and
- synthetic lytic peptide of SEQ. No. 128.

**4.** The fusion protein according to any one of the claims 1 to 3, which between domain (a) and domain (b) or between domains (b) contains domain (c) containing protease cleavage site, selected from a sequence recognized by metalloprotease MMP, a sequence recognized by urokinase uPA, and sequence recognized by furin and a sequence recognized by native furin.

**5.** The fusion protein according to claim 4, in which a sequence recognized by metalloprotease MMP is Pro Leu Gly Leu Ala Gly, a sequence recognized by urokinase uPA is Arg Val Val Arg, a sequence recognized by furin is Arg Lys Lys Arg, and a sequence recognized by native furin is Arg His Arg Gln Pro Arg Gly Trp Glu Gln Leu or His Arg Gln Pro Arg Gly Trp Glu Gln.

**6.** The fusion protein according to claim 4 or 5, in which domain (c) is a combination of sequence recognized by metalloprotease MMP and a sequence recognized by urokinase uPA located next to each other.

**7.** The fusion protein according to any of preceding claims, in which effector peptide of domain (b) is additionally connected with transporting domain (d), selected from the group consisting of:

- (d1) polyhistidine sequence transporting through the cell membrane comprising 6, 7 , 8, 9, 10 or 11 His residues, and
(d2) polyarginine sequence transporting through a cell membrane, consisting of 6, 7, 8, 9, 10 or 11 Arg residues,
(d3) PD4 transporting sequence (protein transduction domain 4) Tyr Ala Arg Ala Ala Ala Arg Gln Ala Arg Ala,
(d4) a transporting sequence consisting of transferrin receptor binding sequence Thr His Arg Pro Pro Met Trp Ser Pro Val Trp Pro, and
(d5) PD5 transporting sequence (protein transduction domain 5, TAT protein) Tyr Gly Arg Lys Lys Arg Arg Gln Arg Arg Arg,

and combinations thereof.

**8.** The fusion protein according to claim 7, wherein sequence (d) is located at the C-terminus or N-terminus of the effector peptide domain (b).

**9.** The fusion protein according to claim 7, wherein transporting domain (d) is located between domain (b) and domain (c), or between domain (a) and domain (c), or between two domains (c).

**10.** The fusion protein according to claim 7, wherein sequence (d) is located at the C-terminus of the fusion protein.

**11.** The fusion protein according to any one of claims 4 to 10, which between two (c) domains contains domain (e) which is a linker for attachment of PEG molecule, selected from Ala Ser Gly Cys Gly Pro Glu Gly and Ala Ser Gly Cys Gly Pro Glu.

**12.** The fusion protein according to any one of claims from 4 to 11, which additionally comprises a flexible steric linker between domains (a), (b) and/or (c).

**13.** The fusion protein according to claim 12, wherein the steric linker is selected from Gly Gly, Gly Gly Gly, Gly Ser Gly, Gly Gly Gly Gly Ser, Gly Gly Gly Gly Gly Ser, Gly Gly Ser Gly Gly, Gly Gly Gly Ser Gly Gly Gly, Gly Gly Gly Gly Ser Gly, Gly Gly Gly Ser Gly Gly Gly Gly Gly Ser, Gly Gly Gly Gly Ser Gly Gly Gly Gly, Gly Ser Gly Gly Gly Ser Gly Gly Gly, Cys Ala Ala Cys Ala Ala Ala Cys, Cys Ala Ala Ala Cys Ala Ala Cys, Ser Gly Gly, single glycine residue Gly, and single cysteine residue Cys, and combinations thereof.

**14.** The fusion protein according to claim 1, having the amino acid sequence selected from the group consisting of SEQ. No. 4; SEQ. No. 5; SEQ. No. 6; SEQ. No. 11; SEQ. No. 12; SEQ. No. 13; SEQ. No. 14; SEQ. No. 15; SEQ. No. 16; SEQ. No. 18; SEQ. No. 19; SEQ. No. 20; SEQ. No. 21; SEQ. No. 22; SEQ. No. 23; SEQ. No. 30; SEQ. No. 31; SEQ. No. 32; SEQ. No. 91; SEQ. No. 92; SEQ. No. 93; SEQ. No. 94; SEQ. No. 95; SEQ. No. 96; SEQ. No. 97, SEQ. No. 98; and SEQ. No. 102.

**15.** The fusion protein according to any one of the preceding claims, which is a recombinant protein.

**16.** A pharmaceutical composition comprising as an active ingredient the fusion protein as defined in any one of claims 1 to 15, in combination with a pharmaceutically acceptable carrier.

**17.** The pharmaceutical composition according to claim 16 in a form for parenteral administration.

**18.** The fusion protein as defined in any one of claims 1 to 15 for use in the treatment of neoplastic diseases in mammals, including humans.

## Patentansprüche

**1.** Ein Fusionsprotein, umfassend:

- Domäne (a), die ein funktionelles Fragment der Sequenz des löslichen hTRAIL-Proteins ist, wobei besagte Sequenz des hTRAIL-Proteins als SEQ. Nr. 90 dargestellt ist, welches Fragment mit einer Aminosäure an einer Position im Bereich von hTRAIL95 bis hTRAIL121, einschließlich, beginnt, und mit der Aminosäure an der Position hTRAIL281 endet, oder ein Homolog des funktionellen Fragments mit mindestens 70% Sequenzidentität, vorzugsweise 85% Identität, wobei besagtes funktionelles Fragment oder ein Homolog dieses funktionellen Fragments nach der Bindung deren Rezeptoren an die Zelloberflächen die Fähigkeit hat, ein apoptotisches Signal in Säugetierzellen zu induzieren, und
- mindestens eine Domäne (b), die die Sequenz eines zytolytischen Effektor-Peptids mit amphipathischer alpha-Helix Konformation ist, das Poren in der Zellmembran bildet,

wobei die Sequenz der Domäne (b) am C-Terminus oder N-Terminus der Domäne (a), angebracht ist.

**2.** Das Fusionsprotein gemäß Anspruch 1, wobei die Domäne (a) ausgewählt ist aus der Gruppe bestehend aus hTRAIL95-281, hTRAIL114-281, hTRAIL115-281, hTRAIL116-281, hTRAIL119-281 und hTRAIL121-281.

**3.** Das Fusionsprotein gemäß Anspruch 1 oder 2, wobei die Domäne (b) ausgewählt ist aus der Gruppe bestehend aus:

- Pilosulin-1 von SEQ. Nr. 36,
- Pilosulin-5 von SEQ. Nr. 37,

- 14-Aminosäuren synthetisches lytisches Peptid von SEQ. Nr. 41,
- 27-Aminosäuren Peptid FFhCAP18 von SEQ. Nr. 43,
- BAMP-28 Peptid von SEQ. Nr. 44,
- Analogon der Isoform C des lytischen Peptids aus Entamoeba histolytica von SEQ. Nr. 45,
- Analogon der Isoform A des lytischen Peptids aus Entamoeba histolytica SEQ. Nr. 46,
- Analogon von Isoform B des lytischen Peptids aus Entamoeba histolytica von SEQ. Nr. 47,
- Fragment der HA2-Domäne des Influenzavirus Hämagglutinin von SEQ. Nr. 48,
- aktives Fragment des menschlichen Perforins von SEQ. Nr. 54,
- Parasporin-2 von Bacillus thuringensis von SEQ. Nr. 55,
- Fusionsprotein, umfassend synthetisches lytisches Peptid mit KLLK-Motiv und ein Peptid, das ein Agonist des PDGF-Rezeptors von SEQ Nr. 125 ist,
- Pleurocidin-Analogon von SEQ. Nr. 126,
- Pleurocidin-Analogon von SEQ. Nr. 127, und
- synthetisches lytisches Peptid von SEQ. Nr. 128.

**4.** Das Fusionsprotein gemäß einem der Ansprüche 1 bis 3, welches zwischen Domäne (a) und Domäne (b) oder zwischen Domänen (b) Domäne (c) enthält, die eine Protease-Spaltstelle enthält, ausgewählt aus einer Sequenz, die von der Metalloprotease MMP erkannt wird, eine Sequenz die von Urokinase uPA erkannt wird, und eine Sequenz, die von Furin erkannt wird, und eine Sequenz, die von nativem Furin erkannt wird.

**5.** Das Fusionsprotein gemäß Anspruch 4, worin eine von der Metalloprotease MMP erkannte Sequenz Pro Leu Gly Leu Ala Gly ist, eine von Urokinase uPA erkannte Sequenz Arg Val Val Arg ist, eine von Furin erkannte Sequenz Arg Lys Lys Arg ist und eine durch natives Furin erkannte Sequenz Arg His Arg Gin Pro Arg Gly Trp Glu Gin Leu oder His Arg Gin Pro Arg Gly Trp Glu Gin ist.

**6.** Das Fusionsprotein gemäß Anspruch 4 oder 5, worin die Domäne (c) eine Kombination von nebeneinander angeordneten durch die Metalloprotease MMP erkannten Sequenz und durch die Urokinase uPA erkannten Sequenz ist.

**7.** Das Fusionsprotein gemäß einem der vorhergehenden Ansprüche, worin das Effektorpeptid der Domäne (b) zusätzlich mit der Transportdomäne (d) verbunden ist, ausgewählt aus der Gruppe bestehend aus:

- (d1) durch die Zellmembran transportierende Polyhistidinsequenz bestehend aus 6, 7, 8, 9, 10 oder 11 His-Resten, und
(d2) durch die Zellmembran transportierende Polyarginin-Sequenz bestehend aus 6, 7, 8, 9, 10 oder 11 Arg-Resten,
(d3) PD4-Transportisequenz (Protein Transduktionsdomäne 4) Tyr Ala Arg Ala Ala Ala Arg Gin Ala Arg Ala,
(d4) eine Transportsequenz bestehend aus Transferrinrezeptorbindungssequenz Thr His Arg Pro Pro Met Trp Ser Pro Val Trp Pro, und
(d5) PD5 Transportsequenz (Protein Transduktionsdomäne 5, TAT-Protein) Tyr Gly Arg Lys Lys Arg Arg Gin Arg Arg Arg,

und Kombinationen davon.

**8.** Das Fusionsprotein gemäß Anspruch 7, worin die Sequenz (d) am C-Terminus oder N-Terminus der Effektor-Peptid-Domäne (b) angeordnet ist.

**9.** Das Fusionsprotein gemäß Anspruch 7, worin die Transportdomäne (d) zwischen Domäne (b) und Domäne (c) oder zwischen Domäne (a) und Domäne (c) oder zwischen zwei Domänen (c) angeordnet ist.

**10.** Das Fusionsprotein gemäß Anspruch 7, worin die Sequenz (d) am C-Terminus des Fusionsproteins angeordnet ist.

**11.** Das Fusionsprotein gemäß einem der Ansprüche 4 bis 10, das zwischen zwei (c) Domänen die Domäne (e) enthält, die ein Linker zur Bindung des PEG-Moleküls ist, ausgewählt aus Ala Ser Gly Cys Gly Pro Glu Gly und Ala Ser Gly Cys Gly Pro Glu.

**12.** Das Fusionsprotein gemäß einem der Ansprüche 4 bis 11, das zusätzlich einen flexiblen sterischen Linker zwischen den Domänen (a), (b) und / oder (c) umfasst.

**13.** Fusionsprotein nach Anspruch 12, worin der sterische Linker aus Gly Gly, Gly Gly Gly, Gly Ser Gly, Gly Gly Gly Gly Ser, Gly Gly Gly Gly Gly Ser, Gly Gly Ser Gly Gly, Gly Gly Gly Ser Gly Gly Gly, Gly Gly Gly Gly Ser Gly, Gly Gly Gly Ser Gly Gly Gly Gly Gly Ser, Gly Gly Gly Gly Ser Gly Gly Gly Gly, Gly Ser Gly Gly Gly Ser Gly Gly Gly, Cys Ala Ala Cys Ala Ala Ala Cys, Cys Ala Ala Ala Cys Ala Ala Cys, Ser Gly Gly, einzigen Glycinrest Gly und einzigen Cysteinrest Cys und Kombinationen davon ausgewählt ist.

**14.** Das Fusionsprotein gemäß Anspruch 1 mit der Aminosäuresequenz ausgewählt aus der Gruppe bestehend aus SEQ. Nr. 4; SEQ. Nr. 5; SEQ. Nr. 6; SEQ. Nr. 11; SEQ. Nr. 12; SEQ. Nr. 13; SEQ. Nr. 14; SEQ. Nr. 15; SEQ. Nr. 16; SEQ. Nr. 18; SEQ. Nr. 19; SEQ. Nr. 20; SEQ. Nr. 21; SEQ. Nr. 22; SEQ. Nr. 23; SEQ. Nr. 30; SEQ. Nr. 31; SEQ. Nr. 32; SEQ. Nr. 91; SEQ. Nr. 92; SEQ. Nr. 93; SEQ. Nr. 94; SEQ. Nr. 95; SEQ. Nr. 96; SEQ. Nr. 97, SEQ. Nr. 98; und SEQ. Nr. 102.

**15.** Das Fusionsprotein gemäß einem der vorhergehenden Ansprüche, das ein rekombinantes Protein ist.

**16.** Eine pharmazeutische Zusammensetzung, umfassend als aktiven Wirkstoff ein in einem der Ansprüche 1 bis 15 definiertes Fusionsprotein in Kombination mit einem pharmazeutisch akzeptablen Träger.

**17.** Die pharmazeutische Zusammensetzung gemäß Anspruch 16 in einer Form zur parenteralen Verabreichung.

**18.** Das Fusionsprotein gemäß einem der Ansprüche 1 bis 15 zur Verwendung bei der Behandlungsverfahren von neoplastischen Erkrankungen in Säugetieren, einschließlich Menschen.

## Revendications

**1.** Une protéine de fusion comprenant :

- le domaine (a) qui est un fragment fonctionnel de la séquence de la protéine hTRAIL soluble, ladite séquence de la protéine hTRAIL étant présentée comme SEQ. N° 90, lequel fragment commence avec un acide dans la gamme de hTRAIL95 à hTRAIL121, inclus, et se termine par l'acide aminé à la position hTRAIL281, ou un homologue dudit fragment fonctionnel ayant au moins 70 % d'identité de séquence, de préférence 85 % d'identité, ledit fragment fonctionnel ou leur homologue étant capable d'induire l'apoptose après son liaison à ses récepteurs sur la surface cellulaire, et
- au moins un domaine (b) qui est la séquence d'un peptide effecteur cytolytique avec conformation de l'hélice alpha amphipathique, formant des pores dans la membrane cellulaire,

dans lequel la séquence du domaine (b) est attachée à l'extrémité C-terminale ou N-terminale du domaine (a),

**2.** La protéine de fusion selon la revendication 1, dans laquelle le domaine (a) est choisi dans le groupe consistant en hTRAIL95-281, hTRAIL114-281, hTRAIL115-281, hTRAIL116-281, hTRAIL119-281 et hTRAIL121-281.

**3.** La protéine de fusion selon les revendications 1 ou 2, dans laquelle le domaine (b) est choisi dans le groupe consistant en :

- pilosuline-1 de SEQ. N° 36,
- pilosuline-5 de SEQ. N° 37,
- peptide lytique synthétique à 14 acides aminés de SEQ. N° 41,
- peptide à 27 acides aminés FFhCAP18 de SEQ. N° 43,
- BAMP-28 peptide de SEQ. N° 44,
- analogue de l'isoforme C du peptide lytique d'Entamoeba histolytica de SEQ. N° 45,
- analogue de l'isoforme A du peptide lytique de Entamoeba histolytica SEQ. N° 46,
- analogue de l'isoforme B du peptide lytique d'Entamoeba histolytica de SEQ. N° 47,
- fragment du domaine HA2 de l'hémagglutinine du virus de la grippe de SEQ. N° 48,
- fragment actif de perforation humaine de SEQ. N° 54,
- parasporin-2 z Bacillus thuringensis de SEQ. N° 55,
- protéine de fusion comprenant le peptide lytique synthétique avec le motif KLLK et le peptide étant l'antagoniste du récepteur PDGF de SEQ. N° 125,
- analogue de pleurocidine de SEQ. N° 126,

- analogue de pleurocidine de SEQ. N° 127, et
- peptide lytique synthétique de SEQ. N° 128.

4. La protéine de fusion selon l'une quelconque des revendications 1 à 3, qui entre le domaine (a) et le domaine (b) ou entre les domaines (b) contient un site de clivage protéase contenant le domaine (c), choisi parmi une séquence reconnue par la métalloprotéase MMP, séquence reconnue par l'urokinase uPA, et séquence reconnue par la furine et une séquence reconnue par la furine native.

5. La protéine de fusion selon la revendication 4, dans laquelle une séquence reconnue par la métalloprotéase MMP est Pro Leu Gly Leu Ala Gly, une séquence reconnue par l'urokinase uPA est Arg Val Val Arg, une séquence reconnue par la furine est Arg Lys Lys Arg et une séquence reconnue par la furine native est Arg His Arg Gin Pro Arg Gly Trp Glu Gin Leu ou His Arg Gin Pro Arg Gly Trp Glu Gin.

6. La protéine de fusion selon la revendication 4 ou 5, dans laquelle le domaine (c) est une combinaison de séquence reconnue par la métalloprotéase MMP et une séquence reconnue par l'urokinase uPA située l'une à côté de l'autre.

7. La protéine de fusion selon l'une quelconque des revendications précédentes, dans laquelle le peptide effecteur du domaine (b) est en outre relié au domaine de transport (d), choisi dans le groupe consistant en :

   - (d1) séquence de polyhistidine transportant à travers la membrane cellulaire comprenant 6, 7, 8, 9, 10 ou 11 His résidus, et
   (d2) séquence de polyarginine transportant à travers la membrane cellulaire, consistant en 6, 7, 8, 9, 10 ou 11 Arg résidus,
   (d3) séquence de transport PD4 (protéine domaine de transduction 4) Tyr Ala Arg Ala Ala Ala Arg Gin Ala Arg Ala,
   (d4) séquence de transport consistant en une séquence de liaison au récepteur de transferrine Thr His Arg Pro Pro Met Trp Ser Pro Val Trp Pro, et
   (d5) séquence de transport PD5 (protéine domaine de transduction 5, protéine TAT) Tyr Gly Arg Lys Lys Arg Arg Gin Arg Arg Arg, et leurs combinaisons.

8. La protéine de fusion selon la revendication 7, dans laquelle la séquence (d) est située à l'extrémité C-terminale ou N-terminale du domaine du peptide effecteur (b).

9. La protéine de fusion selon la revendication 7, dans laquelle le domaine de transport (d) est situé entre le domaine (b) et le domaine (c), ou entre le domaine (a) et le domaine (c), ou entre deux domaines (c).

10. La protéine de fusion selon la revendication 7, dans laquelle la séquence (d) est située à l'extrémité C-terminale de la protéine de fusion.

11. Protéine de fusion selon l'une quelconque des revendications 4 à 10, qui entre deux domaines (c) contient un domaine (d) qui est un lieur pour la fixation de la molécule de PEG, choisi parmi Ala Ser Gly Cys Gly Pro Glu Gly et Ala Ser Gly Cys Gly Pro Glu.

12. La protéine de fusion selon l'une quelconque des revendications 4 à 11, qui comprend en outre un lieur stérique flexible entre les domaines (a), (b) et/ou (c).

13. La protéine de fusion selon la revendication 12, dans laquelle le lieur stérique est choisi parmi Gly Gly, Gly Gly Gly, Gly Ser Gly, Gly Gly Gly Gly Ser, Gly Gly Gly Gly Gly Ser, Gly Gly Ser Gly Gly, Gly Gly Gly Ser Gly Gly Gly, Gly Gly Gly Gly Ser Gly, Gly Gly Gly Ser Gly Gly Gly Gly Gly Ser, Gly Gly Gly Gly Ser Gly Gly Gly Gly, Gly Ser Gly Gly Gly Ser Gly Gly Gly, Cys Ala Ala Cys Ala Ala Ala Cys, Cys Ala Ala Ala Cys Ala Ala Cys, Ser Gly Gly, un seul résidu de glycine Gly, et un seul résidu de cystéine Cys, et leurs combinaisons.

14. La protéine de fusion selon la revendication 1, ayant la séquence d'acides aminés choisie dans le groupe consistant en la SEQ. N° 4 ; SEQ. N° 5 ; SEQ. N° 6 ; SEQ. N° 11 ; SEQ. N° 12 ; SEQ. N° 13 ; SEQ.n ° 14 ; SEQ. N° 15 ; SEQ. N° 16 ; SEQ. N° 18 ; SEQ. N° 19 ; SEQ. N° 20 ; SEQ. N° 21 ; SEQ. N° 22 ; SEQ. N° 23 ; SEQ. N° 30 ; SEQ. N° 31 ; SEQ. N° 32 ; SEQ. N° 91 ; SEQ. N° 92 ; SEQ. N° 93 ; SEQ. N° 94 ; SEQ. N° 95 ; SEQ. N° 96 ; SEQ. N° 97 ; SEQ. N° 98 ; et SEQ. N° 102.

15. La protéine de fusion selon l'une quelconque des revendications précédentes, qui est une protéine recombinante.

**16.** Composition pharmaceutique comprenant comme ingrédient actif la protéine de fusion selon l'une quelconque des revendications 1 à 15, en combinaison avec un support pharmaceutiquement acceptable.

**17.** Composition pharmaceutique selon la revendication 16 sous une forme pour l'administration parentérale.

**18.** La protéine de fusion selon l'une quelconque des revendications 1 à 15 pour une utilisation dans le traitement de maladies néoplasiques chez les mammifères, y compris les humains.

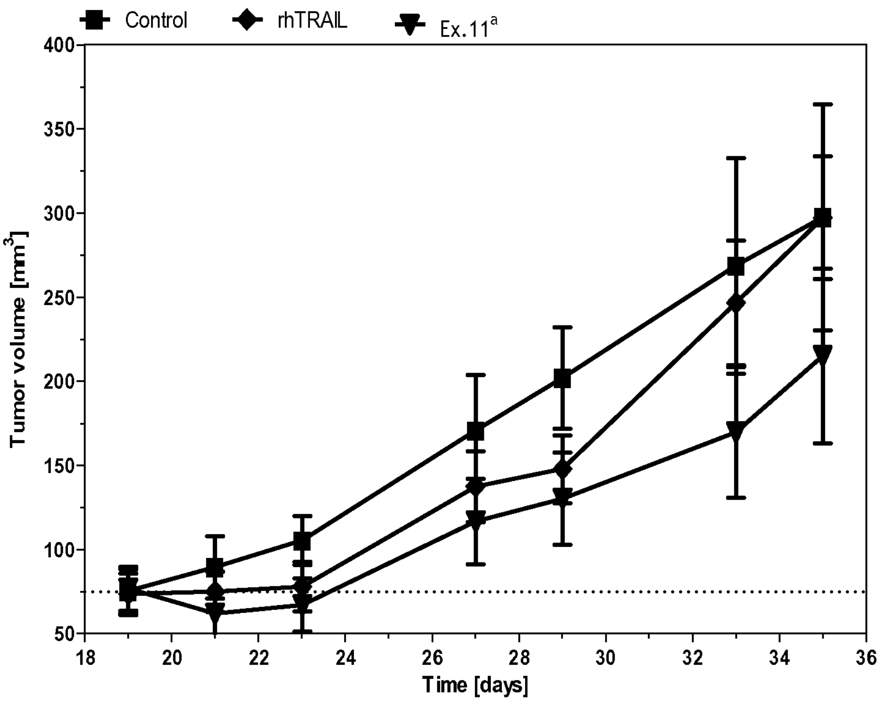
Control
rhTRAIL
Ex.11a
Tumor volume [mm3]
Time [days]
400
350
300
250
200
150
100
50
18
20
22
24
26
28
30
32
34
36

Fig. 1

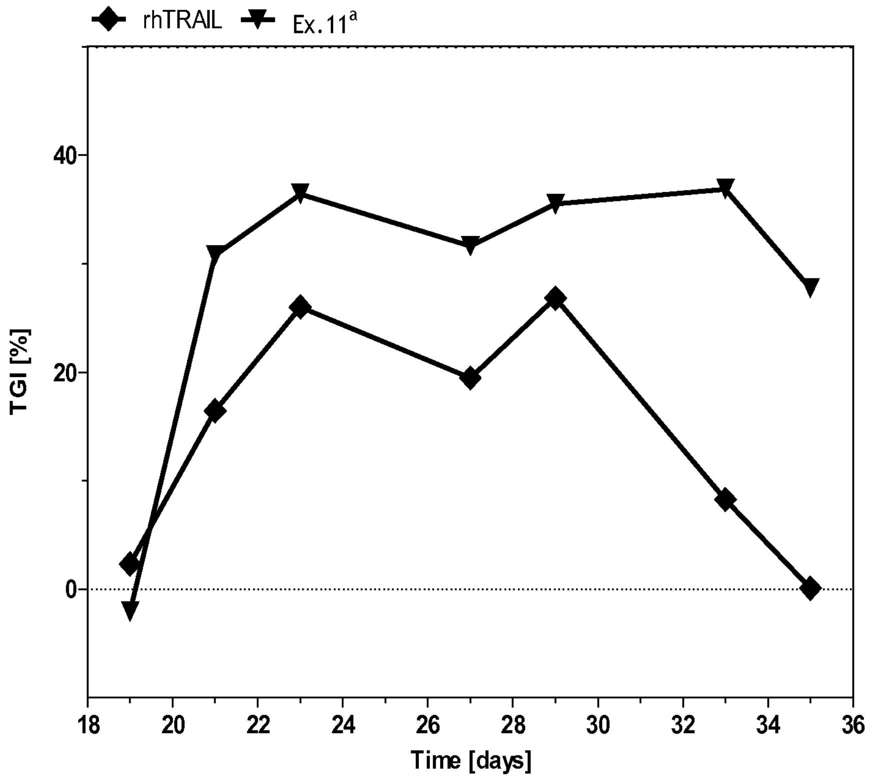
rhTRAIL
Ex.11a
TGI [%]
Time [days]
40
20
0
18
20
22
24
26
28
30
32
34
36

Fig. 2

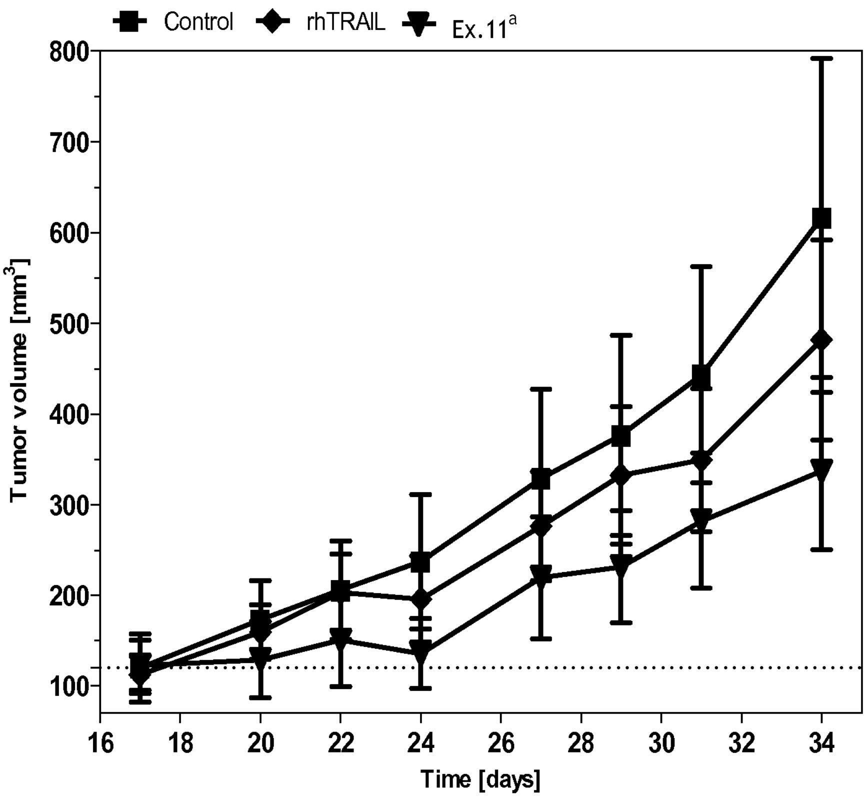
Control
rhTRAIL
Ex.11a
Tumor volume [mm3]
800
700
600
500
400
300
200
100
16
18
20
22
24
26
28
30
32
34
Time [days]

Fig. 3

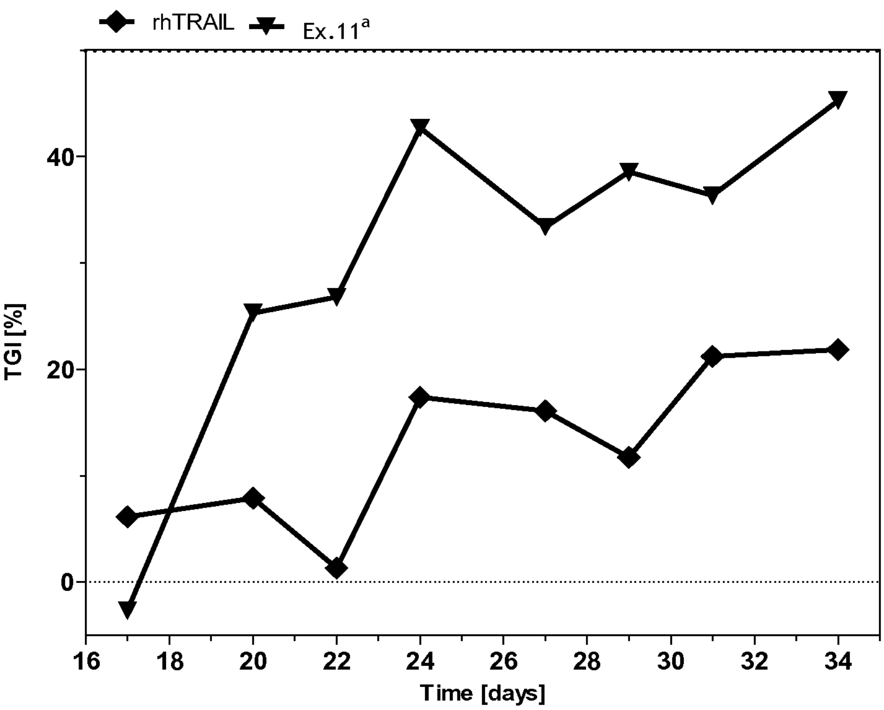
rhTRAIL
Ex.11a
TGI [%]
40
20
0
16
18
20
22
24
26
28
30
32
34
Time [days]

Fig. 4

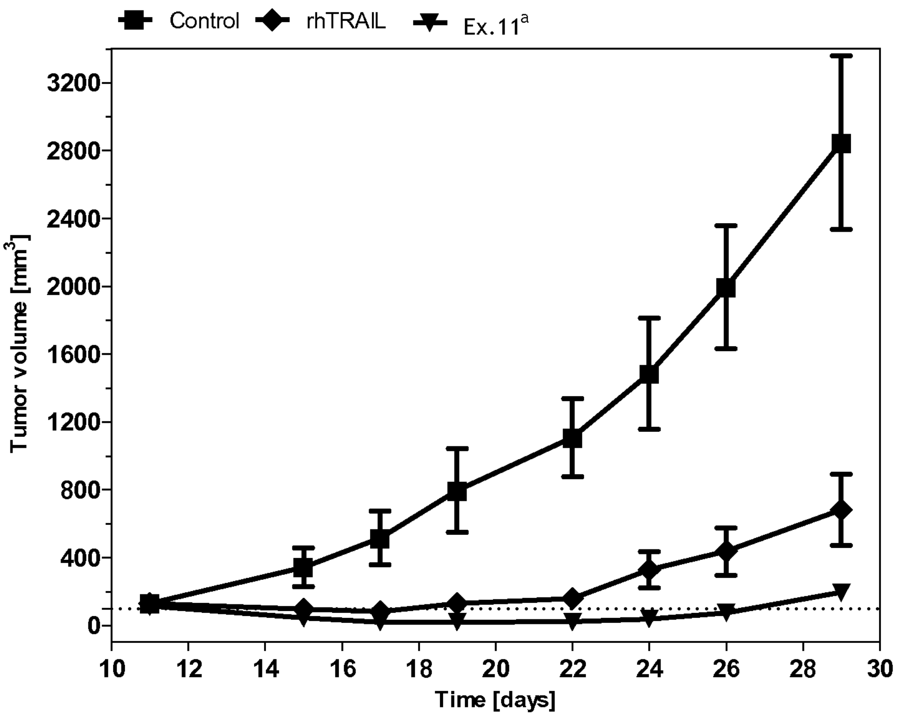
Control
rhTRAIL
Ex.11a
3200
2800
2400
2000
1600
1200
800
400
0
Tumor volume [mm3]
10
12
14
16
18
20
22
24
26
28
30
Time [days]

Fig. 5

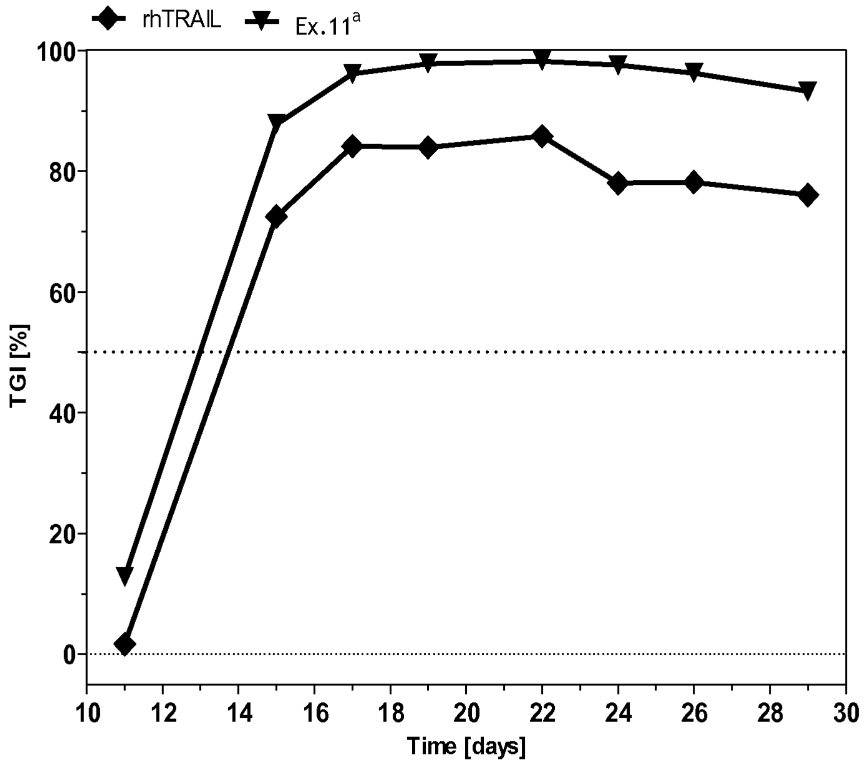
rhTRAIL
Ex.11a
100
80
60
40
20
0
TGI [%]
10
12
14
16
18
20
22
24
26
28
30
Time [days]

Fig. 6

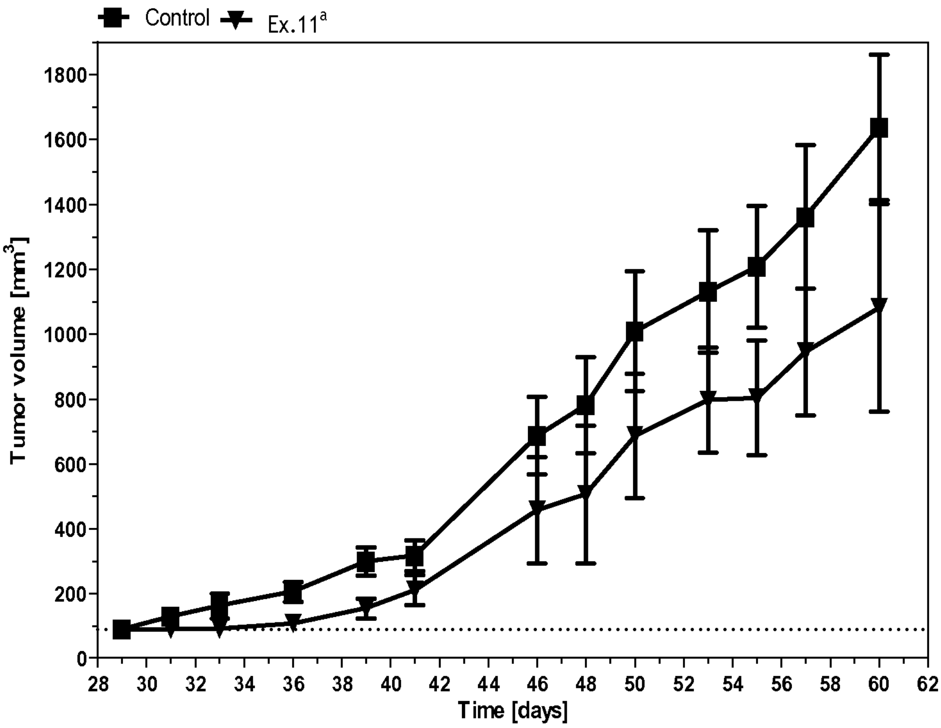
Control
Ex.11a
Tumor volume [mm3]
0
200
400
600
800
1000
1200
1400
1600
1800
28 30 32 34 36 38 40 42 44 46 48 50 52 54 56 58 60 62
Time [days]

Fig. 7

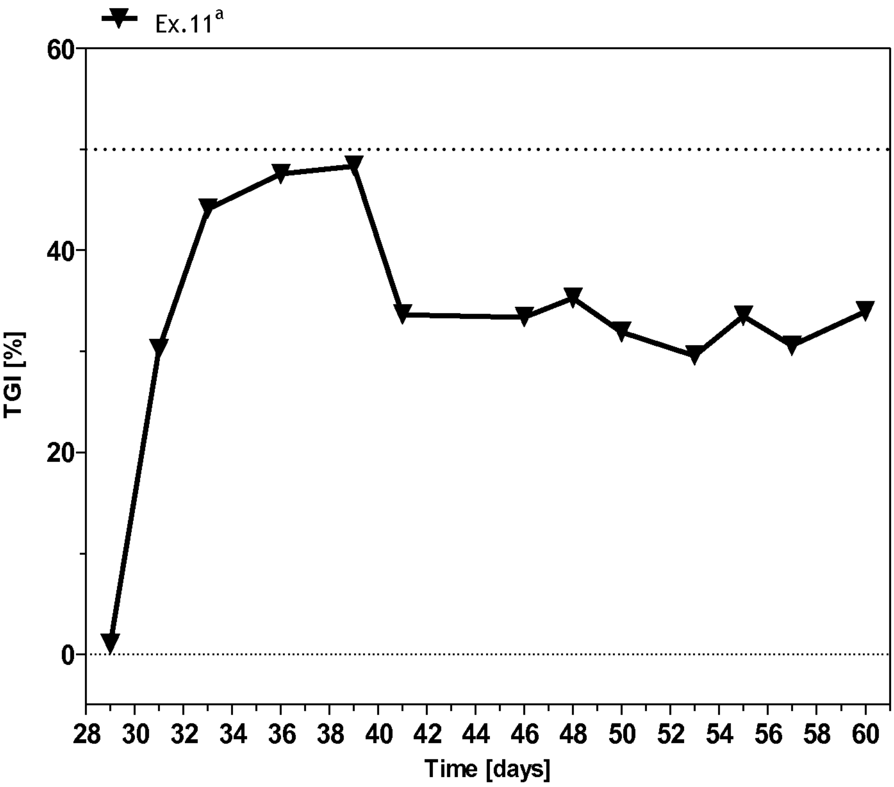
Ex.11a
TGI [%]
0
20
40
60
28 30 32 34 36 38 40 42 44 46 48 50 52 54 56 58 60
Time [days]

Fig. 8

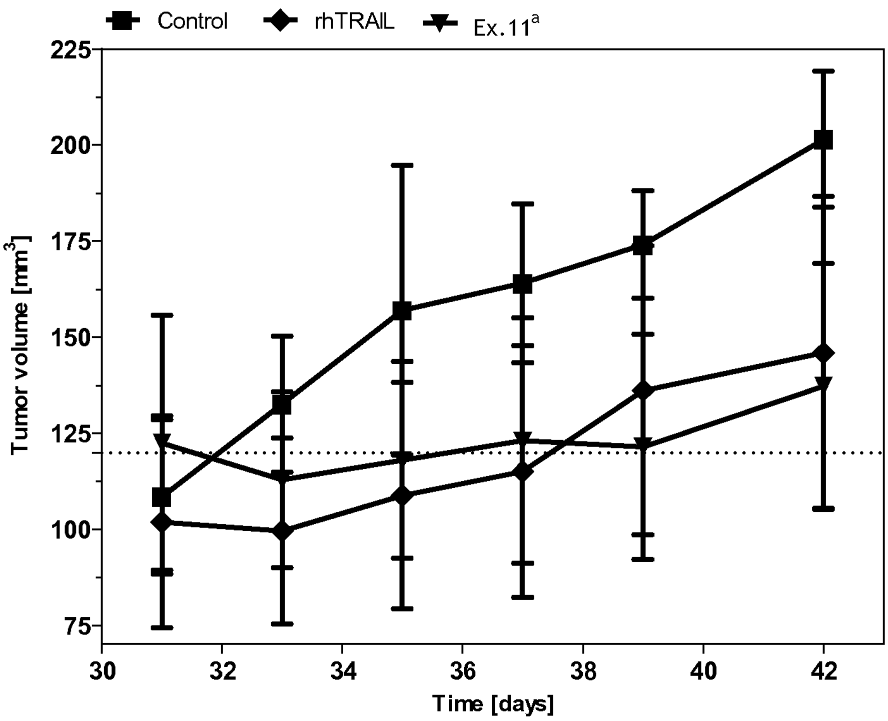
Control
rhTRAIL
Ex.11a
225
200
175
150
125
100
75
Tumor volume [mm3]
30
32
34
36
38
40
42
Time [days]

Fig. 9

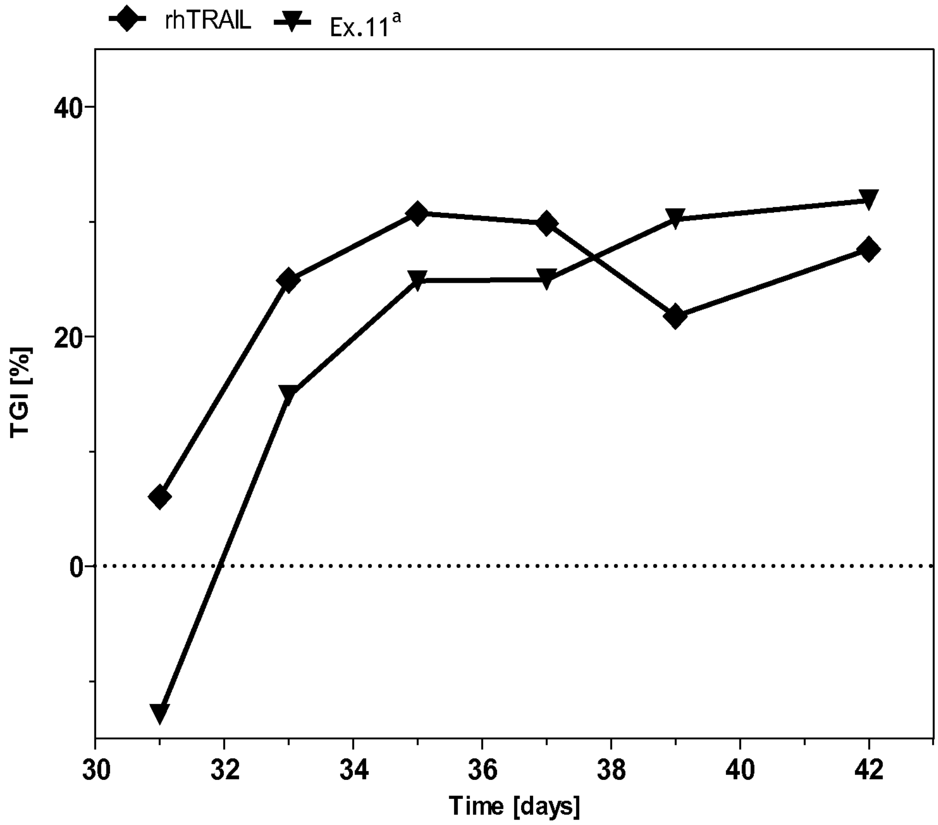
rhTRAIL
Ex.11a
40
20
0
TGI [%]
30
32
34
36
38
40
42
Time [days]

Fig. 10

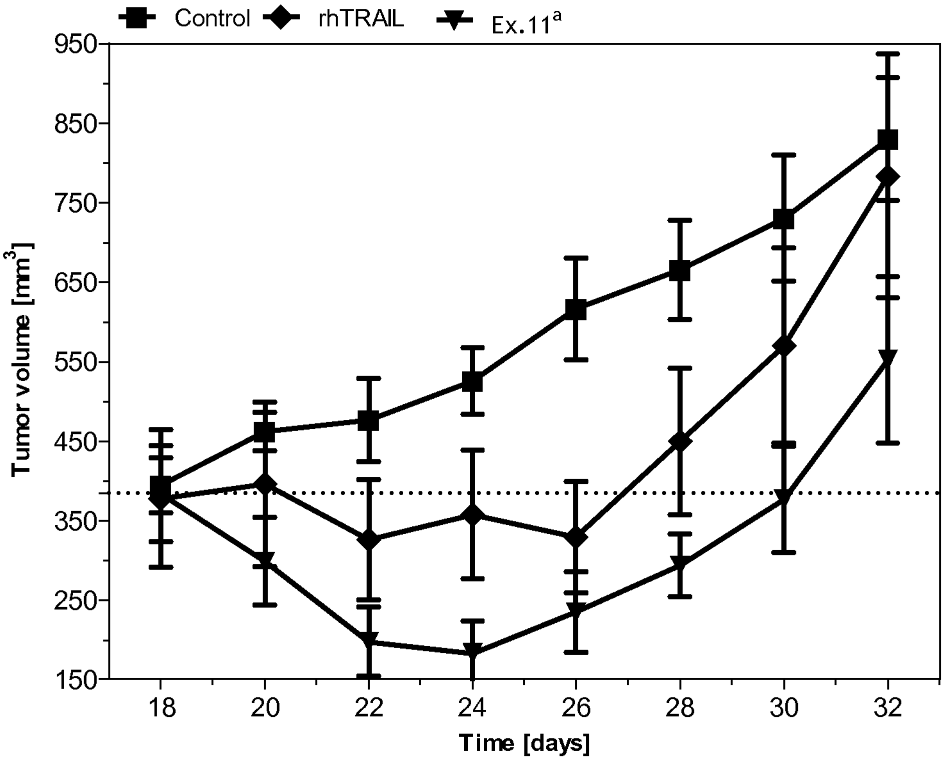
Control
rhTRAIL
Ex.11a
Tumor volume [mm3]
950
850
750
650
550
450
350
250
150
18
20
22
24
26
28
30
32
Time [days]

Fig. 11

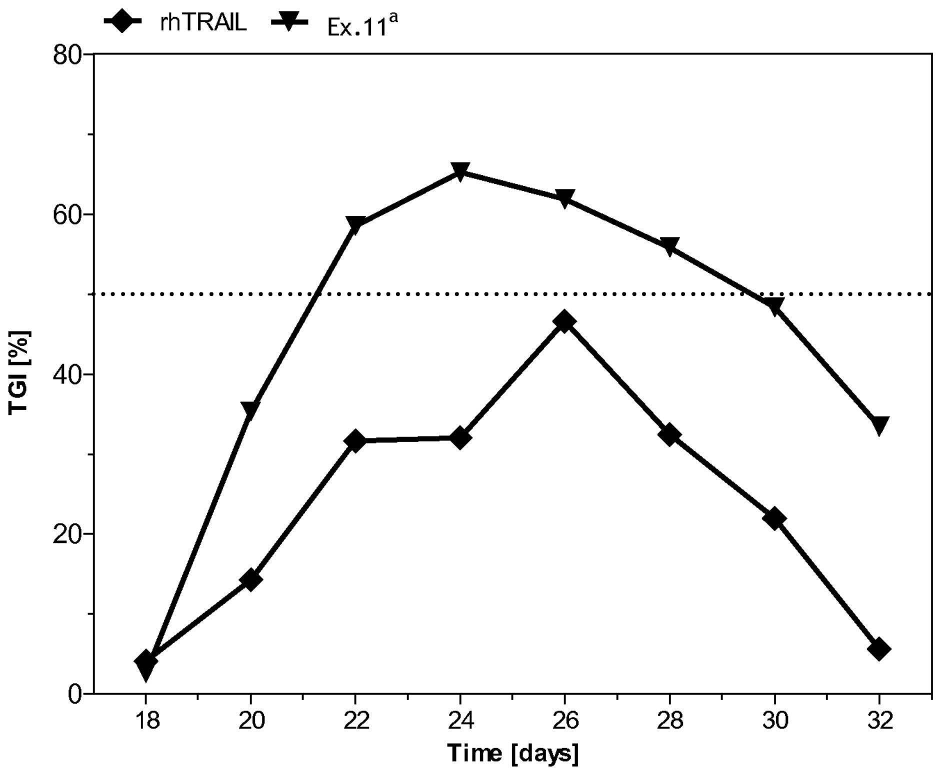
rhTRAIL
Ex.11a
TGI [%]
80
60
40
20
0
18
20
22
24
26
28
30
32
Time [days]

Fig.12

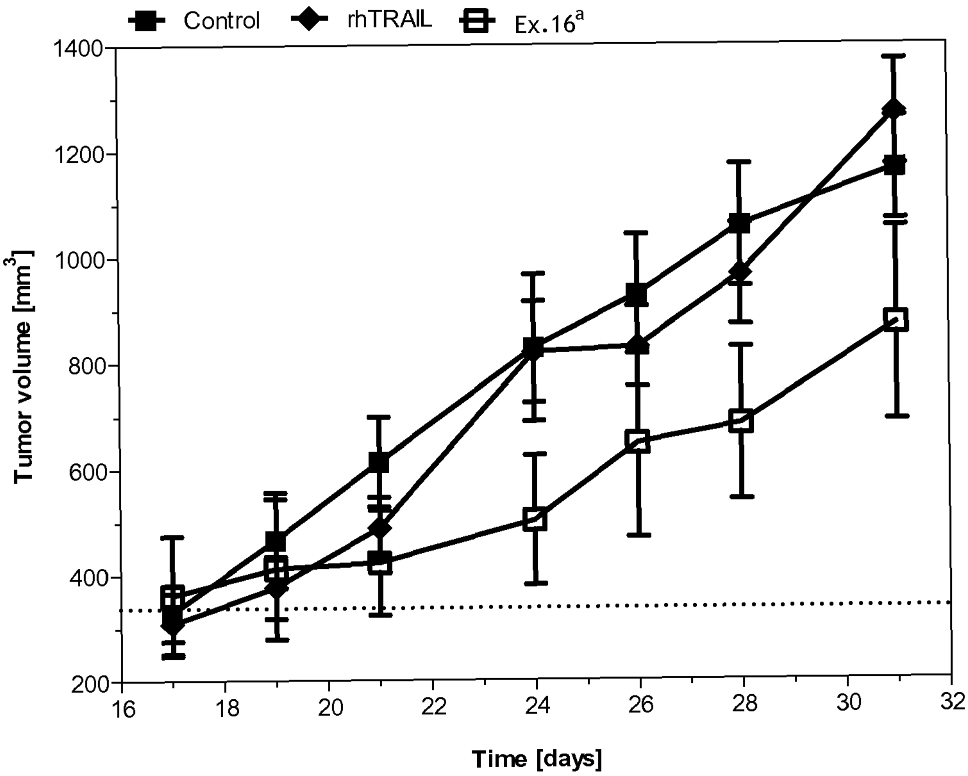
Control
rhTRAIL
Ex.16a
1400
1200
1000
800
600
400
200
Tumor volume [mm3]
16
18
20
22
24
26
28
30
32
Time [days]

Fig. 13

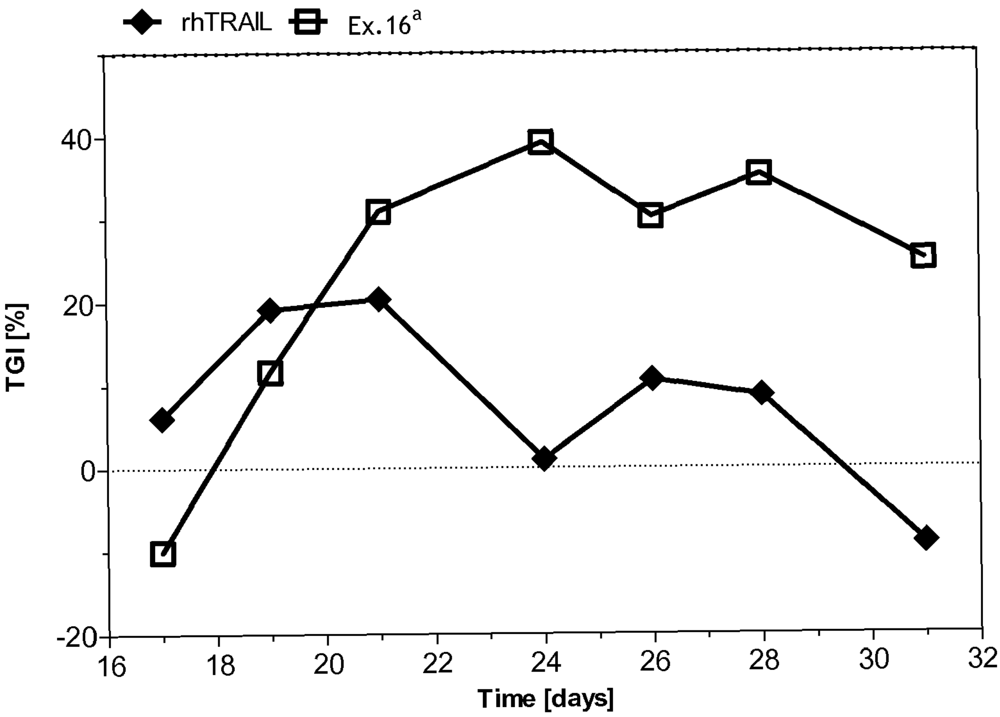
rhTRAIL
Ex.16a
40
20
0
-20
TGI [%]
16
18
20
22
24
26
28
30
32
Time [days]

Fig. 14

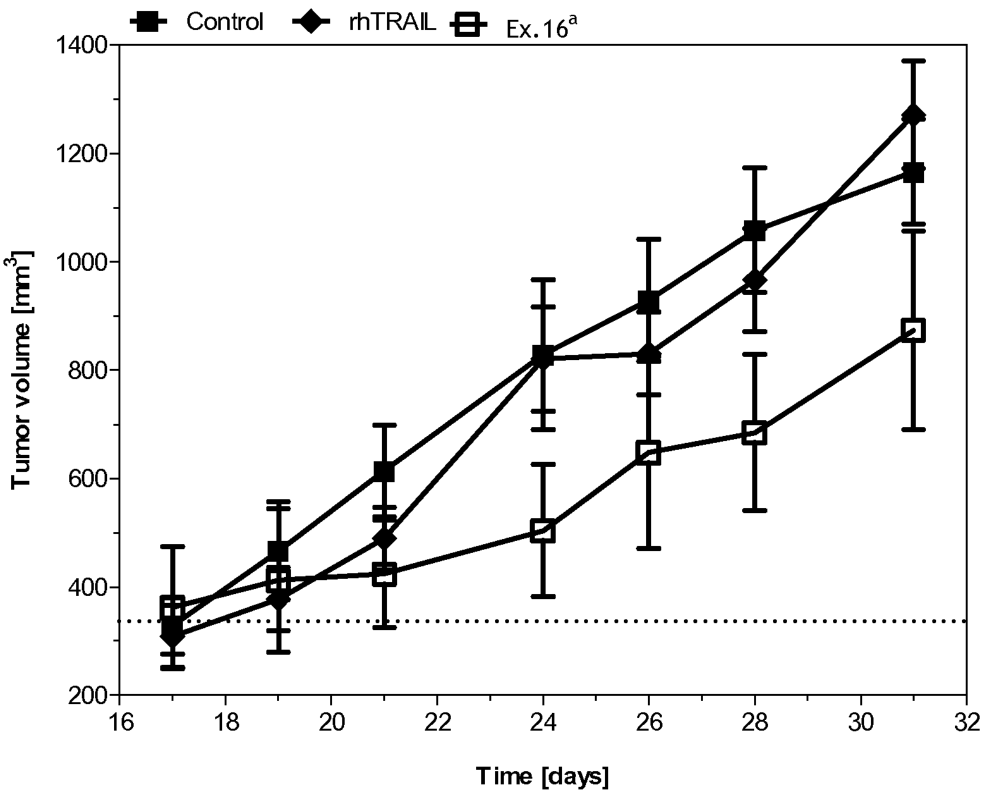

Control
rhTRAIL
Ex.16[a]
Tumor volume [mm3]
Time [days]


Fig. 15

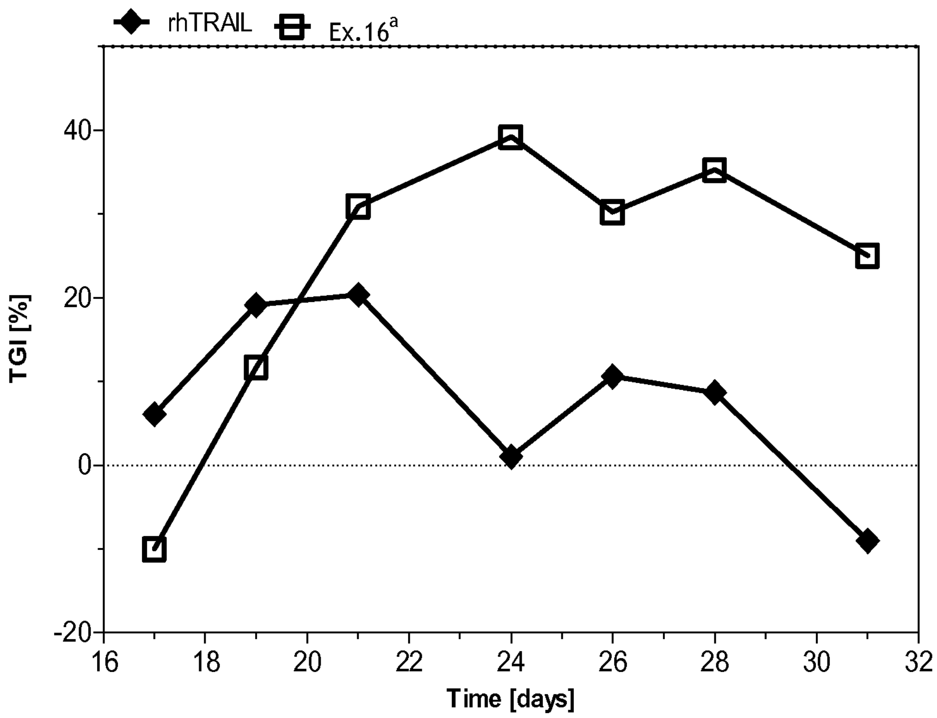

rhTRAIL
Ex.16[a]
TGI [%]
Time [days]


Fig. 16

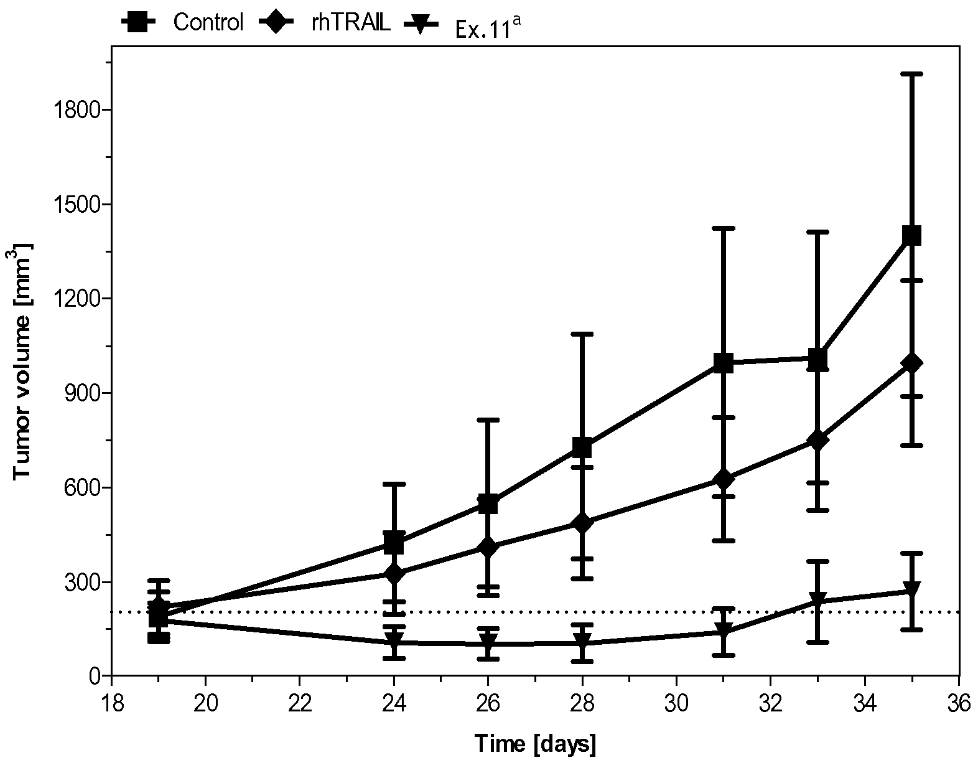
Control
rhTRAIL
Ex.11a
Tumor volume [mm3]
1800
1500
1200
900
600
300
0
18
20
22
24
26
28
30
32
34
36
Time [days]

Fig. 17

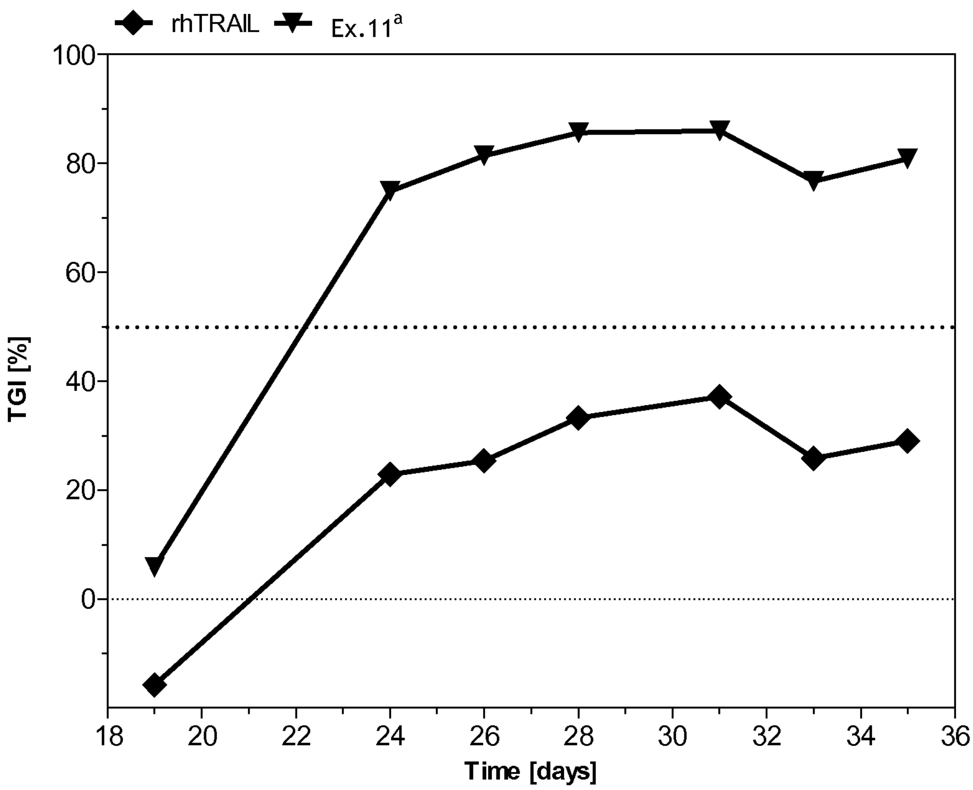
rhTRAIL
Ex.11a
TGI [%]
100
80
60
40
20
0
18
20
22
24
26
28
30
32
34
36
Time [days]

Fig. 18

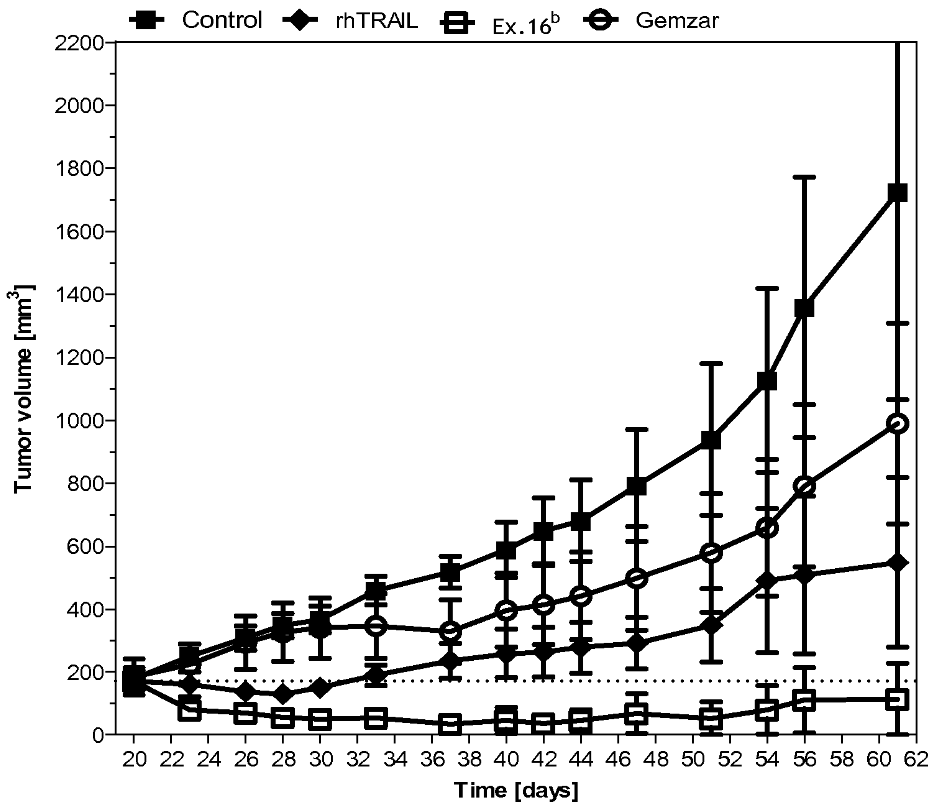
Control
rhTRAIL
Ex.16b
Gemzar
Tumor volume [mm3]
Time [days]

Fig. 19

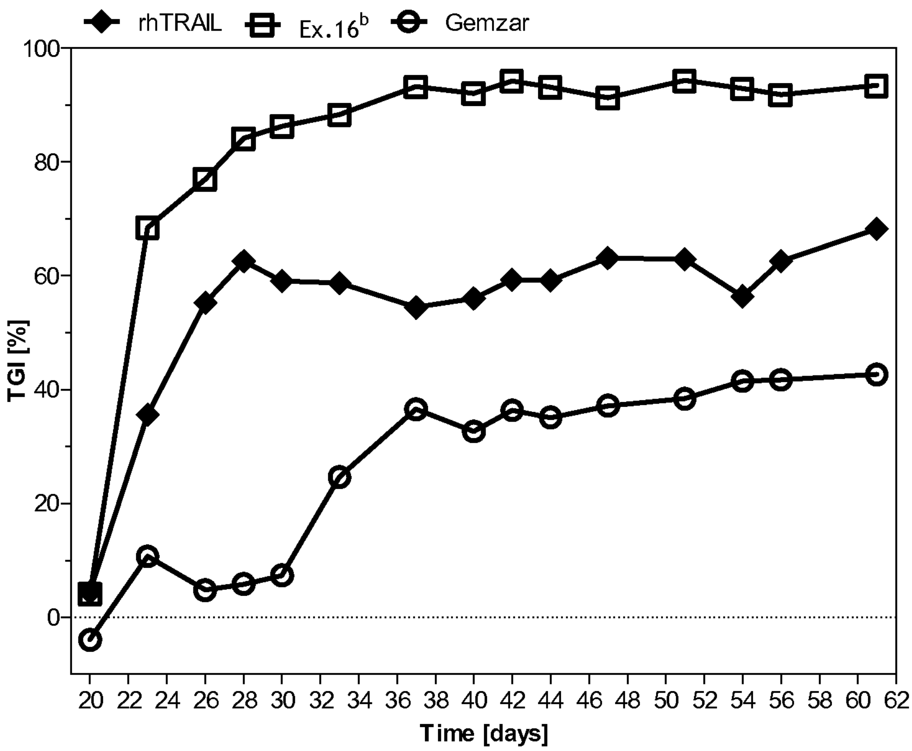
rhTRAIL
Ex.16b
Gemzar
TGI [%]
Time [days]

Fig. 20

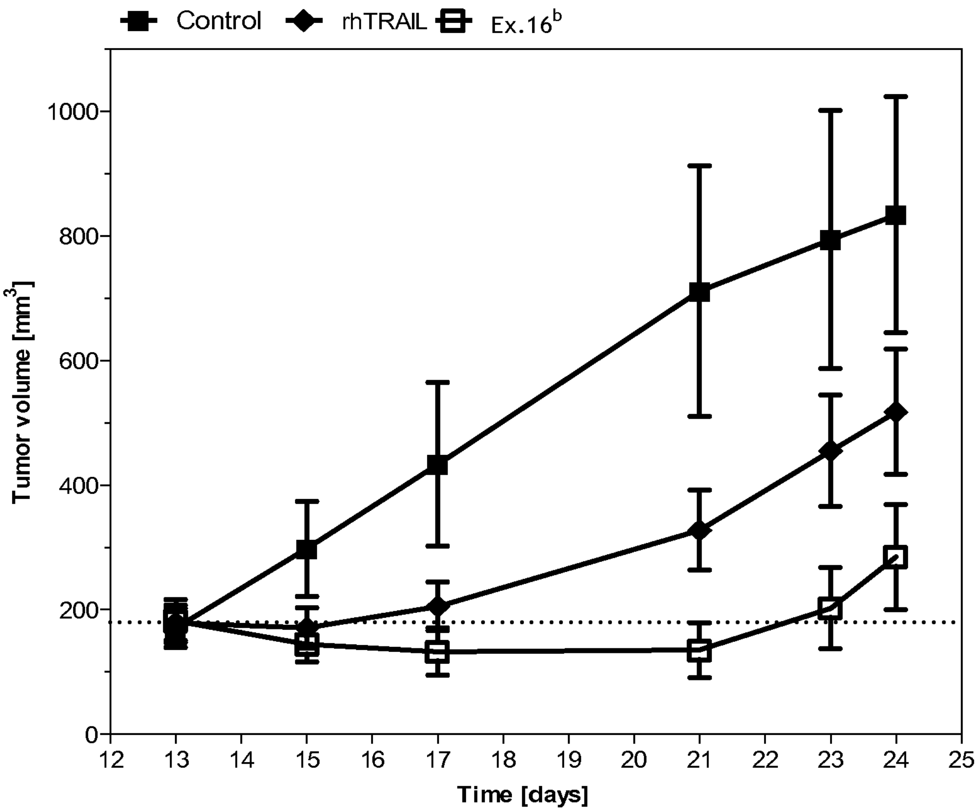
Control
rhTRAIL
Ex.16b
Tumor volume [mm3]
1000
800
600
400
200
0
12
13
14
15
16
17
18
19
20
21
22
23
24
25
Time [days]

Fig. 21

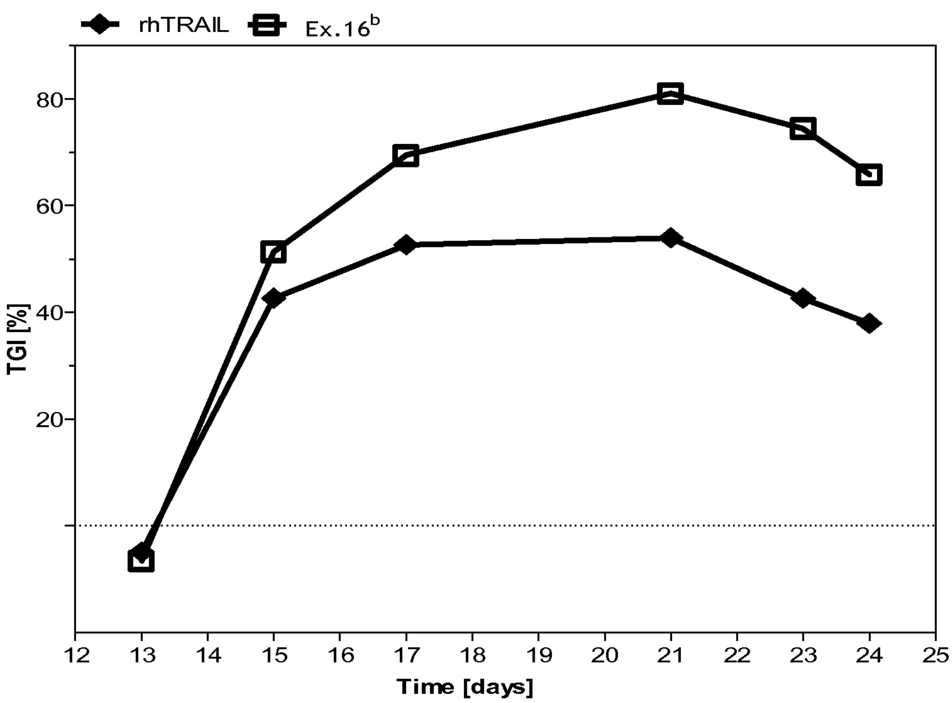
rhTRAIL
Ex.16b
TGI [%]
80
60
40
20
12
13
14
15
16
17
18
19
20
21
22
23
24
25
Time [days]

Fig. 22

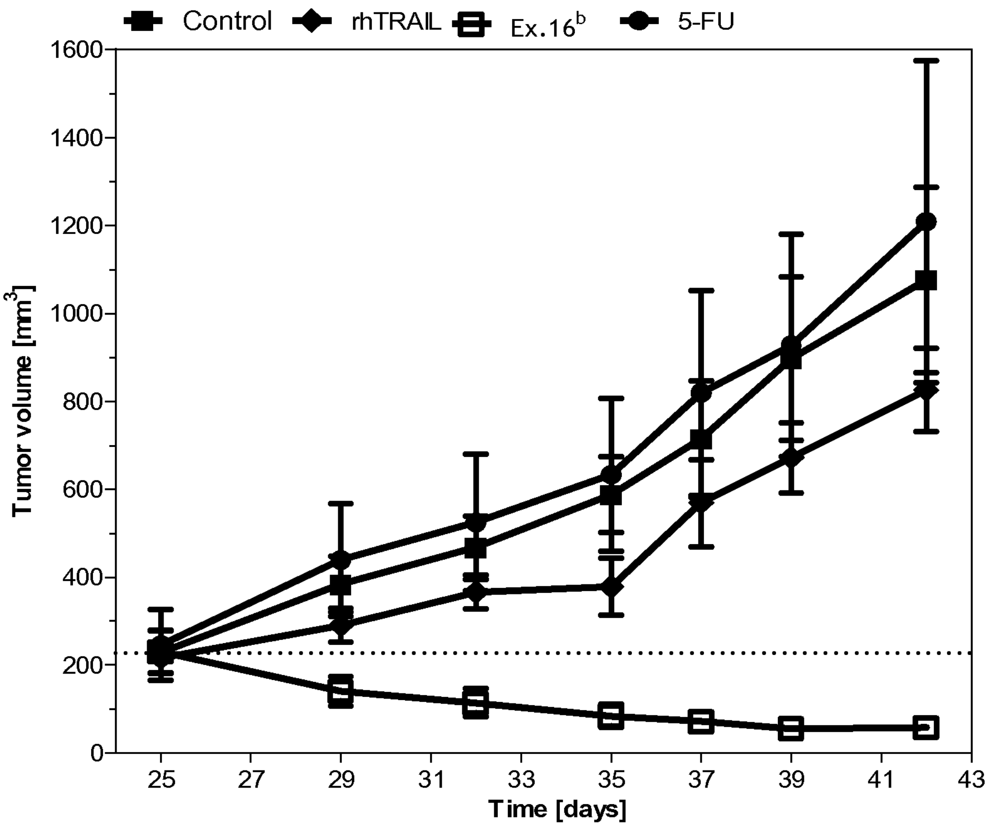

Control
rhTRAIL
Ex.16b
5-FU
Tumor volume [mm3]
1600
1400
1200
1000
800
600
400
200
0
25
27
29
31
33
35
37
39
41
43
Time [days]


Fig. 23

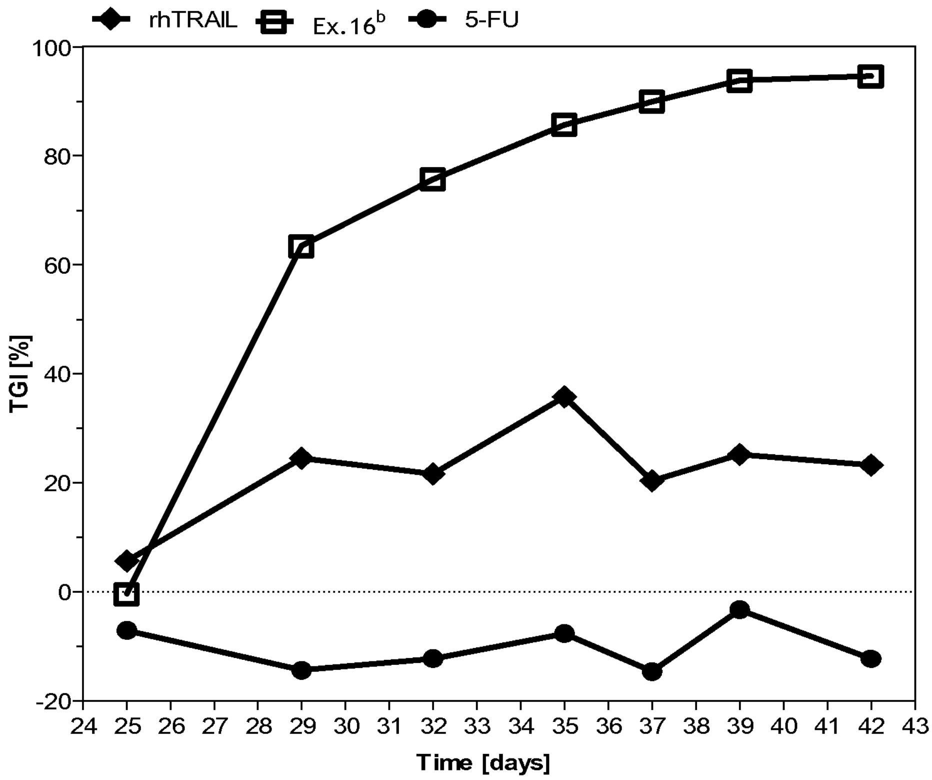

rhTRAIL
Ex.16b
5-FU
TGI [%]
100
80
60
40
20
0
-20
24 25 26 27 28 29 30 31 32 33 34 35 36 37 38 39 40 41 42 43
Time [days]


Fig. 24

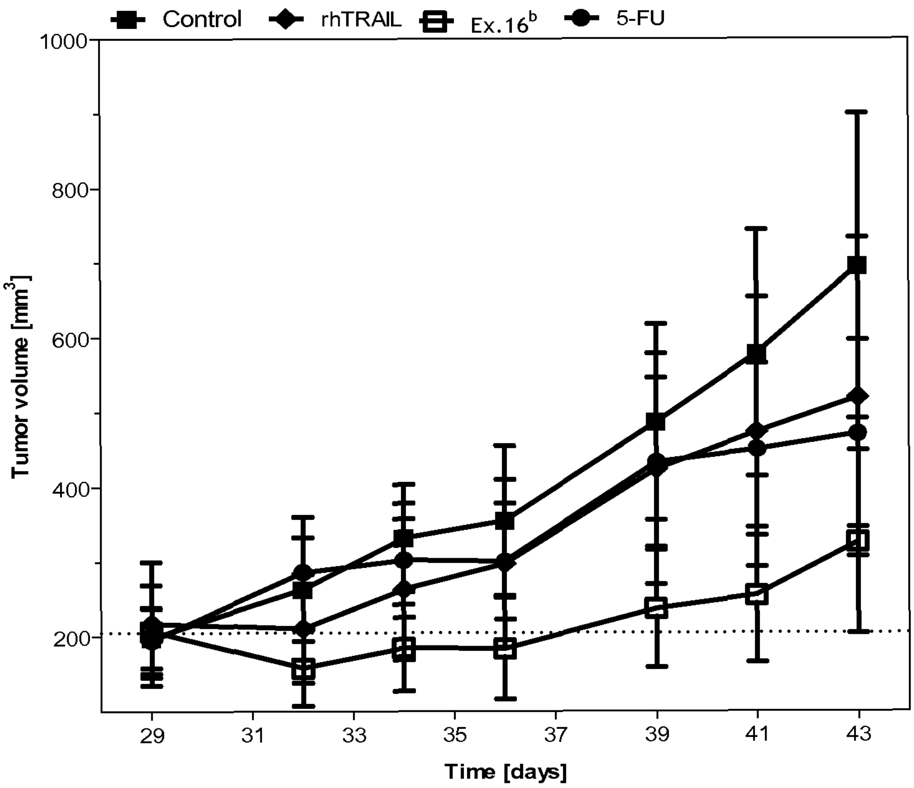
Control
rhTRAIL
Ex.16b
5-FU
1000
800
600
400
200
Tumor volume [mm3]
29
31
33
35
37
39
41
43
Time [days]

Fig. 25

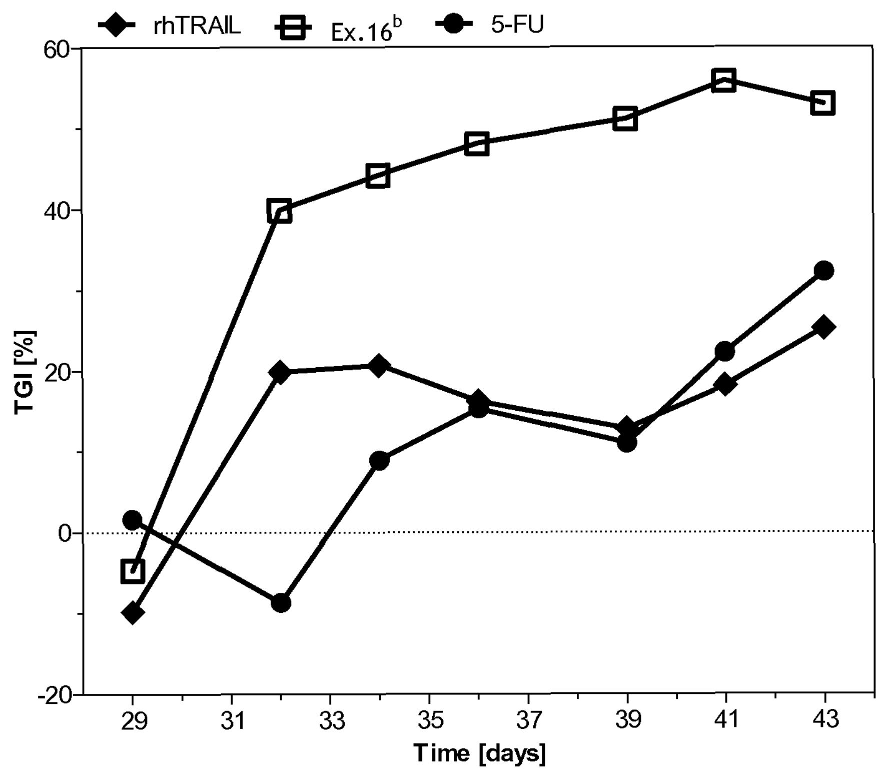
rhTRAIL
Ex.16b
5-FU
60
40
20
0
-20
TGI [%]
29
31
33
35
37
39
41
43
Time [days]

Fig. 26

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0835305 A1
- EP 1688498 A
- WO 2007022214 A
- WO 2009002947 A
- WO 2009140469 A
- US 5817771 B1
- WO 2009077857 A
- WO 2009066174 A
- WO 2010064207 A


### Non-patent literature cited in the description


- **THORBURN A ; BEHBAKHT K ; FORD H.** TRAIL receptor-targeted therapeutics: resistance mechanisms and strategies to avoid them. *Drug Resist Updat,* 2008, vol. 11, 17-24
- **A. ALMASAN ; A. ASHKENAZI.** *Cytokine Growth Factor Reviews,* 2003, vol. 14, 337-348
- **RK SRIVASTAVA.** *Neoplasis,* 2001, vol. 3 (6), 535-546
- **SORIA JC et al.** *J. Clin. Oncology,* 2010, vol. 28 (9), 1527-1533
- **REGEN et al.** *Biochem. Biophys. Res. Commun.,* 1989, vol. 159, 566-571
- **PANCHAL RG. et al.** Poreforming proteins and their application in biotechnology. *Curr Pharm Biotechnol,* 2002, vol. 3, 99-115
- **PANCHAL RG.** Novel therapeutic strategies to selectively kill cancer cells. *Biochem Pharmacol,* 1998, vol. 55, 247-252
- **HOSKIN DW ; RAMAMOORTHY A.** Studies on anticancer activities of antimicrobial peptides. *Biochim Biophys Acta-Biomembr,* 2008, vol. 1778, 357-87
- **SOLETTI RC.** Potentiation of anticancer-drug cytotoxicity by sea anemone pore-forming proteins in human glioblastoma cells. *Anti-Cancer Drugs,* 2008, vol. 19, 517-525
- **PEDERZOLLI C.** Biochemical and cytotoxic properties of conjugates of transferrin with equinatoxin-II, a cytolysin from a sea anemone. *Bioconjugate Chem,* 1995, vol. 6, 166-173
- **VAN DER SPEK JC.** Fusion protein toxins based on diphtheria toxin: Selective targeting of growth factor receptors of eukaryotic cells. *Appl Chimeric Genes Hybrid Proteins Pt B,* 2000, vol. 327, 239-249
- **TEJUCA M. et al.** Construction of an immunotoxin with the pore forming protein St1 and/or C5, a monoclonal antibody against a colon cancer cell line. *Int. Immunopharmacol.,* 2004, vol. 4, 731-744
- **MURPHY JR ; VAN DER SPEK JC.** Targeting diphtheria-toxin to growth-factor receptors. *Semin Cancer Biol,* 1995, vol. 6, 259-267
- **PANCHAL R. et al.** *Nat Biotechnol,* 1996, vol. 14, 852-856
- **WILLIAMS S.A. et al.** *JNCI J. Natl. Cancer Inst.,* 2007, vol. 99 (5), 376-385
- **BARUA et al.** *Cancer Letters,* 2010, vol. 293, 240-253
- **TUR V ; VAN DER SLOOT AM ; REIS CR ; SZEGEZDI E ; COOL RH ; SAMALI A ; SERRANO L ; QUAX WJ.** DR4-selective tumor necrosis factor-related apoptosis-inducing ligand (TRAIL) variants obtained by structure-based design. *J. Biol. Chem.,* 18 July 2008, vol. 283 (29), 20560-8
- **GASPARIAN ME ; CHERNYAK BV ; DOLGIKH DA ; YAGOLOVICH AV ; POPOVA EN ; SYCHEVA AM ; MOSHKOVSKII SA ; KIRPICHNIKOV MP.** Generation of new TRAIL mutants DR5-A and DR5-B with improved selectivity to death receptor 5. *Apoptosis.,* June 2009, vol. 14 (6), 778-87
- **GASPARIAN ME et al.** Generation of new TRAIL mutants DR5-A and DR5-B with improved selectivity to death receptor 5. *Apoptosis.,* June 2009, vol. 14 (6), 778-87
- **KOURIE et al.** *Am J Physiol Cell Physiol,* 2000, vol. 278, 1063-1087
- **JAVADPOUR et al.** *J Med Chem,* 1996, vol. 39, 3107-13
- **PAPO N ; SHAI Y.** New lytic peptides based on the D,L-amphipathic helix motif preferentially kill tumor cells compared to normal cells. *Biochemistry,* 12 August 2003, vol. 42 (31), 9346-54
- **ISOGAI E.** Antimicrobial and Lipopolysaccharide-Binding Activities of C-Terminal Domain of Human CAP18 Peptides to Genus Leptospira. *The Journal of Applied Research,* 2004, vol. 4 (1), 180-185

- **HUGOSSON, M., D. et al.** Antibacterial peptides and mitochondrial presequences affect mitochondrial coupling, respiration and protein import. *Eur. J. Biochem.,* 1994, vol. 223, 1027-1033
- **A. RISSO et al.** BMAP-28, an Antibiotic Peptide of Innate Immunity, Induces Cell Death through Opening of the Mitochondrial Permeability Transition Pore. *MOLECULAR AND CELLULAR BIOLOGY,* March 2002, 1926-1935
- **LEIPPE, M et al.** *EMBO J.,* 1992, vol. 11, 3501-3506
- **LEIPPE, M. ; MÜLLER-EBERHARD, H.J.** *Toxicology,* 18 May 1994, vol. 87
- **ANDRÄ et al.** *FEBS Letters,* 1996, vol. 385, 96-100
- **DÜRRER P et al.** *J Biol Chem,* 1996, vol. 271, 13417-13421
- **TAKAHASHI S.** *Biochemistry,* 1990, vol. 29, 6257-6264
- **FRANKEL AE.** Reducing the immune response to immunotoxin. *Clin Cancer Res.,* 01 January 2004, vol. 10 (1), 13-5
- **LIU CC ; WALSH CM ; YOUNG JD.** Perforin: structure and function. *Immunol Today,* April 1995, vol. 16 (4), 194-201
- **WAN L.** Expression, purification, and refolding of a novel immunotoxin containing humanized single-chain fragment variable antibody against CTLA4 and the N-terminal fragment of human perforin. *Protein Expr. Purif.,* August 2006, vol. 48 (2), 307-13
- **OHBA M.** Parasporin, a new anticancer protein group from Bacillus thuringiensis. *Anticancer Res.,* January 2009, vol. 29 (1), 427-33
- *J. Biol. Chem.,* 08 September 2006, vol. 281 (36), 26350-60
- *J. Mol. Biol.,* 13 February 2009, vol. 386 (1), 121-33
- **OSTMAN A. et al.** PDGF Receptors as Targets in Tumor Treatment. *Adv. Cancer Res.,* 2007, vol. 97, 247-74
- **COLE AM et al.** Isolation and characterization of pleurocidin, an antimicrobial peptide in the skin secretions of winter flounder. *J Biol Chem.,* 02 May 1997, vol. 272 (18), 12008-13
- **HILCHIE AL et al.** Pleurocidin-family cationic antimicrobial peptides are cytolytic for breast carcinoma cells and prevent growth of tumor xenografts. *Breast Cancer Res.,* 24 October 2011, vol. 13 (5), R102
- **M. GORDON et al.** *Inf. and Immun,* 1995, vol. 63 (1), 82-87
- **BODANSZKY ; BODANSZKY.** The Practice of Peptide Synthesis. Springer- Verlag, 1984
- **STEWART et al.** Solid Phase Peptide Synthesis. Pierce Chemical Company, 1984
- Gene Expression Technology. **GOEDDEL.** Methods in Enzymology. Academic Press, 1990, vol. 185
- **A. STARON et al.** *Advances Mikrobiol.,* 2008, vol. 47 (2), 1983-1995
- **MANIATIS et al.** Molecular Cloning. Cold Spring Harbor, 1982
- Remington’s Pharmaceutical Sciences. Mack Publishing Company, 2000
- **CELIS, JE.** Cell Biology, a Laboratory Handbook. Academic Press, 1998
- **YANG, Y. ; KOH, LW ; TSAI, JH.** Involvement of viral and chemical factors with oral cancer in Taiwan. *Jpn J Clin Oncol,* 2004, vol. 34 (4), 176-183
- **PAPO N ; SHAHAR M ; EISENBACH L ; SHAI Y.** A novel lytic peptide composed of DL-amino acids selectively kills cancer cells in culture and in mice. *J. Biol. Chem.,* 06 June 2003, vol. 278 (3), 21018-23
- **LIU S ; YANG H ; WAN L ; CAI HW ; LI SF ; LI YP ; CHENG JQ ; LU XF.** Enhancement of cytotoxicity of antimicrobial peptide magainin II in tumor cells by bombesin-targeted delivery. *Acta Pharmacol. Sin. <304>,* 01 January 2011, vol. 32 (1), 79-88
- **AVADPOUR MM ; JUBAN MM ; LO WC ; BISHOP SM ; ALBERTY JB ; COWELL SM ; BECKER CL ; MCLAUGHLIN ML.** De novo antimicrobial Peptides with low mammalian cell toxicity <303>. *J. Med. Chem.,* 02 August 1996, vol. 39 (16), 3107-13
- **M., VELASCO ; M., J., DIAZ-GUERRA ; P., DIAZ-ACHIRICA ; D., ANDREU ; L., RIVAS ; L., BOSCA.** Macrophage triggering with cecropin A and melittin-derived peptides induces type II nitric oxide synthase expression. *The Journal of Immunology,* 13 September 1997, vol. 158 (9), 4437-4443
- **ISOGAI E. <302>.** Antimicrobial and Lipopolysaccharide-Binding Activities of C-Terminal Domain of Human CAP18 Peptides to Genus Leptospira <303>. *The Journal of Applied Research,* 01 December 2004, vol. 4 (1), 180-185
- **RISSO A ; BRAIDOT E ; SORDANO MC ; VIANELLO A ; MACR? F ; SKERLAVAJ B ; ZANETTI M ; GENNARO R ; BERNARDI P.** MAP-28, an antibiotic oeptide of innate immunity, induces cell death through opening of the mitochondrial permeability transition pore. *Mol. Cell. Biol.,* March 2002, vol. 22 (6), 1926-35
- **ANDRÄ J ; BERNINGHAUSEN 0 ; WÜLFKEN J ; LEIPPE M.** Shortened amoebapore analogs with enhanced antibacterial and cytolytic activity. *FEBS Lett.,* 29 April 1996, vol. 385 (12), 96-100
- **ANDRÄ J ; BERNINGHAUSEN 0 ; WÜLFKEN J ; LEIPPE M.** Shortened amoebapore analogs with enhanced antibacterial and cytolytic activity. *FEBS Lett.,* 29 April 1996, vol. 385 (1), 96-100

- **INES NEUNDORF ; ROBERT RENNERT ; JAN HOYER ; FRANZISKA SCHRAMM ; KRISTIN LÖBNER IGOR KITANOVIC ; STEFAN WÖLFL.** Fusion of a Short HA2-Derived Peptide Sequence to Cell-Penetrating Peptides Improves Cytosolic Uptake, but Enhances Cytotoxic Activity. *Pharmaceuticals,* 2009, vol. 2 (2), 49-65
- **HILCHIE AL ; DOUCETTE CD ; PINTO DM ; PATRZYKAT A ; DOUGLAS S ; HOSKIN DW.** Pleurocidin-family cationic antimicrobial peptides are cytolytic for breast carcinoma cells and prevent growth of tumor xenografts. *Breast Cancer Res.,* 24 October 2011, vol. 13 (5), R102
- **PAPO N ; SHAI Y.** New lytic peptides based on the D,L-amphipathic helix motif preferentially kill tumor cells compared to normal cells. *Biochemistry,* 12 August 2003, vol. 42 (31), 9346-54

SEQUENCE LISTING

<110> ADAMED
PIECZYKOLAN Jerzy Szczepan
PAWLAK Sebastian Dominik
ZEREK Bartłomiej Maciej
RÓZGA Piotr Kamil

<120> ANTICANCER FUSION PROTEIN

<130> P070 02 PL

<160> 132

<170> PatentIn version 3.5

<210> 4
<211> 227
<212> PRT
<213> Artificial Sequence

<220>
<223> Fusion Peptide

<400> 4

```
Gly Leu Gly Ser Val Phe Gly Arg Leu Ala Arg Ile Leu Gly Arg Val
1               5                   10                  15

Ile Pro Lys Val Ala Lys Lys Leu Gly Pro Lys Val Ala Lys Val Leu
            20                  25                  30

Pro Lys Val Met Lys Glu Ala Ile Pro Met Ala Val Glu Met Ala Lys
        35                  40                  45

Ser Gln Glu Glu Gln Gln Pro Gln Arg Val Val Arg Pro Leu Gly Leu
    50                  55                  60

Ala Gly Arg Val Ala Ala His Ile Thr Gly Thr Arg Gly Arg Ser Asn
65                  70                  75                  80

Thr Leu Ser Ser Pro Asn Ser Lys Asn Glu Lys Ala Leu Gly Arg Lys
                85                  90                  95

Ile Asn Ser Trp Glu Ser Ser Arg Ser Gly His Ser Phe Leu Ser Asn
            100                 105                 110
```

```
Leu His Leu Arg Asn Gly Glu Leu Val Ile His Glu Lys Gly Phe Tyr
        115                 120                 125

Tyr Ile Tyr Ser Gln Thr Tyr Phe Arg Phe Gln Glu Glu Ile Lys Glu
    130                 135                 140

Asn Thr Lys Asn Asp Lys Gln Met Val Gln Tyr Ile Tyr Lys Tyr Thr
145                 150                 155                 160

Ser Tyr Pro Asp Pro Ile Leu Leu Met Lys Ser Ala Arg Asn Ser Cys
                165                 170                 175

Trp Ser Lys Asp Ala Glu Tyr Gly Leu Tyr Ser Ile Tyr Gln Gly Gly
            180                 185                 190

Ile Phe Glu Leu Lys Glu Asn Asp Arg Ile Phe Val Ser Val Thr Asn
        195                 200                 205

Glu His Leu Ile Asp Met Asp His Glu Ala Ser Phe Phe Gly Ala Phe
    210                 215                 220

Leu Val Gly
225
```

<210> 5
<211> 264
<212> PRT
<213> Artificial Sequence

<220>
<223> Fusion Peptide

<400> 5

```
Thr Ser Glu Glu Thr Ile Ser Thr Val Gln Glu Lys Gln Gln Asn Ile
1               5                   10                  15

Ser Pro Leu Val Arg Glu Arg Gly Pro Gln Arg Val Ala Ala His Ile
            20                  25                  30

Thr Gly Thr Arg Gly Arg Ser Asn Thr Leu Ser Ser Pro Asn Ser Lys
        35                  40                  45
```

```
Asn Glu Lys Ala Leu Gly Arg Lys Ile Asn Ser Trp Glu Ser Ser Arg
    50                  55                  60

Ser Gly His Ser Phe Leu Ser Asn Leu His Leu Arg Asn Gly Glu Leu
65                  70                  75                  80

Val Ile His Glu Lys Gly Phe Tyr Tyr Ile Tyr Ser Gln Thr Tyr Phe
                85                  90                  95

Arg Phe Gln Glu Glu Ile Lys Glu Asn Thr Lys Asn Asp Lys Gln Met
            100                 105                 110

Val Gln Tyr Ile Tyr Lys Tyr Thr Ser Tyr Pro Asp Pro Ile Leu Leu
        115                 120                 125

Met Lys Ser Ala Arg Asn Ser Cys Trp Ser Lys Asp Ala Glu Tyr Gly
    130                 135                 140

Leu Tyr Ser Ile Tyr Gln Gly Gly Ile Phe Glu Leu Lys Glu Asn Asp
145                 150                 155                 160

Arg Ile Phe Val Ser Val Thr Asn Glu His Leu Ile Asp Met Asp His
                165                 170                 175

Glu Ala Ser Phe Phe Gly Ala Phe Leu Val Gly Cys Ala Ala Cys Ala
            180                 185                 190

Ala Ala Cys Gly Gly Gly Pro Leu Gly Leu Ala Gly Arg Val Val Arg
        195                 200                 205

Gly Leu Gly Ser Val Phe Gly Arg Leu Ala Arg Ile Leu Gly Arg Val
    210                 215                 220

Ile Pro Lys Val Ala Lys Lys Leu Gly Pro Lys Val Ala Lys Val Leu
225                 230                 235                 240

Pro Lys Val Met Lys Glu Ala Ile Pro Met Ala Val Glu Met Ala Lys
                245                 250                 255

Ser Gln Glu Glu Gln Gln Pro Gln
```

260

<210> 6
<211> 299
<212> PRT
<213> Artificial Sequence

<220>
<223> Fusion Peptide

<400> 6

```
Thr Ser Glu Glu Thr Ile Ser Thr Val Gln Glu Lys Gln Gln Asn Ile
1               5                   10                  15

Ser Pro Leu Val Arg Glu Arg Gly Pro Gln Arg Val Ala Ala His Ile
            20                  25                  30

Thr Gly Thr Arg Gly Arg Ser Asn Thr Leu Ser Ser Pro Asn Ser Lys
        35                  40                  45

Asn Glu Lys Ala Leu Gly Arg Lys Ile Asn Ser Trp Glu Ser Ser Arg
    50                  55                  60

Ser Gly His Ser Phe Leu Ser Asn Leu His Leu Arg Asn Gly Glu Leu
65                  70                  75                  80

Val Ile His Glu Lys Gly Phe Tyr Tyr Ile Tyr Ser Gln Thr Tyr Phe
                85                  90                  95

Arg Phe Gln Glu Glu Ile Lys Glu Asn Thr Lys Asn Asp Lys Gln Met
            100                 105                 110

Val Gln Tyr Ile Tyr Lys Tyr Thr Ser Tyr Pro Asp Pro Ile Leu Leu
        115                 120                 125

Met Lys Ser Ala Arg Asn Ser Cys Trp Ser Lys Asp Ala Glu Tyr Gly
    130                 135                 140

Leu Tyr Ser Ile Tyr Gln Gly Gly Ile Phe Glu Leu Lys Glu Asn Asp
145                 150                 155                 160

Arg Ile Phe Val Ser Val Thr Asn Glu His Leu Ile Asp Met Asp His
```

165 170 175

Glu Ala Ser Phe Phe Gly Ala Phe Leu Val Gly Gly Ser Gly Cys Ala
180 185 190

Ala Cys Ala Ala Ala Cys Pro Leu Gly Leu Ala Gly Arg Val Val Arg
195 200 205

Gly Lys Leu Ser Cys Leu Ser Leu Ala Leu Ala Ile Ile Leu Ile Leu
210 215 220

Ala Ile Val His Ser Pro Asn Met Glu Val Lys Ala Leu Ala Asp Pro
225 230 235 240

Glu Ala Asp Ala Phe Gly Glu Ala Asn Ala Phe Gly Glu Ala Asp Ala
245 250 255

Phe Ala Glu Ala Asn Ala Asp Val Lys Gly Met Lys Lys Ala Ile Lys
260 265 270

Glu Ile Leu Asp Cys Val Ile Glu Lys Gly Tyr Asp Lys Leu Ala Ala
275 280 285

Lys Leu Lys Lys Val Ile Gln Gln Leu Trp Glu
290 295

<210> 11
<211> 202
<212> PRT
<213> Artificial Sequence

<220>
<223> Fusion Peptide

<400> 11

Arg Val Ala Ala His Ile Thr Gly Thr Arg Gly Arg Ser Asn Thr Leu
1 5 10 15

Ser Ser Pro Asn Ser Lys Asn Glu Lys Ala Leu Gly Arg Lys Ile Asn
20 25 30

Ser Trp Glu Ser Ser Arg Ser Gly His Ser Phe Leu Ser Asn Leu His

```
            35                  40                  45

Leu Arg Asn Gly Glu Leu Val Ile His Glu Lys Gly Phe Tyr Tyr Ile
    50                  55                  60

Tyr Ser Gln Thr Tyr Phe Arg Phe Gln Glu Glu Ile Lys Glu Asn Thr
65                  70                  75                  80

Lys Asn Asp Lys Gln Met Val Gln Tyr Ile Tyr Lys Tyr Thr Ser Tyr
                85                  90                  95

Pro Asp Pro Ile Leu Leu Met Lys Ser Ala Arg Asn Ser Cys Trp Ser
            100                 105                 110

Lys Asp Ala Glu Tyr Gly Leu Tyr Ser Ile Tyr Gln Gly Gly Ile Phe
        115                 120                 125

Glu Leu Lys Glu Asn Asp Arg Ile Phe Val Ser Val Thr Asn Glu His
    130                 135                 140

Leu Ile Asp Met Asp His Glu Ala Ser Phe Phe Gly Ala Phe Leu Val
145                 150                 155                 160

Gly Gly Gly Gly Gly Ser Gly Gly Gly Gly Arg Val Val Arg Pro Leu
                165                 170                 175

Gly Leu Ala Gly Arg Arg Arg Arg Arg Arg Arg Arg Lys Leu Ala Lys
            180                 185                 190

Leu Ala Lys Lys Leu Ala Lys Leu Ala Lys
        195                 200
```

<210> 12
<211> 205
<212> PRT
<213> Artificial Sequence

<220>
<223> Fusion Peptide

<400> 12

```
Arg Val Ala Ala His Ile Thr Gly Thr Arg Gly Arg Ser Asn Thr Leu
```

```
1               5                   10                  15

Ser Ser Pro Asn Ser Lys Asn Glu Lys Ala Leu Gly Arg Lys Ile Asn
            20                  25                  30

Ser Trp Glu Ser Ser Arg Ser Gly His Ser Phe Leu Ser Asn Leu His
        35                  40                  45

Leu Arg Asn Gly Glu Leu Val Ile His Glu Lys Gly Phe Tyr Tyr Ile
    50                  55                  60

Tyr Ser Gln Thr Tyr Phe Arg Phe Gln Glu Glu Ile Lys Glu Asn Thr
65                  70                  75                  80

Lys Asn Asp Lys Gln Met Val Gln Tyr Ile Tyr Lys Tyr Thr Ser Tyr
                85                  90                  95

Pro Asp Pro Ile Leu Leu Met Lys Ser Ala Arg Asn Ser Cys Trp Ser
            100                 105                 110

Lys Asp Ala Glu Tyr Gly Leu Tyr Ser Ile Tyr Gln Gly Gly Ile Phe
        115                 120                 125

Glu Leu Lys Glu Asn Asp Arg Ile Phe Val Ser Val Thr Asn Glu His
    130                 135                 140

Leu Ile Asp Met Asp His Glu Ala Ser Phe Phe Gly Ala Phe Leu Val
145                 150                 155                 160

Gly Gly Gly Ala Ser Gly Cys Gly Pro Glu Gly Gly Gly Gly Pro Leu
                165                 170                 175

Gly Leu Ala Gly Arg Val Val Arg Arg Arg Arg Arg Arg Arg Arg Lys
            180                 185                 190

Leu Ala Lys Leu Ala Lys Lys Leu Ala Lys Leu Ala Lys
        195                 200                 205
```

<210> 13
<211> 228
<212> PRT
<213> Artificial Sequence

<220>
<223> fusion protein

<400> 13

```
Thr Ser Glu Glu Thr Ile Ser Thr Val Gln Glu Lys Gln Gln Asn Ile
1               5                   10                  15

Ser Pro Leu Val Arg Glu Arg Gly Pro Gln Arg Val Ala Ala His Ile
            20                  25                  30

Thr Gly Thr Arg Gly Arg Ser Asn Thr Leu Ser Ser Pro Asn Ser Lys
        35                  40                  45

Asn Glu Lys Ala Leu Gly Arg Lys Ile Asn Ser Trp Glu Ser Ser Arg
    50                  55                  60

Ser Gly His Ser Phe Leu Ser Asn Leu His Leu Arg Asn Gly Glu Leu
65                  70                  75                  80

Val Ile His Glu Lys Gly Phe Tyr Tyr Ile Tyr Ser Gln Thr Tyr Phe
                85                  90                  95

Arg Phe Gln Glu Glu Ile Lys Glu Asn Thr Lys Asn Asp Lys Gln Met
            100                 105                 110

Val Gln Tyr Ile Tyr Lys Tyr Thr Ser Tyr Pro Asp Pro Ile Leu Leu
        115                 120                 125

Met Lys Ser Ala Arg Asn Ser Cys Trp Ser Lys Asp Ala Glu Tyr Gly
    130                 135                 140

Leu Tyr Ser Ile Tyr Gln Gly Gly Ile Phe Glu Leu Lys Glu Asn Asp
145                 150                 155                 160

Arg Ile Phe Val Ser Val Thr Asn Glu His Leu Ile Asp Met Asp His
                165                 170                 175

Glu Ala Ser Phe Phe Gly Ala Phe Leu Val Gly Gly Gly Gly Gly Ser
            180                 185                 190
```

Gly Gly Gly Gly Arg Val Val Arg Pro Leu Gly Leu Ala Gly Arg Arg
195 200 205

Arg Arg Arg Arg Arg Arg Lys Leu Ala Lys Leu Ala Lys Lys Leu Ala
210 215 220

Lys Leu Ala Lys
225

<210> 14
<211> 192
<212> PRT
<213> Artificial Sequence

<220>
<223> Fusion Peptide

<400> 14

His His His His His His Lys Leu Ala Lys Leu Ala Lys Lys Leu Ala
1 5 10 15

Lys Leu Ala Lys Cys Arg Val Val Arg Pro Leu Gly Leu Ala Gly Arg
20 25 30

Val Ala Ala His Ile Thr Gly Thr Arg Gly Arg Ser Asn Thr Leu Ser
35 40 45

Ser Pro Asn Ser Lys Asn Glu Lys Ala Leu Gly Arg Lys Ile Asn Ser
50 55 60

Trp Glu Ser Ser Arg Ser Gly His Ser Phe Leu Ser Asn Leu His Leu
65 70 75 80

Arg Asn Gly Glu Leu Val Ile His Glu Lys Gly Phe Tyr Tyr Ile Tyr
85 90 95

Ser Gln Thr Tyr Phe Arg Phe Gln Glu Glu Ile Lys Glu Asn Thr Lys
100 105 110

Asn Asp Lys Gln Met Val Gln Tyr Ile Tyr Lys Tyr Thr Ser Tyr Pro
115 120 125

Asp Pro Ile Leu Leu Met Lys Ser Ala Arg Asn Ser Cys Trp Ser Lys
130 135 140

Asp Ala Glu Tyr Gly Leu Tyr Ser Ile Tyr Gln Gly Gly Ile Phe Glu
145 150 155 160

Leu Lys Glu Asn Asp Arg Ile Phe Val Ser Val Thr Asn Glu His Leu
165 170 175

Ile Asp Met Asp His Glu Ala Ser Phe Phe Gly Ala Phe Leu Val Gly
180 185 190

<210> 15
<211> 200
<212> PRT
<213> Artificial Sequence

<220>
<223> Fusion Peptide

<400> 15

His His His His His His Lys Leu Ala Lys Leu Ala Lys Lys Leu Ala
1 5 10 15

Lys Leu Ala Lys Cys Arg Arg Arg Arg Arg Arg Arg Arg Arg Val Val
20 25 30

Arg Pro Leu Gly Leu Ala Gly Arg Val Ala Ala His Ile Thr Gly Thr
35 40 45

Arg Gly Arg Ser Asn Thr Leu Ser Ser Pro Asn Ser Lys Asn Glu Lys
50 55 60

Ala Leu Gly Arg Lys Ile Asn Ser Trp Glu Ser Ser Arg Ser Gly His
65 70 75 80

Ser Phe Leu Ser Asn Leu His Leu Arg Asn Gly Glu Leu Val Ile His
85 90 95

Glu Lys Gly Phe Tyr Tyr Ile Tyr Ser Gln Thr Tyr Phe Arg Phe Gln
100 105 110

```
Glu Glu Ile Lys Glu Asn Thr Lys Asn Asp Lys Gln Met Val Gln Tyr
        115                 120                 125

Ile Tyr Lys Tyr Thr Ser Tyr Pro Asp Pro Ile Leu Leu Met Lys Ser
    130                 135                 140

Ala Arg Asn Ser Cys Trp Ser Lys Asp Ala Glu Tyr Gly Leu Tyr Ser
145                 150                 155                 160

Ile Tyr Gln Gly Gly Ile Phe Glu Leu Lys Glu Asn Asp Arg Ile Phe
                165                 170                 175

Val Ser Val Thr Asn Glu His Leu Ile Asp Met Asp His Glu Ala Ser
            180                 185                 190

Phe Phe Gly Ala Phe Leu Val Gly
        195                 200
```

<210> 16
<211> 202
<212> PRT
<213> Artificial Sequence

<220>
<223> Fusion Peptide

<400> 16

```
Arg Val Ala Ala His Ile Thr Gly Thr Arg Gly Arg Ser Asn Thr Leu
1               5                   10                  15

Ser Ser Pro Asn Ser Lys Asn Glu Lys Ala Leu Gly Arg Lys Ile Asn
            20                  25                  30

Ser Trp Glu Ser Ser Arg Ser Gly His Ser Phe Leu Ser Asn Leu His
        35                  40                  45

Leu Arg Asn Gly Glu Leu Val Ile His Glu Lys Gly Phe Tyr Tyr Ile
    50                  55                  60

Tyr Ser Gln Thr Tyr Phe Arg Phe Gln Glu Glu Ile Lys Glu Asn Thr
65                  70                  75                  80
```

```
Lys Asn Asp Lys Gln Met Val Gln Tyr Ile Tyr Lys Tyr Thr Ser Tyr
                85                  90                  95

Pro Asp Pro Ile Leu Leu Met Lys Ser Ala Arg Asn Ser Cys Trp Ser
            100                 105                 110

Lys Asp Ala Glu Tyr Gly Leu Tyr Ser Ile Tyr Gln Gly Gly Ile Phe
        115                 120                 125

Glu Leu Lys Glu Asn Asp Arg Ile Phe Val Ser Val Thr Asn Glu His
    130                 135                 140

Leu Ile Asp Met Asp His Glu Ala Ser Phe Phe Gly Ala Phe Leu Val
145                 150                 155                 160

Gly Gly Gly Gly Gly Ser Gly Gly Gly Gly Pro Leu Gly Leu Ala Gly
                165                 170                 175

Arg Val Val Arg Arg Arg Arg Arg Arg Arg Arg Arg Lys Leu Ala Lys
            180                 185                 190

Leu Ala Lys Lys Leu Ala Lys Leu Ala Lys
        195                 200
```

<210> 18
<211> 203
<212> PRT
<213> Artificial Sequence

<220>
<223> Fusion Peptide

<400> 18

```
Phe Arg Lys Ser Lys Glu Lys Ile Gly Lys Phe Phe Lys Arg Ile Val
1               5                   10                  15

Gln Arg Ile Phe Asp Phe Leu Arg Asn Leu Val Arg Val Val Arg Pro
            20                  25                  30

Leu Gly Leu Ala Gly Glu Arg Gly Pro Gln Arg Val Ala Ala His Ile
        35                  40                  45
```

Thr Gly Thr Arg Gly Arg Ser Asn Thr Leu Ser Ser Pro Asn Ser Lys
50 55 60

Asn Glu Lys Ala Leu Gly Arg Lys Ile Asn Ser Trp Glu Ser Ser Arg
65 70 75 80

Ser Gly His Ser Phe Leu Ser Asn Leu His Leu Arg Asn Gly Glu Leu
85 90 95

Val Ile His Glu Lys Gly Phe Tyr Tyr Ile Tyr Ser Gln Thr Tyr Phe
100 105 110

Arg Phe Gln Glu Glu Ile Lys Glu Asn Thr Lys Asn Asp Lys Gln Met
115 120 125

Val Gln Tyr Ile Tyr Lys Tyr Thr Ser Tyr Pro Asp Pro Ile Leu Leu
130 135 140

Met Lys Ser Ala Arg Asn Ser Cys Trp Ser Lys Asp Ala Glu Tyr Gly
145 150 155 160

Leu Tyr Ser Ile Tyr Gln Gly Gly Ile Phe Glu Leu Lys Glu Asn Asp
165 170 175

Arg Ile Phe Val Ser Val Thr Asn Glu His Leu Ile Asp Met Asp His
180 185 190

Glu Ala Ser Phe Phe Gly Ala Phe Leu Val Gly
195 200

<210> 19
<211> 203
<212> PRT
<213> Artificial Sequence

<220>
<223> Fusion Peptide

<400> 19

Gly Gly Leu Arg Ser Leu Gly Arg Lys Ile Leu Arg Ala Trp Lys Lys
1 5 10 15

Tyr Gly Pro Ile Ile Val Pro Ile Ile Arg Ile Arg Val Val Arg Pro
20 25 30

Leu Gly Leu Ala Gly Glu Arg Gly Pro Gln Arg Val Ala Ala His Ile
35 40 45

Thr Gly Thr Arg Gly Arg Ser Asn Thr Leu Ser Ser Pro Asn Ser Lys
50 55 60

Asn Glu Lys Ala Leu Gly Arg Lys Ile Asn Ser Trp Glu Ser Ser Arg
65 70 75 80

Ser Gly His Ser Phe Leu Ser Asn Leu His Leu Arg Asn Gly Glu Leu
85 90 95

Val Ile His Glu Lys Gly Phe Tyr Tyr Ile Tyr Ser Gln Thr Tyr Phe
100 105 110

Arg Phe Gln Glu Glu Ile Lys Glu Asn Thr Lys Asn Asp Lys Gln Met
115 120 125

Val Gln Tyr Ile Tyr Lys Tyr Thr Ser Tyr Pro Asp Pro Ile Leu Leu
130 135 140

Met Lys Ser Ala Arg Asn Ser Cys Trp Ser Lys Asp Ala Glu Tyr Gly
145 150 155 160

Leu Tyr Ser Ile Tyr Gln Gly Gly Ile Phe Glu Leu Lys Glu Asn Asp
165 170 175

Arg Ile Phe Val Ser Val Thr Asn Glu His Leu Ile Asp Met Asp His
180 185 190

Glu Ala Ser Phe Phe Gly Ala Phe Leu Val Gly
195 200

<210> 20
<211> 200
<212> PRT
<213> Artificial Sequence

<220>
<223> Fusion Peptide

<400> 20

```
Arg Val Ala Ala His Ile Thr Gly Thr Arg Gly Arg Ser Asn Thr Leu
1               5                   10                  15

Ser Ser Pro Asn Ser Lys Asn Glu Lys Ala Leu Gly Arg Lys Ile Asn
            20                  25                  30

Ser Trp Glu Ser Ser Arg Ser Gly His Ser Phe Leu Ser Asn Leu His
        35                  40                  45

Leu Arg Asn Gly Glu Leu Val Ile His Glu Lys Gly Phe Tyr Tyr Ile
    50                  55                  60

Tyr Ser Gln Thr Tyr Phe Arg Phe Gln Glu Glu Ile Lys Glu Asn Thr
65                  70                  75                  80

Lys Asn Asp Lys Gln Met Val Gln Tyr Ile Tyr Lys Tyr Thr Ser Tyr
                85                  90                  95

Pro Asp Pro Ile Leu Leu Met Lys Ser Ala Arg Asn Ser Cys Trp Ser
            100                 105                 110

Lys Asp Ala Glu Tyr Gly Leu Tyr Ser Ile Tyr Gln Gly Gly Ile Phe
        115                 120                 125

Glu Leu Lys Glu Asn Asp Arg Ile Phe Val Ser Val Thr Asn Glu His
    130                 135                 140

Leu Ile Asp Met Asp His Glu Ala Ser Phe Phe Gly Ala Phe Leu Val
145                 150                 155                 160

Gly Gly Gly Ser Gly Gly Pro Leu Gly Leu Ala Gly Arg Val Val Arg
                165                 170                 175

Gly Leu Val Glu Thr Leu Thr Lys Ile Val Ser Tyr Gly Ile Asp Lys
            180                 185                 190

Leu Ile Glu Lys Ile Leu Glu Gly
                    195                 200
```

<210> 21
<211> 200
<212> PRT
<213> Artificial Sequence

<220>
<223> Fusion Peptide

<400> 21

```
Arg Val Ala Ala His Ile Thr Gly Thr Arg Gly Arg Ser Asn Thr Leu
1               5                   10                  15

Ser Ser Pro Asn Ser Lys Asn Glu Lys Ala Leu Gly Arg Lys Ile Asn
            20                  25                  30

Ser Trp Glu Ser Ser Arg Ser Gly His Ser Phe Leu Ser Asn Leu His
        35                  40                  45

Leu Arg Asn Gly Glu Leu Val Ile His Glu Lys Gly Phe Tyr Tyr Ile
    50                  55                  60

Tyr Ser Gln Thr Tyr Phe Arg Phe Gln Glu Glu Ile Lys Glu Asn Thr
65                  70                  75                  80

Lys Asn Asp Lys Gln Met Val Gln Tyr Ile Tyr Lys Tyr Thr Ser Tyr
                85                  90                  95

Pro Asp Pro Ile Leu Leu Met Lys Ser Ala Arg Asn Ser Cys Trp Ser
            100                 105                 110

Lys Asp Ala Glu Tyr Gly Leu Tyr Ser Ile Tyr Gln Gly Gly Ile Phe
        115                 120                 125

Glu Leu Lys Glu Asn Asp Arg Ile Phe Val Ser Val Thr Asn Glu His
    130                 135                 140

Leu Ile Asp Met Asp His Glu Ala Ser Phe Phe Gly Ala Phe Leu Val
145                 150                 155                 160

Gly Gly Gly Ser Gly Gly Pro Leu Gly Leu Ala Gly Arg Val Val Arg
```

165 170 175

Gly Phe Ile Ala Thr Leu Thr Lys Val Leu Asp Phe Gly Ile Asp Lys
180 185 190

Leu Ile Gln Leu Ile Glu Asp Lys
195 200

<210> 22
<211> 200
<212> PRT
<213> Artificial Sequence

<220>
<223> Fusion Peptide

<400> 22

Arg Val Ala Ala His Ile Thr Gly Thr Arg Gly Arg Ser Asn Thr Leu
1 5 10 15

Ser Ser Pro Asn Ser Lys Asn Glu Lys Ala Leu Gly Arg Lys Ile Asn
20 25 30

Ser Trp Glu Ser Ser Arg Ser Gly His Ser Phe Leu Ser Asn Leu His
35 40 45

Leu Arg Asn Gly Glu Leu Val Ile His Glu Lys Gly Phe Tyr Tyr Ile
50 55 60

Tyr Ser Gln Thr Tyr Phe Arg Phe Gln Glu Glu Ile Lys Glu Asn Thr
65 70 75 80

Lys Asn Asp Lys Gln Met Val Gln Tyr Ile Tyr Lys Tyr Thr Ser Tyr
85 90 95

Pro Asp Pro Ile Leu Leu Met Lys Ser Ala Arg Asn Ser Cys Trp Ser
100 105 110

Lys Asp Ala Glu Tyr Gly Leu Tyr Ser Ile Tyr Gln Gly Gly Ile Phe
115 120 125

Glu Leu Lys Glu Asn Asp Arg Ile Phe Val Ser Val Thr Asn Glu His

130 135 140

Leu Ile Asp Met Asp His Glu Ala Ser Phe Phe Gly Ala Phe Leu Val
145 150 155 160

Gly Gly Gly Ser Gly Gly Pro Leu Gly Leu Ala Gly Arg Val Val Arg
165 170 175

Gly Phe Leu Gly Thr Leu Glu Lys Ile Leu Ser Phe Gly Val Asp Glu
180 185 190

Leu Val Lys Leu Ile Glu Asn His
195 200

<210> 23
<211> 190
<212> PRT
<213> Artificial Sequence

<220>
<223> Fusion Peptide

<400> 23

Gly Leu Leu Glu Ala Leu Ala Glu Leu Leu Glu Gly Arg Arg Arg Arg
1 5 10 15

Arg Arg Arg Arg Val Val Arg Pro Leu Gly Leu Ala Gly Arg Val Ala
20 25 30

Ala His Ile Thr Gly Thr Arg Gly Arg Ser Asn Thr Leu Ser Ser Pro
35 40 45

Asn Ser Lys Asn Glu Lys Ala Leu Gly Arg Lys Ile Asn Ser Trp Glu
50 55 60

Ser Ser Arg Ser Gly His Ser Phe Leu Ser Asn Leu His Leu Arg Asn
65 70 75 80

Gly Glu Leu Val Ile His Glu Lys Gly Phe Tyr Tyr Ile Tyr Ser Gln
85 90 95

Thr Tyr Phe Arg Phe Gln Glu Glu Ile Lys Glu Asn Thr Lys Asn Asp

100 105 110

Lys Gln Met Val Gln Tyr Ile Tyr Lys Tyr Thr Ser Tyr Pro Asp Pro
115 120 125

Ile Leu Leu Met Lys Ser Ala Arg Asn Ser Cys Trp Ser Lys Asp Ala
130 135 140

Glu Tyr Gly Leu Tyr Ser Ile Tyr Gln Gly Gly Ile Phe Glu Leu Lys
145 150 155 160

Glu Asn Asp Arg Ile Phe Val Ser Val Thr Asn Glu His Leu Ile Asp
165 170 175

Met Asp His Glu Ala Ser Phe Phe Gly Ala Phe Leu Val Gly
180 185 190

<210> 29
<211> 224
<212> PRT
<213> Artificial Sequence

<220>
<223> Fusion Peptide

<400> 29

Arg Val Ala Ala His Ile Thr Gly Thr Arg Gly Arg Ser Asn Thr Leu
1 5 10 15

Ser Ser Pro Asn Ser Lys Asn Glu Lys Ala Leu Gly Arg Lys Ile Asn
20 25 30

Ser Trp Glu Ser Ser Arg Ser Gly His Ser Phe Leu Ser Asn Leu His
35 40 45

Leu Arg Asn Gly Glu Leu Val Ile His Glu Lys Gly Phe Tyr Tyr Ile
50 55 60

Tyr Ser Gln Thr Tyr Phe Arg Phe Gln Glu Glu Ile Lys Glu Asn Thr
65 70 75 80

Lys Asn Asp Lys Gln Met Val Gln Tyr Ile Tyr Lys Tyr Thr Ser Tyr

```
                85                  90                  95

Pro Asp Pro Ile Leu Leu Met Lys Ser Ala Arg Asn Ser Cys Trp Ser
            100                 105                 110

Lys Asp Ala Glu Tyr Gly Leu Tyr Ser Ile Tyr Gln Gly Gly Ile Phe
        115                 120                 125

Glu Leu Lys Glu Asn Asp Arg Ile Phe Val Ser Val Thr Asn Glu His
    130                 135                 140

Leu Ile Asp Met Asp His Glu Ala Ser Phe Phe Gly Ala Phe Leu Val
145                 150                 155                 160

Gly Gly Gly Gly Ser Gly Gly Gly Pro Leu Gly Leu Ala Gly Arg Val
                165                 170                 175

Val Arg Lys Ser Cys Cys Pro Asn Thr Thr Gly Arg Asn Ile Tyr Asn
            180                 185                 190

Ala Cys Arg Leu Thr Gly Ala Pro Arg Pro Thr Cys Ala Lys Leu Ser
        195                 200                 205

Gly Cys Lys Ile Ile Ser Gly Ser Thr Cys Pro Ser Asp Tyr Pro Lys
    210                 215                 220
```

<210> 30
<211> 200
<212> PRT
<213> Artificial Sequence

<220>
<223> Fusion Peptide

<400> 30

```
Arg Val Ala Ala His Ile Thr Gly Thr Arg Gly Arg Ser Asn Thr Leu
1               5                   10                  15

Ser Ser Pro Asn Ser Lys Asn Glu Lys Ala Leu Gly Arg Lys Ile Asn
            20                  25                  30

Ser Trp Glu Ser Ser Arg Ser Gly His Ser Phe Leu Ser Asn Leu His
```

35 40 45

Leu Arg Asn Gly Glu Leu Val Ile His Glu Lys Gly Phe Tyr Tyr Ile
50 55 60

Tyr Ser Gln Thr Tyr Phe Arg Phe Gln Glu Glu Ile Lys Glu Asn Thr
65 70 75 80

Lys Asn Asp Lys Gln Met Val Gln Tyr Ile Tyr Lys Tyr Thr Ser Tyr
85 90 95

Pro Asp Pro Ile Leu Leu Met Lys Ser Ala Arg Asn Ser Cys Trp Ser
100 105 110

Lys Asp Ala Glu Tyr Gly Leu Tyr Ser Ile Tyr Gln Gly Gly Ile Phe
115 120 125

Glu Leu Lys Glu Asn Asp Arg Ile Phe Val Ser Val Thr Asn Glu His
130 135 140

Leu Ile Asp Met Asp His Glu Ala Ser Phe Phe Gly Ala Phe Leu Val
145 150 155 160

Gly Gly Gly Gly Gly Ser Gly Ala Pro Cys His Thr Ala Ala Arg Ser
165 170 175

Glu Cys Lys Arg Ser His Lys Phe Val Pro Gly Ala Trp Leu Ala Gly
180 185 190

Glu Gly Val Asp Val Thr Ser Leu
195 200

<210> 31
<211> 210
<212> PRT
<213> Artificial Sequence

<220>
<223> Fusion Peptide

<400> 31

Arg Val Ala Ala His Ile Thr Gly Thr Arg Gly Arg Ser Asn Thr Leu

```
1               5                   10                  15

Ser Ser Pro Asn Ser Lys Asn Glu Lys Ala Leu Gly Arg Lys Ile Asn
            20                  25                  30

Ser Trp Glu Ser Ser Arg Ser Gly His Ser Phe Leu Ser Asn Leu His
        35                  40                  45

Leu Arg Asn Gly Glu Leu Val Ile His Glu Lys Gly Phe Tyr Tyr Ile
    50                  55                  60

Tyr Ser Gln Thr Tyr Phe Arg Phe Gln Glu Glu Ile Lys Glu Asn Thr
65                  70                  75                  80

Lys Asn Asp Lys Gln Met Val Gln Tyr Ile Tyr Lys Tyr Thr Ser Tyr
                85                  90                  95

Pro Asp Pro Ile Leu Leu Met Lys Ser Ala Arg Asn Ser Cys Trp Ser
            100                 105                 110

Lys Asp Ala Glu Tyr Gly Leu Tyr Ser Ile Tyr Gln Gly Gly Ile Phe
        115                 120                 125

Glu Leu Lys Glu Asn Asp Arg Ile Phe Val Ser Val Thr Asn Glu His
    130                 135                 140

Leu Ile Asp Met Asp His Glu Ala Ser Phe Phe Gly Ala Phe Leu Val
145                 150                 155                 160

Gly Gly Gly Gly Gly Ser Gly Arg Val Val Arg Pro Leu Gly Leu Ala
                165                 170                 175

Gly Ala Pro Cys His Thr Ala Ala Arg Ser Glu Cys Lys Arg Ser His
            180                 185                 190

Lys Phe Val Pro Gly Ala Trp Leu Ala Gly Glu Gly Val Asp Val Thr
        195                 200                 205

Ser Leu
    210
```

<210> 32
<211> 436
<212> PRT
<213> Artificial Sequence

<220>
<223> Fusion Peptide

<400> 32

```
Asp Val Ile Arg Glu Tyr Leu Met Phe Asn Glu Leu Ser Ala Leu Ser
1               5                   10                  15

Ser Ser Pro Glu Ser Val Arg Ser Arg Phe Ser Ser Ile Tyr Gly Thr
            20                  25                  30

Asn Pro Asp Gly Ile Ala Leu Asn Asn Glu Thr Tyr Phe Asn Ala Val
        35                  40                  45

Lys Pro Pro Ile Thr Ala Gln Tyr Gly Tyr Tyr Cys Tyr Lys Asn Val
    50                  55                  60

Gly Thr Val Gln Tyr Val Asn Arg Pro Thr Asp Ile Asn Pro Asn Val
65                  70                  75                  80

Ile Leu Ala Gln Asp Thr Leu Thr Asn Asn Thr Asn Glu Pro Phe Thr
                85                  90                  95

Thr Thr Ile Thr Ile Thr Gly Ser Phe Thr Asn Thr Ser Thr Val Thr
            100                 105                 110

Ser Ser Thr Thr Thr Gly Phe Lys Phe Thr Ser Lys Leu Ser Ile Lys
        115                 120                 125

Lys Val Phe Glu Ile Gly Gly Glu Val Ser Phe Ser Thr Thr Ile Gly
    130                 135                 140

Thr Ser Glu Thr Thr Thr Glu Thr Ile Thr Val Ser Lys Ser Val Thr
145                 150                 155                 160

Val Thr Val Pro Ala Gln Ser Arg Arg Thr Ile Gln Leu Thr Ala Lys
                165                 170                 175
```

```
Ile Ala Lys Glu Ser Ala Asp Phe Ser Ala Pro Ile Thr Val Asp Gly
            180                 185                 190

Tyr Phe Gly Ala Asn Phe Pro Lys Arg Val Gly Pro Gly Gly His Tyr
        195                 200                 205

Phe Trp Phe Asn Pro Ala Arg Asp Val Leu Asn Thr Thr Ser Gly Thr
    210                 215                 220

Leu Arg Gly Thr Val Thr Asn Val Ser Ser Phe Asp Phe Gln Thr Ile
225                 230                 235                 240

Val Gln Pro Ala Arg Ser Leu Leu Asp Glu Gln Arg Val Val Arg Pro
                245                 250                 255

Leu Gly Leu Ala Gly Gly Ser Gly Gly Gly Ser Gly Gly Gly Glu Arg
            260                 265                 270

Gly Pro Gln Arg Val Ala Ala His Ile Thr Gly Thr Arg Gly Arg Ser
        275                 280                 285

Asn Thr Leu Ser Ser Pro Asn Ser Lys Asn Glu Lys Ala Leu Gly Arg
    290                 295                 300

Lys Ile Asn Ser Trp Glu Ser Ser Arg Ser Gly His Ser Phe Leu Ser
305                 310                 315                 320

Asn Leu His Leu Arg Asn Gly Glu Leu Val Ile His Glu Lys Gly Phe
                325                 330                 335

Tyr Tyr Ile Tyr Ser Gln Thr Tyr Phe Arg Phe Gln Glu Glu Ile Lys
            340                 345                 350

Glu Asn Thr Lys Asn Asp Lys Gln Met Val Gln Tyr Ile Tyr Lys Tyr
        355                 360                 365

Thr Ser Tyr Pro Asp Pro Ile Leu Leu Met Lys Ser Ala Arg Asn Ser
    370                 375                 380

Cys Trp Ser Lys Asp Ala Glu Tyr Gly Leu Tyr Ser Ile Tyr Gln Gly
```

385 390 395 400

Gly Ile Phe Glu Leu Lys Glu Asn Asp Arg Ile Phe Val Ser Val Thr
405 410 415

Asn Glu His Leu Ile Asp Met Asp His Glu Ala Ser Phe Phe Gly Ala
420 425 430

Phe Leu Val Gly
435

<210> 33
<211> 215
<212> PRT
<213> Artificial Sequence

<220>
<223> Fusion Peptide

<400> 33

Arg Val Ala Ala His Ile Thr Gly Thr Arg Gly Arg Ser Asn Thr Leu
1 5 10 15

Ser Ser Pro Asn Ser Lys Asn Glu Lys Ala Leu Gly Arg Lys Ile Asn
20 25 30

Ser Trp Glu Ser Ser Arg Ser Gly His Ser Phe Leu Ser Asn Leu His
35 40 45

Leu Arg Asn Gly Glu Leu Val Ile His Glu Lys Gly Phe Tyr Tyr Ile
50 55 60

Tyr Ser Gln Thr Tyr Phe Arg Phe Gln Glu Glu Ile Lys Glu Asn Thr
65 70 75 80

Lys Asn Asp Lys Gln Met Val Gln Tyr Ile Tyr Lys Tyr Thr Ser Tyr
85 90 95

Pro Asp Pro Ile Leu Leu Met Lys Ser Ala Arg Asn Ser Cys Trp Ser
100 105 110

Lys Asp Ala Glu Tyr Gly Leu Tyr Ser Ile Tyr Gln Gly Gly Ile Phe

115 120 125

Glu Leu Lys Glu Asn Asp Arg Ile Phe Val Ser Val Thr Asn Glu His
130 135 140

Leu Ile Asp Met Asp His Glu Ala Ser Phe Phe Gly Ala Phe Leu Val
145 150 155 160

Gly Gly Cys Ala Ala Cys Ala Ala Ala Cys Gly Gly Gly Pro Leu Gly
165 170 175

Leu Ala Gly Arg Val Val Arg Tyr Lys Trp Tyr Gly Tyr Thr Pro Gln
180 185 190

Asn Val Ile Gly Gly Gly Lys Leu Leu Leu Lys Leu Leu Lys Lys Leu
195 200 205

Leu Lys Leu Leu Lys Lys Lys
210 215

<210> 34
<211> 83
<212> PRT
<213> Artificial Sequence

<220>
<223> effector

<300>
<308> GenBank/AAH23576.1
<309> 2006-07-15
<313> (63)..(145)

<400> 34

Gly Arg Asp Tyr Arg Thr Cys Leu Thr Ile Val Gln Lys Leu Lys Lys
1 5 10 15

Met Val Asp Lys Pro Thr Gln Arg Ser Val Ser Asn Ala Ala Thr Arg
20 25 30

Val Cys Arg Thr Gly Arg Ser Arg Trp Arg Asp Val Cys Arg Asn Phe
35 40 45

```
Met Arg Arg Tyr Gln Ser Arg Val Thr Gln Gly Leu Val Ala Gly Glu
    50                  55                  60

Thr Ala Gln Gln Ile Cys Glu Asp Leu Arg Leu Cys Ile Pro Ser Thr
65                  70                  75                  80

Gly Pro Leu
```

<210> 35
<211> 15
<212> PRT
<213> Artificial Sequence

<220>
<223> effector

<300>
<301> Papo N, Shahar M, Eisenbach L, Shai Y.<302> A novel lytic peptide composed of DL-amino acids selectively kills cancer cells in culture and in mice.<303> J. Biol. Chem.<304> 278<305> 3<306> 21018-23<307> 2003-06-06

<400> 35

```
Lys Leu Leu Leu Arg Leu Leu Lys Lys Leu Leu Arg Leu Leu Lys
1               5                   10                  15
```

<210> 36
<211> 56
<212> PRT
<213> Artificial Sequence

<220>
<223> effector

<300>
<308> Swiss-Prot/Q07932.1
<309> 2011-01-11
<313> (57)..(112)

<400> 36

```
Gly Leu Gly Ser Val Phe Gly Arg Leu Ala Arg Ile Leu Gly Arg Val
1               5                   10                  15
```

```
Ile Pro Lys Val Ala Lys Lys Leu Gly Pro Lys Val Ala Lys Val Leu
            20                  25                  30

Pro Lys Val Met Lys Glu Ala Ile Pro Met Ala Val Glu Met Ala Lys
        35                  40                  45

Ser Gln Glu Glu Gln Gln Pro Gln
    50                  55
```

<210> 37
<211> 90
<212> PRT
<213> Artificial Sequence

<220>
<223> effector

<300>
<308> GenBank/BAF95069.1
<309> 2008-08-04
<313> (2)..(91)

<400> 37

```
Lys Leu Ser Cys Leu Ser Leu Ala Leu Ala Ile Ile Leu Ile Leu Ala
1               5                   10                  15

Ile Val His Ser Pro Asn Met Glu Val Lys Ala Leu Ala Asp Pro Glu
            20                  25                  30

Ala Asp Ala Phe Gly Glu Ala Asn Ala Phe Gly Glu Ala Asp Ala Phe
        35                  40                  45

Ala Glu Ala Asn Ala Asp Val Lys Gly Met Lys Lys Ala Ile Lys Glu
    50                  55                  60

Ile Leu Asp Cys Val Ile Glu Lys Gly Tyr Asp Lys Leu Ala Ala Lys
65                  70                  75                  80

Leu Lys Lys Val Ile Gln Gln Leu Trp Glu
                85                  90
```

<210> 38
<211> 17
<212> PRT
<213> Artificial Sequence

<220>

<223> effector

<300>
<308> GenBank/AAA63538.1
<309> 1995-03-07
<313> (24)..(40)

<400> 38

```
Lys Trp Cys Phe Arg Val Cys Tyr Arg Gly Ile Cys Tyr Arg Arg Cys
1               5                   10                  15

Arg
```

<210> 39
<211> 38
<212> PRT
<213> Artificial Sequence

<220>
<223> effector

<300>
<301> Liu S, Yang H, Wan L, Cai HW, Li SF, Li YP, Cheng JQ, Lu XF.<302> Enhancement of cytotoxicity of antimicrobial peptide magainin II in tumor cells by bombesin-targeted delivery <303> Acta Pharmacol. Sin.<304> 32<305> 1<306> 79-88<307> 2011-01-01

<400> 39

```
Gly Ile Gly Lys Phe Leu His Ser Ala Lys Lys Phe Gly Lys Ala Phe
1               5                   10                  15

Val Gly Glu Ile Met Asn Ser Gly Gly Gln Arg Leu Gly Asn Gln Trp
            20                  25                  30

Ala Val Gly His Leu Met
        35
```

<210> 40
<211> 23
<212> PRT
<213> Artificial Sequence

<220>
<223> effector

<300>
<308> Swiss-Prot/P11006.1
<309> 2010-11-30
<313> (83)..(105)

<400> 40

Gly Ile Gly Lys Phe Leu His Ser Ala Lys Lys Phe Gly Lys Ala Phe
1 5 10 15

Val Gly Glu Ile Met Asn Ser 20

<210> 41
<211> 14
<212> PRT
<213> Artificial Sequence

<220>
<223> effector

<300>
<301> avadpour MM, Juban MM, Lo WC, Bishop SM, Alberty JB, Cowell SM, Becker CL, McLaughlin ML <302> De novo antimicrobial Peptides with low mammalian cell toxicity<303> J. Med. Chem.<304> 39<305> 16<306> 3107-13<307> 1996-08-02

<400> 41

Lys Leu Ala Lys Leu Ala Lys Lys Leu Ala Lys Leu Ala Lys
1 5 10

<210> 42
<211> 26
<212> PRT
<213> Artificial Sequence

<220>
<223> effector

<300>
<301> M., Velasco, M., J., Diaz-Guerra, P., Diaz-Achirica, D., Andreu, L., Rivas and L., Bosca,<302> Macrophage triggering with cecropin A and melittin-derived peptides induces type II nitric oxide synthase expression <303> The Journal of Immunology<304> 158<305> 9<306> 4437-4443<307> 1997-09-13

<400> 42

Lys Trp Lys Leu Phe Lys Lys Ile Gly Ile Gly Ala Val Leu Lys Val
1 5 10 15

Leu Thr Thr Gly Leu Pro Ala Leu Ile Ser
20 25

<210> 43
<211> 27
<212> PRT
<213> Artificial Sequence

<220>
<223> effector

<300>
<301> Isogai E.<302> Antimicrobial and Lipopolysaccharide-Binding Activities of C-Terminal Domain of Human CAP18 Peptides to Genus Leptospira<303> The Journal of Applied Research <304> 4 <305> 1 <306> 180-185<307> 2004-12-01

<400> 43

```
Phe Arg Lys Ser Lys Glu Lys Ile Gly Lys Phe Phe Lys Arg Ile Val
1               5                   10                  15

Gln Arg Ile Phe Asp Phe Leu Arg Asn Leu Val
            20                  25
```

<210> 44
<211> 27
<212> PRT
<213> Artificial Sequence

<220>
<223> effector

<300>
<301> Risso A, Braidot E, Sordano MC, Vianello A, Macr? F, Skerlavaj B, Zanetti M, Gennaro R, Bernardi P. <302> MAP-28, an antibiotic oeptide of innate immunity, induces cell death through opening of the mitochondrial permeability transition pore.<303> Mol. Cell. Biol. <304> 22<305> 6<306> 1926-35<307> 2002-03

<400> 44

```
Gly Gly Leu Arg Ser Leu Gly Arg Lys Ile Leu Arg Ala Trp Lys Lys
1               5                   10                  15

Tyr Gly Pro Ile Ile Val Pro Ile Ile Arg Ile
            20                  25
```

<210> 45
<211> 24
<212> PRT
<213> Artificial Sequence

<220>
<223> effector

<300>
<301> Andrä J, Berninghausen 0, Wülfken J, Leippe M. <302> Shortened amoebapore analogs with enhanced antibacterial and cytolytic activity.<303> FEBS Lett.<304> 385<305> 12<306> 96-100<307> 1996-04-29

<400> 45

```
Gly Leu Val Glu Thr Leu Thr Lys Ile Val Ser Tyr Gly Ile Asp Lys
1               5                   10                  15

Leu Ile Glu Lys Ile Leu Glu Gly
            20
```

<210> 46
<211> 24
<212> PRT
<213> Artificial Sequence

<220>
<223> effector

<300>
<301> Andrä J, Berninghausen 0, Wülfken J, Leippe M. <302> Shortened amoebapore analogs with enhanced antibacterial and cytolytic activity.<303> FEBS Lett.<304> 385<305> 1<306> 96-100<307> 1996-04-29

<400> 46

```
Gly Phe Ile Ala Thr Leu Thr Lys Val Leu Asp Phe Gly Ile Asp Lys
1               5                   10                  15

Leu Ile Gln Leu Ile Glu Asp Lys
            20
```

<210> 47
<211> 24
<212> PRT
<213> Artificial Sequence

<220>
<223> effector

<300>
<301> Andrä J, Berninghausen 0, Wülfken J, Leippe M. <302> Shortened amoebapore analogs with enhanced antibacterial and cytolytic activity.<303> FEBS Lett.<304> 385<305> 1<306> 96-100<307> 1996-04-29

<400> 47

```
Gly Phe Leu Gly Thr Leu Glu Lys Ile Leu Ser Phe Gly Val Asp Glu
1               5                   10                  15

Leu Val Lys Leu Ile Glu Asn His
            20
```

<210> 48
<211> 12
<212> PRT
<213> Artificial Sequence

<220>
<223> effector

<300>
<301> Ines Neundorf, Robert Rennert, Jan Hoyer, Franziska Schramm, Kristin Löbner Igor Kitanovic and Stefan Wölfl <302> Fusion of a Short HA2-Derived Peptide Sequence to Cell-Penetrating Peptides Improves Cytosolic Uptake, but Enhances Cytotoxic Activity.<303> Pharmaceuticals <304> 2<305> 2<306> 49-65<307> 2009

<400> 48

```
Gly Leu Leu Glu Ala Leu Ala Glu Leu Leu Glu Gly
1               5                   10
```

<210> 49
<211> 247
<212> PRT
<213> Artificial Sequence

<220>
<223> effector

<300>
<308> GenBank/AAP15462.1
<309> 2003-04-27
<313> (1)..(247)

<400> 49

```
Trp Asp Gly Lys Ile Asp Gly Thr Gly Thr His Ala Met Ile Val Thr
1               5                   10                  15

Gln Gly Val Ser Ile Leu Glu Asn Asp Met Ser Lys Asn Glu Pro Glu
            20                  25                  30

Ser Val Arg Lys Asn Leu Glu Ile Leu Lys Asp Asn Met His Glu Leu
        35                  40                  45
```

```
Gln Leu Gly Ser Thr Tyr Pro Asp Tyr Asp Lys Asn Ala Tyr Asp Leu
    50                  55                  60

Tyr Gln Asp His Phe Trp Asp Pro Asp Thr Asn Asn Asn Phe Ser Lys
65                  70                  75                  80

Asp Asn Ser Trp Tyr Leu Ala Tyr Ser Ile Pro Asp Thr Gly Glu Ser
                85                  90                  95

Gln Ile Arg Lys Phe Ser Ala Leu Ala Arg Tyr Glu Trp Gln Arg Gly
            100                 105                 110

Asn Tyr Lys Gln Ala Thr Phe Tyr Leu Gly Glu Ala Met His Tyr Phe
        115                 120                 125

Gly Asp Ile Asp Thr Pro Tyr His Pro Ala Asn Val Thr Ala Val Asp
    130                 135                 140

Ser Ala Gly His Val Lys Phe Glu Thr Phe Ala Glu Glu Arg Lys Glu
145                 150                 155                 160

Gln Tyr Lys Ile Asn Thr Val Gly Cys Lys Thr Asn Glu Asp Phe Tyr
                165                 170                 175

Ala Asp Ile Leu Lys Asn Lys Asp Phe Asn Ala Trp Ser Lys Glu Tyr
            180                 185                 190

Ala Arg Gly Phe Ala Lys Thr Gly Lys Ser Ile Tyr Tyr Ser His Ala
        195                 200                 205

Ser Met Ser His Ser Trp Asp Asp Trp Asp Tyr Ala Ala Lys Val Thr
    210                 215                 220

Leu Ala Asn Ser Gln Lys Gly Thr Ala Gly Tyr Ile Tyr Arg Phe Leu
225                 230                 235                 240

His Asp Val Ser Glu Gly Asn
                245
```

<210> 50
<211> 468
<212> PRT
<213> Artificial Sequence

<220>
<223> effector

<300>
<308> GenBank/AAS85208.1
<309> 2008-08-26
<313> (57)..(524)

<400> 50

```
His Ala Asp Glu Ile Asp Lys Tyr Ile Gln Gly Leu Asp Tyr Asn Lys
1               5                   10                  15

Asn Asn Val Leu Val Tyr His Gly Asp Ala Val Thr Asn Val Pro Pro
            20                  25                  30

Arg Lys Gly Tyr Lys Asp Gly Asn Glu Tyr Ile Val Val Glu Lys Lys
        35                  40                  45

Lys Lys Ser Ile Asn Gln Asn Asn Ala Asp Ile Gln Val Val Asn Ala
    50                  55                  60

Ile Ser Ser Leu Thr Tyr Pro Gly Ala Leu Val Lys Ala Asn Ser Glu
65                  70                  75                  80

Leu Val Glu Asn Gln Pro Asp Val Leu Pro Val Lys Arg Asp Ser Leu
                85                  90                  95

Thr Leu Ser Ile Asp Leu Pro Gly Met Thr Asn Gln Asp Asn Lys Ile
            100                 105                 110

Val Val Lys Asn Ala Thr Lys Ser Asn Val Asn Asn Ala Val Asn Thr
        115                 120                 125

Leu Val Glu Arg Trp Asn Glu Lys Tyr Ala Gln Ala Tyr Pro Asn Val
    130                 135                 140

Ser Ala Lys Ile Asp Tyr Asp Asp Glu Met Ala Tyr Ser Glu Ser Gln
145                 150                 155                 160

Leu Ile Ala Lys Phe Gly Thr Ala Phe Lys Ala Val Asn Asn Ser Leu
```

165 170 175

Asn Val Asn Phe Gly Ala Ile Ser Glu Gly Lys Met Gln Glu Glu Val
180 185 190

Ile Ser Phe Lys Gln Ile Tyr Tyr Asn Val Asn Val Asn Glu Pro Thr
195 200 205

Arg Pro Ser Arg Phe Phe Gly Lys Ala Val Thr Lys Glu Gln Leu Gln
210 215 220

Ala Leu Gly Val Asn Ala Glu Asn Pro Pro Ala Tyr Ile Ser Ser Val
225 230 235 240

Ala Tyr Gly Arg Gln Val Tyr Leu Lys Leu Ser Thr Asn Ser His Ser
245 250 255

Thr Lys Val Lys Ala Ala Phe Asp Ala Ala Val Ser Gly Lys Ser Val
260 265 270

Ser Gly Asp Val Glu Leu Thr Asn Ile Ile Lys Asn Ser Ser Phe Lys
275 280 285

Ala Val Ile Tyr Gly Gly Ser Ala Lys Asp Glu Val Gln Ile Ile Asp
290 295 300

Gly Asn Leu Gly Asp Leu Arg Asp Ile Leu Lys Lys Gly Ala Thr Phe
305 310 315 320

Asn Arg Glu Thr Pro Gly Val Pro Ile Ala Tyr Thr Thr Asn Phe Leu
325 330 335

Lys Asp Asn Glu Leu Ala Val Ile Lys Asn Asn Ser Glu Tyr Ile Glu
340 345 350

Thr Thr Ser Lys Ala Tyr Thr Asp Gly Lys Ile Asn Ile Asp His Ser
355 360 365

Gly Gly Tyr Val Ala Gln Phe Asn Ile Ser Trp Asp Glu Ile Asn Tyr
370 375 380

Asp Pro Glu Gly Asn Glu Ile Val Gln His Lys Asn Trp Ser Glu Asn
385                 390                 395                 400

Asn Lys Ser Lys Leu Ala His Phe Thr Ser Ser Ile Tyr Leu Pro Gly
                405                 410                 415

Asn Ala Arg Asn Ile Asn Val Tyr Ala Lys Glu Cys Thr Gly Leu Ala
            420                 425                 430

Trp Glu Trp Trp Arg Thr Val Ile Asp Asp Arg Asn Leu Pro Leu Val
        435                 440                 445

Lys Asn Arg Asn Ile Ser Ile Trp Gly Thr Thr Leu Tyr Pro Lys Tyr
    450                 455                 460

Ser Asn Ser Val
465

<210> 51
<211> 289
<212> PRT
<213> Artificial Sequence

<220>
<223> effector

<300>
<308> genbank/ABG57041.1
<309> 2011-05-06
<313> (5)..(292)

<400> 51

Lys Phe Lys Lys Val Val Leu Gly Met Cys Leu Thr Ala Ser Val Leu
1               5                   10                  15

Val Phe Pro Val Thr Ile Lys Ala Ser Ala Cys Cys Asp Glu Tyr Leu
            20                  25                  30

Lys Pro Pro Ala Ala Pro His Asp Ile Asp Ser Lys Leu Pro His Lys
        35                  40                  45

Leu Ser Trp Ser Ala Asp Asn Pro Thr Asn Thr Asp Val Asn Thr His
    50                  55                  60

```
Tyr Trp Leu Phe Lys Gln Ala Glu Lys Ile Leu Ala Lys Asp Val Asp
65                  70                  75                  80

His Met Arg Ala Asn Leu Met Asn Glu Leu Lys Asn Phe Asp Lys Gln
                85                  90                  95

Ile Ala Gln Gly Ile Tyr Asp Ala Asp His Lys Asn Pro Tyr Tyr Asp
            100                 105                 110

Thr Ser Thr Phe Leu Ser His Phe Tyr Asn Pro Asp Lys Asp Asn Thr
        115                 120                 125

Tyr Leu Pro Gly Phe Ala Asn Ala Lys Ile Thr Gly Ala Lys Tyr Phe
    130                 135                 140

Asn Gln Ser Val Ala Asp Tyr Arg Glu Gly Lys Phe Asp Thr Ala Phe
145                 150                 155                 160

Tyr Lys Leu Gly Leu Ala Ile His Tyr Tyr Thr Asp Ile Ser Gln Pro
                165                 170                 175

Met His Ala Asn Asn Phe Thr Ala Ile Ser Tyr Pro Pro Gly Tyr His
            180                 185                 190

Cys Ala Tyr Glu Asn Tyr Val Asp Thr Ile Lys His Asn Tyr Gln Ala
        195                 200                 205

Thr Glu Asp Met Val Val Gln Arg Phe Cys Ser Asn Asp Val Lys Glu
    210                 215                 220

Trp Leu Tyr Glu Asn Ala Lys Arg Ala Lys Ala Asp Tyr Pro Lys Ile
225                 230                 235                 240

Val Asn Ala Lys Thr Lys Lys Ser Tyr Leu Val Gly Asn Ser Glu Trp
                245                 250                 255

Lys Lys Asp Thr Val Glu Pro Thr Gly Ala Arg Leu Arg Asp Ser Gln
            260                 265                 270
```

```
Gln Thr Leu Ala Gly Phe Leu Glu Phe Trp Ser Lys Lys Thr Asn Glu
        275                 280                 285

Gly
```

<210> 52
<211> 179
<212> PRT
<213> Artificial Sequence

<220>
<223> effector

<300>
<308> PDB/1KD6_A
<309> 2009-07-10
<313> (1)..(179)

<400> 52

```
Ser Ala Asp Val Ala Gly Ala Val Ile Asp Gly Ala Ser Leu Ser Phe
1               5                   10                  15

Asp Ile Leu Lys Thr Val Leu Glu Ala Leu Gly Asn Val Lys Arg Lys
            20                  25                  30

Ile Ala Val Gly Val Asp Asn Glu Ser Gly Lys Thr Trp Thr Ala Leu
        35                  40                  45

Asn Thr Tyr Phe Arg Ser Gly Thr Ser Asp Ile Val Leu Pro His Lys
    50                  55                  60

Val Pro His Gly Lys Ala Leu Leu Tyr Asn Gly Gln Lys Asp Arg Gly
65                  70                  75                  80

Pro Val Ala Thr Gly Ala Val Gly Val Leu Ala Tyr Leu Met Ser Asp
                85                  90                  95

Gly Asn Thr Leu Ala Val Leu Phe Ser Val Pro Tyr Asp Tyr Asn Trp
            100                 105                 110

Tyr Ser Asn Trp Trp Asn Val Arg Ile Tyr Lys Gly Lys Arg Arg Ala
        115                 120                 125
```

```
Asp Gln Arg Met Tyr Glu Glu Leu Tyr Tyr Asn Leu Ser Pro Phe Arg
    130                 135                 140

Gly Asp Asn Gly Trp His Thr Arg Asn Leu Gly Tyr Gly Leu Lys Ser
145                 150                 155                 160

Arg Gly Phe Met Asn Ser Ser Gly His Ala Ile Leu Glu Ile His Val
                165                 170                 175

Thr Lys Ala
```

<210> 53
<211> 46
<212> PRT
<213> Artificial Sequence

<220>
<223> effector

<300>
<308> Swiss-Prot/P01538.2
<309> 2011-05-31
<313> (27)..(72)

<400> 53

```
Lys Ser Cys Cys Pro Asn Thr Thr Gly Arg Asn Ile Tyr Asn Ala Cys
1               5                   10                  15

Arg Leu Thr Gly Ala Pro Arg Pro Thr Cys Ala Lys Leu Ser Gly Cys
            20                  25                  30

Lys Ile Ile Ser Gly Ser Thr Cys Pro Ser Asp Tyr Pro Lys
        35                  40                  45
```

<210> 54
<211> 33
<212> PRT
<213> Artificial Sequence

<220>
<223> effector

<300>
<308> GenBank/CAA31612.1
<309> 2008-10-07
<313> (21)..(53)

<400> 54

Ala Pro Cys His Thr Ala Ala Arg Ser Glu Cys Lys Arg Ser His Lys
1               5                   10                  15

Phe Val Pro Gly Ala Trp Leu Ala Gly Glu Gly Val Asp Val Thr Ser
            20                  25                  30

Leu

<210> 55
<211> 251
<212> PRT
<213> Artificial Sequence

<220>
<223> effector

<300>
<308> PDB/2ZTB_A
<309> 2009-03-27
<313> (2)..(251)

<400> 55

Asp Val Ile Arg Glu Tyr Leu Met Phe Asn Glu Leu Ser Ala Leu Ser
1               5                   10                  15

Ser Ser Pro Glu Ser Val Arg Ser Arg Phe Ser Ser Ile Tyr Gly Thr
            20                  25                  30

Asn Pro Asp Gly Ile Ala Leu Asn Asn Glu Thr Tyr Phe Asn Ala Val
        35                  40                  45

Lys Pro Pro Ile Thr Ala Gln Tyr Gly Tyr Tyr Cys Tyr Lys Asn Val
    50                  55                  60

Gly Thr Val Gln Tyr Val Asn Arg Pro Thr Asp Ile Asn Pro Asn Val
65                  70                  75                  80

Ile Leu Ala Gln Asp Thr Leu Thr Asn Asn Thr Asn Glu Pro Phe Thr
                85                  90                  95

Thr Thr Ile Thr Ile Thr Gly Ser Phe Thr Asn Thr Ser Thr Val Thr
100 105 110

Ser Ser Thr Thr Thr Gly Phe Lys Phe Thr Ser Lys Leu Ser Ile Lys
115 120 125

Lys Val Phe Glu Ile Gly Gly Glu Val Ser Phe Ser Thr Thr Ile Gly
130 135 140

Thr Ser Glu Thr Thr Thr Glu Thr Ile Thr Val Ser Lys Ser Val Thr
145 150 155 160

Val Thr Val Pro Ala Gln Ser Arg Arg Thr Ile Gln Leu Thr Ala Lys
165 170 175

Ile Ala Lys Glu Ser Ala Asp Phe Ser Ala Pro Ile Thr Val Asp Gly
180 185 190

Tyr Phe Gly Ala Asn Phe Pro Lys Arg Val Gly Pro Gly Gly His Tyr
195 200 205

Phe Trp Phe Asn Pro Ala Arg Asp Val Leu Asn Thr Thr Ser Gly Thr
210 215 220

Leu Arg Gly Thr Val Thr Asn Val Ser Ser Phe Asp Phe Gln Thr Ile
225 230 235 240

Val Gln Pro Ala Arg Ser Leu Leu Asp Glu Gln
245 250

<210> 56
<211> 32
<212> PRT
<213> Artificial Sequence

<220>
<223> effector

<300>
<302> SELECTIVE ANTICANCER CHIMERIC PEPTIDE
<310> WO2010064207
<311> 2009-12-03
<312> 2010-06-10

<400> 56

Tyr Lys Trp Tyr Gly Tyr Thr Pro Gln Asn Val Ile Gly Gly Gly Lys
1 5 10 15

Leu Leu Leu Lys Leu Leu Lys Lys Leu Leu Lys Leu Leu Lys Lys Lys
20 25 30

<210> 60
<211> 681
<212> DNA
<213> Artificial Sequence

<220>
<223> Fusion Peptide

<400> 60

ggtctgggta gcgtttttgg tcgtctggca cgtattctgg gtcgtgttat tccgaaagtt 60

gcaaaaaaac tgggtccgaa agtggccaaa gttctgccga aagttatgaa agaagcaatt 120

ccgatggcag ttgaaatggc aaaaagccaa gaagaacagc agccgcagcg tgttgttcgt 180

ccgctgggtc tggcaggtcg tgttgcagca catattaccg gcacccgtgg tcgtagcaat 240

accctgagca gcccgaatag caaaaatgaa aaagcactgg gtcgcaaaat caatagctgg 300

gaaagcagcc gtagcggtca tagctttctg agcaatctgc atctgcgtaa tggtgaactg 360

gtgattcatg aaaaaggctt ttattatatt tatagccaga cctattttcg ctttcaagaa 420

gagattaaag aaaataccaa aaatgataaa caaatggtgc agtacattta caaatatacc 480

agctatccgg acccgattct gctgatgaaa agcgcacgta atagctgttg gagcaaagat 540

gcagaatatg gtctgtatag catttatcag ggtggcatct ttgagctgaa agaaaatgat 600

cgcatctttg ttagcgtgac caacgaacat ctgatcgata tggatcatga agccagcttt 660

tttggtgcat ttctggtggg t 681

<210> 61
<211> 792
<212> DNA
<213> Artificial Sequence

<220>
<223> Fusion Peptide

<400> 61

```
accagcgaag aaaccattag caccgttcaa gaaaaacagc agaatattag tccgctggtt        60
cgtgaacgtg gtccgcagcg tgttgcagca catattaccg gcacccgtgg tcgtagcaat       120
accctgagca gcccgaatag caaaaatgaa aaagccctgg gtcgcaaaat taacagctgg       180
gaaagcagcc gtagcggtca tagctttctg agcaatctgc atctgcgtaa tggtgaactg       240
gtgattcacg agaaaggctt ctattatatc tatagccaga cctattttcg ctttcaagaa       300
gaaattaaag aaaacaccaa aaatgataaa caaatggtgc agtatattta caaatatacc       360
agctatccgg atccgattct gctgatgaaa agcgcacgta atagctgttg gagcaaagat       420
gcagaatatg gcctgtatag catctatcag ggtggcattt ttgaactgaa agaaaacgat       480
cgcatctttg tgagcgtgac caatgaacat ctgattgata tggatcacga agccagcttt       540
tttggtgcat ttctggttgg ttgtgcagca tgtgcagccg catgtggtgg tggtccgctg       600
ggtctggcag gtcgtgttgt tcgtggtctg ggtagcgttt ttggtcgtct ggcacgtatt       660
ctgggtcgtg ttattccgaa agttgcaaaa aaactgggtc cgaaagtggc caaagttctg       720
ccgaaagtta tgaaagaagc aattccgatg gccgttgaaa tggcaaaaag ccaagaagaa       780
cagcagccgc ag                                                           792
```

<210> 62
<211> 897
<212> DNA
<213> Artificial Sequence

<220>
<223> Fusion Peptide

<400> 62

```
accagcgaag aaaccattag caccgttcaa gaaaaacagc agaatattag tccgctggtt        60
cgtgaacgtg gtccgcagcg tgttgcagca catattaccg gcacccgtgg tcgtagcaat       120
accctgagca gcccgaatag caaaaatgaa aaagcactgg gtcgcaaaat caatagctgg       180
gaaagcagcc gtagcggtca tagctttctg agcaatctgc atctgcgtaa tggtgaactg       240
gtgattcatg aaaaaggctt ctactatatc tatagccaga cctatttccg cttccaagaa       300
gaaatcaaag aaaataccaa aaatgataaa caaatggtgc agtatattta caaatatacc       360
agctatccgg atccgattct gctgatgaaa agcgcacgta atagctgttg gagcaaagat       420
gcagaatatg gtctgtatag catttatcag ggtggcatct ttgagctgaa agaaaatgat       480
```

cgcatctttg ttagcgtgac caacgaacat ctgatcgata tggatcatga agccagcttt 540

tttggtgcat ttctggttgg tggtagcggt tgtgcagcat gtgcagccgc atgtccgctg 600

ggtctggcag gtcgtgttgt tcgtggtaaa ctgagctgtc tgagcctggc actggcaatt 660

attctgattc tggcaattgt tcatagcccg aatatggaag ttaaagcact ggcagatccg 720

gaagcagatg catttggtga agcaaatgcc tttggcgaag ccgatgcgtt tgccgaagcc 780

aatgcagatg ttaaaggtat gaaaaaagcc attaaagaaa ttctggattg cgtgatcgag 840

aaaggctatg ataaactggc agccaaactg aaaaaagtta ttcagcagct gtgggaa 897

<210> 67
<211> 606
<212> DNA
<213> Artificial Sequence

<220>
<223> Fusion Peptide

<400> 67

cgtgttgcag cacatattac cggcacccgt ggtcgtagca ataccctgag cagcccgaat 60

agcaaaaatg aaaaagcact gggtcgcaaa attaatagct gggaaagcag ccgtagcggt 120

catagctttc tgagcaatct gcatctgcgt aatggtgaac tggtgattca tgaaaaaggc 180

ttttattata tttatagcca gacctatttt cgctttcaag aagaaattaa agaaaacacc 240

aaaaatgata aacaaatggt gcagtatatt tacaaatata ccagctatcc ggatccgatt 300

ctgctgatga aaagcgcacg taatagctgt tggagcaaag atgcagaata tggtctgtat 360

agcatttatc agggtggcat ttttgaactg aaagaaaatg atcgcatttt tgtgagcgtg 420

accaatgaac atctgattga tatggatcat gaagccagct tttttggtgc atttctggtt 480

ggtggtggtg gcggtagcgg tggtggtggt cgtgttgttc gtccgctggg tctggcaggt 540

cgtcgtcgtc gccgtcgtcg gcgtaaactg gcaaaactgg ccaaaaaact ggcgaaactg 600

gctaaa 606

<210> 68
<211> 615
<212> DNA
<213> Artificial Sequence

<220>
<223> Fusion Peptide

<400> 68

cgtgttgcag cacatattac cggcacccgt ggtcgtagca ataccctgag cagcccgaat 60
agcaaaaatg aaaaagcact gggtcgcaaa attaatagct gggaaagcag ccgtagcggt 120
catagctttc tgagcaatct gcatctgcgt aatggtgaac tggtgattca tgaaaaaggc 180
ttttattata tttatagcca gacctatttt cgctttcaag aagaaattaa agaaaacacc 240
aaaaatgata aacaaatggt gcagtatatc tacaaatata ccagctatcc ggatccgatt 300
ctgctgatga aaagcgcacg taatagctgt tggagcaaag atgcagaata tggtctgtat 360
agcatttatc agggtggcat ttttgaactg aaagaaaatg atcgcatttt tgtgagcgtg 420
accaatgaac atctgattga tatggatcat gaagccagct tttttggtgc atttctggtt 480
ggtggtggtg caagcggttg tggtccggaa ggtggtggtg gtccgctggg tctggcaggt 540
cgtgttgttc gtcgtcgtcg tcgccgtcgc cgtaaactgg caaaactggc caaaaaactg 600
gcgaaactgg ctaaa 615

<210> 69
<211> 684
<212> DNA
<213> Artificial Sequence

<220>
<223> Fusion Peptide

<400> 69

accagcgaag aaaccattag caccgttcag gaaaaacagc agaatattag tccgctggtt 60
cgtgaacgtg gtccgcagcg tgttgcagca catattaccg gcacccgtgg tcgtagcaat 120
accctgagca gcccgaatag caaaaatgaa aaagcactgg gtcgcaaaat taatagctgg 180
gaaagcagcc gtagcggtca tagctttctg agcaatctgc atctgcgtaa tggtgaactg 240
gtgattcatg aaaaaggctt ttattatatt tatagccaga cctattttcg ctttcaggaa 300
gaaattaaag aaaataccaa aaatgataaa caaatggtgc agtatatcta taaatacacc 360
agctatccgg atccgattct gctgatgaaa agcgcacgta atagctgttg gagcaaagat 420
gcagaatatg gtctgtatag catttatcag ggtggcattt ttgaactgaa agaaaatgat 480
cgcatttttg tgagcgtgac caatgaacat ctgattgata tggatcatga agccagcttt 540

tttggtgcat ttctggttgg tggtggtggc ggtagcggtg gtggtggtcg tgttgttcgt 600

ccgctgggtc tggcaggtcg tcgtcgtcgt agacgtcgtc gtaaactggc aaaactggcc 660

aaaaaactgg cgaaactggc taaa 684

<210> 70
<211> 576
<212> DNA
<213> Artificial Sequence

<220>
<223> Fusion Peptide

<400> 70

catcatcatc accatcacaa actggcaaaa ctggccaaaa aactggcgaa actggctaaa 60

tgtcgtgttg ttcgtccgct gggtctggca ggtcgtgttg cagcacatat taccggcacc 120

cgtggtcgta gcaatacct gagcagcccg aatagcaaaa atgaaaaagc actgggtcgc 180

aaaatcaata gctgggaaag cagccgtagc ggtcatagct ttctgagcaa tctgcatctg 240

cgtaatggtg aactggtgat tcatgaaaaa ggcttttatt atatttatag ccagacctat 300

tttcgctttc aagaagagat taaagaaaat accaaaaatg ataaacaaat ggtgcagtat 360

atctataaat ataccagcta tccggacccg attctgctga tgaaaagcgc acgtaatagc 420

tgttggagca aagatgcaga atatggtctg tatagcattt atcagggtgg catctttgag 480

ctgaaagaaa atgatcgcat ctttgttagc gtgaccaacg aacatctgat cgatatggat 540

catgaagcca gctttttggg tgcatttctg gtgggt 576

<210> 71
<211> 600
<212> DNA
<213> Artificial Sequence

<220>
<223> Fusion Peptide

<400> 71

catcatcatc accatcacaa actggcaaaa ctggccaaaa aactggcgaa actggctaaa 60

tgtcgtcgtc gtcgccgtcg gcgtcgtcgt gttgttcgtc cgctgggtct ggcaggtcgt 120

gttgcagcac atattaccgg cacccgtggt cgtagcaata ccctgagcag cccgaatagc 180

aaaaatgaaa aagcactggg tcgcaaaatc aatagctggg aaagcagccg tagcggtcat 240

agctttctga gcaatctgca tctgcgtaat ggtgaactgg tgattcatga aaaaggcttt 300

tattatattt atagccagac ctattttcgc tttcaagaag agattaaaga aaataccaaa 360

aatgataaac aaatggtgca gtatatctat aaatacacca gctatccgga cccgattctg 420

ctgatgaaaa gcgcacgtaa tagctgttgg agcaaagatg cagaatatgg tctgtatagc 480

atttatcagg gtggcatctt tgagctgaaa gaaaatgatc gcatctttgt tagcgtgacc 540

aacgaacatc tgatcgatat ggatcatgaa gccagctttt ttggtgcatt tctggtgggt 600

<210> 72
<211> 606
<212> DNA
<213> Artificial Sequence

<220>
<223> Fusion Peptide

<400> 72

cgtgttgcag cacatattac cggcacccgt ggtcgtagca ataccctgag cagcccgaat 60

agcaaaaatg aaaaagcact gggtcgcaaa attaatagct gggaaagcag ccgtagcggt 120

catagctttc tgagcaatct gcatctgcgt aatggtgaac tggtgattca tgaaaaaggc 180

ttttattata tttatagcca gacctatttt cgctttcagg aagaaattaa agaaaatacc 240

aaaaatgata aacaaatggt gcagtatatc tataaataca ccagctatcc ggatccgatt 300

ctgctgatga aaagcgcacg taatagctgt tggagcaaag atgcagaata tggtctgtat 360

agcatttatc agggtggcat ttttgaactg aaagaaaatg atcgcatttt tgtgagcgtg 420

accaatgaac atctgattga tatggatcat gaagccagct tttttggtgc atttctggtt 480

ggtggtggtg gcggtagcgg tggtggtggt cgtgttgttc gtccgctggg tctggcaggt 540

cgtcgtcgtc gtagacgtcg tcgtaaactg gcaaaactgg ccaaaaaact ggcgaaactg 600

gctaaa 606

<210> 74
<211> 609
<212> DNA
<213> Artificial Sequence

<220>
<223> Fusion Peptide

<400> 74

tttcgcaaaa gcaaagaaaa aattggcaaa ttttttaaac gcattgtgca gcgcattttt 60
gattttctgc gtaatctggt tcgtgttgtt cgtccgctgg gtctggcagg cgaacgtggt 120
ccgcagcgtg ttgcagcaca tattaccggc acccgtggtc gtagcaatac cctgagcagc 180
ccgaatagca aaaatgaaaa agcactgggt cgcaaaatta atagctggga aagcagccgt 240
agcggtcata gctttctgag caatctgcat ctgcgtaatg gtgaactggt gattcatgaa 300
aaaggctttt attatattta tagccagacc tattttcgct ttcaagagga aattaaagaa 360
aataccaaaa atgataaaca aatggtgcag tatatctata aatataccag ctatccggat 420
ccgattctgc tgatgaaaag cgcacgtaat agctgttgga gcaaagatgc agaatatggt 480
ctgtatagca tttatcaggg tggcattttt gaactgaaag aaaatgatcg catttttgtg 540
agcgtgacca atgaacatct gattgatatg gatcatgaag ccagcttttt tggtgcattt 600
ctggttggt 609

<210> 75
<211> 609
<212> DNA
<213> Artificial Sequence

<220>
<223> Fusion Peptide

<400> 75

ggtggtctgc gtagcctggg tcgtaaaatt ctgcgtgcat ggaaaaaata tggtccgatt 60
attgtgccga ttattcgtat tcgtgttgtt cgtccgctgg gtctggcagg cgaacgtggt 120
ccgcagcgtg ttgcagcaca tattaccggc acccgtggtc gtagcaatac cctgagcagc 180
ccgaatagca aaaatgaaaa agcactgggt cgcaaaatta atagctggga aagcagccgt 240
agcggtcata gctttctgag caatctgcat ctgcgtaatg gtgaactggt gattcatgaa 300
aaaggctttt attatattta tagccagacc tattttcgct ttcaagagga aattaaagaa 360
aataccaaaa atgataaaca aatggtgcag tatatctata aatataccag ctatccggat 420
ccgattctgc tgatgaaaag cgcacgtaat agctgttgga gcaaagatgc agaatatggt 480
ctgtatagca tttatcaggg tggcattttt gaactgaaag aaaatgatcg catttttgtg 540
agcgtgacca atgaacatct gattgatatg gatcatgaag ccagcttttt tggtgcattt 600
ctggttggt 609

<210> 76
<211> 600
<212> DNA
<213> Artificial Sequence

<220>
<223> Fusion Peptide

<400> 76

```
cgtgttgcag cacatattac cggcacccgt ggtcgtagca ataccctgag cagcccgaat        60
agcaaaaatg aaaaagcact gggtcgcaaa attaatagct gggaaagcag ccgtagcggt       120
catagctttc tgagcaatct gcatctgcgt aatggtgaac tggtgattca tgaaaaaggc       180
ttttattata tttatagcca gacctatttt cgctttcagg aagaaattaa agaaaatacc       240
aaaaatgata agcagatggt gcagtatatc tataaatata ccagctatcc ggatccgatt       300
ctgctgatga aaagcgcacg taatagctgt tggagcaaag atgcagaata tggtctgtat       360
agcatttatc agggtggcat ttttgaactg aaagaaaatg atcgcatttt tgtgagcgtg       420
accaatgaac atctgattga tatggatcat gaagccagct tttttggtgc atttctggtt       480
ggtggtggta gcggtggtcc gctgggtctg gcaggtcgtg ttgttcgtgg tctggttgaa       540
accctgacca aaattgttag ctatggtatt gataaactga ttgaaaaaat tctggaaggt       600
```

<210> 77
<211> 600
<212> DNA
<213> Artificial Sequence

<220>
<223> Fusion Peptide

<400> 77

```
cgtgttgcag cacatattac cggcacccgt ggtcgtagca ataccctgag cagcccgaat        60
agcaaaaatg aaaaagcact gggtcgcaaa attaatagct gggaaagcag ccgtagcggt       120
catagctttc tgagcaatct gcatctgcgt aatggtgaac tggtgattca tgaaaaaggc       180
ttttattata tttatagcca gacctatttt cgctttcagg aagaaattaa agaaaatacc       240
aaaaatgata agcagatggt gcagtatatc tataaatata ccagctatcc ggatccgatt       300
ctgctgatga aaagcgcacg taatagctgt tggagcaaag atgcagaata tggtctgtat       360
agcatttatc agggtggcat ttttgaactg aaagaaaatg atcgcatttt tgtgagcgtg       420
```

accaatgaac atctgattga tatggatcat gaagccagct tttttggtgc atttctggtt 480

ggtggtggta gcggtggtcc gctgggtctg gcaggtcgtg ttgttcgtgg ttttattgca 540

accctgacca aagttctgga ttttggtatt gataaactga ttcagctgat tgaagataaa 600

<210> 78
<211> 600
<212> DNA
<213> Artificial Sequence

<220>
<223> Fusion Peptide

<400> 78

cgtgttgcag cacatattac cggcacccgt ggtcgtagca ataccctgag cagcccgaat 60

agcaaaaatg aaaaagcact gggtcgcaaa attaatagct gggaaagcag ccgtagcggt 120

catagctttc tgagcaatct gcatctgcgt aatggtgaac tggtgattca tgaaaaaggc 180

ttttattata tttatagcca gacctatttt cgctttcagg aagaaattaa agaaaatacc 240

aaaaatgata agcagatggt gcagtatatc tataaatata ccagctatcc ggatccgatt 300

ctgctgatga aaagcgcacg taatagctgt tggagcaaag atgcagaata tggtctgtat 360

agcatttatc agggtggcat ttttgaactg aaagaaaatg atcgcatttt tgtgagcgtg 420

accaatgaac atctgattga tatggatcat gaagccagct tttttggtgc atttctggtt 480

ggtggtggta gcggtggtcc gctgggtctg gcaggtcgtg ttgttcgtgg ttttctgggc 540

accctggaaa aaattctgag ctttggtgtt gatgaactgg ttaaactgat tgaaaatcat 600

<210> 79
<211> 570
<212> DNA
<213> Artificial Sequence

<220>
<223> Fusion Peptide

<400> 79

ggtctgctgg aagcactggc agaactgctg gaaggtcggc gtcgtcgtcg tcggcgtcgt 60

gttgttcgtc cgctgggtct ggcaggtcgt gttgcagcac atattaccgg cacccgtggt 120

cgtagcaata ccctgagcag cccgaatagc aaaaatgaaa aagcactggg tcgcaaaatt 180

aatagctggg aaagcagccg tagcggtcat agctttctga gcaatctgca tctgcgtaat 240

ggtgaactgg tgattcatga aaaaggcttt tattatattt atagccagac ctattttcgc 300
tttcaggaag aaattaaaga aaacaccaaa aacgataaac aaatggtgca gtatatctat 360
aaatacacca gctatccgga tccgattctg ctgatgaaaa gcgcacgtaa tagctgttgg 420
agcaaagatg cagaatatgg tctgtatagc atttatcagg gtggcatttt tgaactgaaa 480
gaaaatgatc gcatttttgt gagcgtgacc aatgaacatc tgattgatat ggatcatgaa 540
gccagctttt ttggtgcatt tctggttggt 570

<210> 86
<211> 600
<212> DNA
<213> Artificial Sequence

<220>
<223> Fusion Peptide

<400> 86

cgtgttgcag cacatattac cggcacccgt ggtcgtagca ataccctgag cagcccgaat 60
agcaaaaatg aaaaagcact gggtcgcaaa atcaatagct gggaaagcag ccgtagcggt 120
catagctttc tgagcaatct gcatctgcgt aatggtgaac tggtgattca tgaaaaaggc 180
ttttattata tttatagcca gacctatttt cgctttcaag aagagattaa agaaaatacc 240
aaaaatgata aacaaatggt gcagtacatc tataaatata ccagctatcc ggacccgatt 300
ctgctgatga aaagcgcacg taatagctgt tggagcaaag atgcagaata tggtctgtat 360
agcatttatc agggtggcat ctttgagctg aaagaaaatg atcgcatctt tgttagcgtg 420
accaacgaac atctgatcga tatggatcat gaagccagct tttttggtgc atttctggtt 480
ggtggtggtg gcggtagcgg agcaccgtgt cataccgcag cacgtagcga atgtaaacgt 540
agccataaat ttgttccggg tgcatggctg gcaggcgaag gtgttgatgt taccagcctg 600

<210> 87
<211> 630
<212> DNA
<213> Artificial Sequence

<220>
<223> Fusion Peptide

<400> 87

cgtgttgcag cacatattac cggcacccgt ggtcgtagca ataccctgag cagcccgaat 60

agcaaaaatg aaaaagcact gggtcgcaaa atcaatagct gggaaagcag ccgtagcggt 120

catagctttc tgagcaatct gcatctgcgt aatggtgaac tggtgattca tgaaaaaggc 180

ttttattata tttatagcca gacctatttt cgctttcaag aagagattaa agaaaatacc 240

aaaaatgata aacaaatggt gcagtacatc tataaatata ccagctatcc ggacccgatt 300

ctgctgatga aaagcgcacg taatagctgt tggagcaaag atgcagaata tggtctgtat 360

agcatttatc agggtggcat ctttgagctg aaagaaaatg atcgcatctt tgttagcgtg 420

accaacgaac atctgatcga tatggatcat gaagccagct tttttggtgc atttctggtt 480

ggtggtggtg gcggtagcgg tcgtgttgtt cgtccgctgg gtctggctgg cgcaccgtgt 540

cataccgcag cacgtagcga atgtaaacgt agccataaat ttgttccggg tgcatggctg 600

gcaggcgaag gtgttgatgt taccagcctg 630

<210> 88
<211> 1308
<212> DNA
<213> Artificial Sequence

<220>
<223> Fusion Peptide

<400> 88

gatgtgattc gcgaatatct gatgtttaat gaactgagcg cactgagcag cagtccggaa 60

agcgttcgta gccgttttag cagcatttat ggcaccaatc cggatggtat tgcactgaat 120

aatgaaacct atttcaatgc cgtgaaacct ccgattaccg cacagtatgg ttattattgc 180

tacaaaaatg ttggcaccgt gcagtatgtt aatcgtccga ccgatattaa tccgaatgtt 240

attctggcac aggataccct gaccaataat accaatgaac cgtttaccac caccattacc 300

attaccggta gctttaccaa taccagcacc gttaccagca gcaccaccac cggtttcaaa 360

tttaccagca aactgagcat caaaaaagtg tttgaaattg gtggcgaagt gagctttagc 420

accaccattg gcaccagcga aaccaccacc gaaaccatta ccgtgagcaa aagcgttacc 480

gttaccgttc cggcacagag ccgtcgtacc attcagctga ccgcaaaaat tgcaaaagaa 540

agcgcagatt ttagcgcacc gattaccgtt gatggttatt ttggtgcaaa ttttccgaaa 600

cgtgttggtc cgggtggtca ttacttttgg tttaatccgg cacgtgatgt gctgaatacc 660

accagtggca ccctgcgtgg tacagttacc aatgtttcta gctttgattt tcagaccatt 720

gttcagcctg cacgtagcct gctggatgaa cagcgtgttg ttcgtccgct gggtctggca 780

ggcggtagcg gtggtggttc aggtggtggt gaacgtggtc cgcagcgtgt tgcagcacat 840

attaccggca cccgtggtcg tagcaatacc ctgagcagcc cgaatagcaa aaatgaaaaa 900

gcactgggtc gcaaaatcaa tagctgggaa agcagccgta gcggtcatag ctttctgagc 960

aatctgcatc tgcgtaatgg tgaactggtg attcatgaaa aaggcttcta ctatatttac 1020

agccagacct attttcgctt tcaggaagaa attaaagaaa ataccaaaaa tgataaacaa 1080

atggtgcagt atatctataa atacaccagc tatccggatc cgattctgct gatgaaaagc 1140

gcacgtaata gctgttggag caaagatgca gaatatggcc tgtatagcat ttatcagggt 1200

ggcatttttg aactgaaaga aaatgatcgc atttttgtga gcgtgaccaa tgaacatctg 1260

attgatatgg atcatgaagc aagtttcttt ggtgcatttc tggtgggc 1308

<210> 90
<211> 281
<212> PRT
<213> Homo sapiens

<400> 90

Met Ala Met Met Glu Val Gln Gly Gly Pro Ser Leu Gly Gln Thr Cys
1               5                   10                  15

Val Leu Ile Val Ile Phe Thr Val Leu Leu Gln Ser Leu Cys Val Ala
            20                  25                  30

Val Thr Tyr Val Tyr Phe Thr Asn Glu Leu Lys Gln Met Gln Asp Lys
        35                  40                  45

Tyr Ser Lys Ser Gly Ile Ala Cys Phe Leu Lys Glu Asp Asp Ser Tyr
    50                  55                  60

Trp Asp Pro Asn Asp Glu Glu Ser Met Asn Ser Pro Cys Trp Gln Val
65                  70                  75                  80

Lys Trp Gln Leu Arg Gln Leu Val Arg Lys Met Ile Leu Arg Thr Ser
                85                  90                  95

Glu Glu Thr Ile Ser Thr Val Gln Glu Lys Gln Gln Asn Ile Ser Pro
            100                 105                 110

```
Leu Val Arg Glu Arg Gly Pro Gln Arg Val Ala Ala His Ile Thr Gly
        115                 120                 125

Thr Arg Gly Arg Ser Asn Thr Leu Ser Ser Pro Asn Ser Lys Asn Glu
    130                 135                 140

Lys Ala Leu Gly Arg Lys Ile Asn Ser Trp Glu Ser Ser Arg Ser Gly
145                 150                 155                 160

His Ser Phe Leu Ser Asn Leu His Leu Arg Asn Gly Glu Leu Val Ile
                165                 170                 175

His Glu Lys Gly Phe Tyr Tyr Ile Tyr Ser Gln Thr Tyr Phe Arg Phe
            180                 185                 190

Gln Glu Glu Ile Lys Glu Asn Thr Lys Asn Asp Lys Gln Met Val Gln
        195                 200                 205

Tyr Ile Tyr Lys Tyr Thr Ser Tyr Pro Asp Pro Ile Leu Leu Met Lys
    210                 215                 220

Ser Ala Arg Asn Ser Cys Trp Ser Lys Asp Ala Glu Tyr Gly Leu Tyr
225                 230                 235                 240

Ser Ile Tyr Gln Gly Gly Ile Phe Glu Leu Lys Glu Asn Asp Arg Ile
                245                 250                 255

Phe Val Ser Val Thr Asn Glu His Leu Ile Asp Met Asp His Glu Ala
            260                 265                 270

Ser Phe Phe Gly Ala Phe Leu Val Gly
        275                 280
```

<210> 91
<211> 223
<212> PRT
<213> Artificial Sequence

<220>
<223> -

<400> 91

```
Lys Leu Leu Leu Lys Leu Leu Lys Lys Leu Leu Lys Leu Leu Lys Lys
1               5                   10                  15

Lys Gly Gly Gly Tyr Gly Arg Pro Arg Gln Ser Gly Lys Lys Arg Lys
            20                  25                  30

Arg Lys Arg Leu Lys Pro Thr Arg Val Val Arg Pro Leu Gly Leu Ala
        35                  40                  45

Gly Gly Gly Cys Ala Ala Ala Cys Ala Ala Cys Ser Gly Gly Arg Val
    50                  55                  60

Ala Ala His Ile Thr Gly Thr Arg Gly Arg Ser Asn Thr Leu Ser Ser
65                  70                  75                  80

Pro Asn Ser Lys Asn Glu Lys Ala Leu Gly Arg Lys Ile Asn Ser Trp
                85                  90                  95

Glu Ser Ser Arg Ser Gly His Ser Phe Leu Ser Asn Leu His Leu Arg
            100                 105                 110

Asn Gly Glu Leu Val Ile His Glu Lys Gly Phe Tyr Tyr Ile Tyr Ser
        115                 120                 125

Gln Thr Tyr Phe Arg Phe Gln Glu Glu Ile Lys Glu Asn Thr Lys Asn
    130                 135                 140

Asp Lys Gln Met Val Gln Tyr Ile Tyr Lys Tyr Thr Ser Tyr Pro Asp
145                 150                 155                 160

Pro Ile Leu Leu Met Lys Ser Ala Arg Asn Ser Cys Trp Ser Lys Asp
                165                 170                 175

Ala Glu Tyr Gly Leu Tyr Ser Ile Tyr Gln Gly Gly Ile Phe Glu Leu
            180                 185                 190

Lys Glu Asn Asp Arg Ile Phe Val Ser Val Thr Asn Glu His Leu Ile
        195                 200                 205
```

```
Asp Met Asp His Glu Ala Ser Phe Phe Gly Ala Phe Leu Val Gly
    210                 215                 220
```

<210> 92
<211> 223
<212> PRT
<213> Artificial Sequence

<220>
<223> -

<400> 92

```
Lys Leu Ala Lys Leu Ala Lys Lys Leu Ala Lys Leu Ala Lys Arg Arg
1               5                   10                  15

Arg Arg Arg Arg Arg Arg Lys Lys Arg Gly Gly Gly Cys Ala Ala Ala
            20                  25                  30

Cys Ala Ala Cys Thr Ser Glu Glu Thr Ile Ser Thr Val Gln Glu Lys
        35                  40                  45

Gln Gln Asn Ile Ser Pro Leu Val Arg Glu Arg Gly Pro Gln Arg Val
    50                  55                  60

Ala Ala His Ile Thr Gly Thr Arg Gly Arg Ser Asn Thr Leu Ser Ser
65                  70                  75                  80

Pro Asn Ser Lys Asn Glu Lys Ala Leu Gly Arg Lys Ile Asn Ser Trp
                85                  90                  95

Glu Ser Ser Arg Ser Gly His Ser Phe Leu Ser Asn Leu His Leu Arg
            100                 105                 110

Asn Gly Glu Leu Val Ile His Glu Lys Gly Phe Tyr Tyr Ile Tyr Ser
        115                 120                 125

Gln Thr Tyr Phe Arg Phe Gln Glu Glu Ile Lys Glu Asn Thr Lys Asn
    130                 135                 140

Asp Lys Gln Met Val Gln Tyr Ile Tyr Lys Tyr Thr Ser Tyr Pro Asp
145                 150                 155                 160
```

Pro Ile Leu Leu Met Lys Ser Ala Arg Asn Ser Cys Trp Ser Lys Asp
165 170 175

Ala Glu Tyr Gly Leu Tyr Ser Ile Tyr Gln Gly Gly Ile Phe Glu Leu
180 185 190

Lys Glu Asn Asp Arg Ile Phe Val Ser Val Thr Asn Glu His Leu Ile
195 200 205

Asp Met Asp His Glu Ala Ser Phe Phe Gly Ala Phe Leu Val Gly
210 215 220

<210> 93
<211> 232
<212> PRT
<213> Artificial Sequence

<220>
<223> -

<400> 93

Lys Leu Ala Lys Leu Ala Lys Lys Leu Ala Lys Leu Ala Lys Arg Arg
1 5 10 15

Arg Arg Arg Arg Arg Arg Lys Lys Arg His Arg Gln Pro Arg Gly Trp
20 25 30

Glu Gln Gly Gly Gly Cys Ala Ala Ala Cys Ala Ala Cys Thr Ser Glu
35 40 45

Glu Thr Ile Ser Thr Val Gln Glu Lys Gln Gln Asn Ile Ser Pro Leu
50 55 60

Val Arg Glu Arg Gly Pro Gln Arg Val Ala Ala His Ile Thr Gly Thr
65 70 75 80

Arg Gly Arg Ser Asn Thr Leu Ser Ser Pro Asn Ser Lys Asn Glu Lys
85 90 95

Ala Leu Gly Arg Lys Ile Asn Ser Trp Glu Ser Ser Arg Ser Gly His
100 105 110

```
Ser Phe Leu Ser Asn Leu His Leu Arg Asn Gly Glu Leu Val Ile His
        115                 120                 125

Glu Lys Gly Phe Tyr Tyr Ile Tyr Ser Gln Thr Tyr Phe Arg Phe Gln
    130                 135                 140

Glu Glu Ile Lys Glu Asn Thr Lys Asn Asp Lys Gln Met Val Gln Tyr
145                 150                 155                 160

Ile Tyr Lys Tyr Thr Ser Tyr Pro Asp Pro Ile Leu Leu Met Lys Ser
                165                 170                 175

Ala Arg Asn Ser Cys Trp Ser Lys Asp Ala Glu Tyr Gly Leu Tyr Ser
            180                 185                 190

Ile Tyr Gln Gly Gly Ile Phe Glu Leu Lys Glu Asn Asp Arg Ile Phe
        195                 200                 205

Val Ser Val Thr Asn Glu His Leu Ile Asp Met Asp His Glu Ala Ser
    210                 215                 220

Phe Phe Gly Ala Phe Leu Val Gly
225                 230
```

<210> 94
<211> 207
<212> PRT
<213> Artificial Sequence

<220>
<223> -

<400> 94

```
Arg Val Ala Ala His Ile Thr Gly Thr Arg Gly Arg Ser Asn Thr Leu
1               5                   10                  15

Ser Ser Pro Asn Ser Lys Asn Glu Lys Ala Leu Gly Arg Lys Ile Asn
            20                  25                  30

Ser Trp Glu Ser Ser Arg Ser Gly His Ser Phe Leu Ser Asn Leu His
        35                  40                  45
```

```
Leu Arg Asn Gly Glu Leu Val Ile His Glu Lys Gly Phe Tyr Tyr Ile
    50                  55                  60

Tyr Ser Gln Thr Tyr Phe Arg Phe Gln Glu Glu Ile Lys Glu Asn Thr
65                  70                  75                  80

Lys Asn Asp Lys Gln Met Val Gln Tyr Ile Tyr Lys Tyr Thr Ser Tyr
                85                  90                  95

Pro Asp Pro Ile Leu Leu Met Lys Ser Ala Arg Asn Ser Cys Trp Ser
            100                 105                 110

Lys Asp Ala Glu Tyr Gly Leu Tyr Ser Ile Tyr Gln Gly Gly Ile Phe
        115                 120                 125

Glu Leu Lys Glu Asn Asp Arg Ile Phe Val Ser Val Thr Asn Glu His
    130                 135                 140

Leu Ile Asp Met Asp His Glu Ala Ser Phe Phe Gly Ala Phe Leu Val
145                 150                 155                 160

Gly Gly Gly Gly Gly Ser Gly Gly Gly Gly Pro Leu Gly Leu Ala Gly
                165                 170                 175

Arg Val Val Arg Tyr Ala Arg Ala Ala Ala Arg Gln Ala Arg Ala Gly
            180                 185                 190

Gly Lys Leu Ala Lys Leu Ala Lys Lys Leu Ala Lys Leu Ala Lys
        195                 200                 205
```

<210> 95
<211> 218
<212> PRT
<213> Artificial Sequence

<220>
<223> -

<400> 95

```
Arg Val Ala Ala His Ile Thr Gly Thr Arg Gly Arg Ser Asn Thr Leu
1               5                   10                  15
```

```
Ser Ser Pro Asn Ser Lys Asn Glu Lys Ala Leu Gly Arg Lys Ile Asn
            20                  25                  30

Ser Trp Glu Ser Ser Arg Ser Gly His Ser Phe Leu Ser Asn Leu His
        35                  40                  45

Leu Arg Asn Gly Glu Leu Val Ile His Glu Lys Gly Phe Tyr Tyr Ile
    50                  55                  60

Tyr Ser Gln Thr Tyr Phe Arg Phe Gln Glu Glu Ile Lys Glu Asn Thr
65                  70                  75                  80

Lys Asn Asp Lys Gln Met Val Gln Tyr Ile Tyr Lys Tyr Thr Ser Tyr
                85                  90                  95

Pro Asp Pro Ile Leu Leu Met Lys Ser Ala Arg Asn Ser Cys Trp Ser
            100                 105                 110

Lys Asp Ala Glu Tyr Gly Leu Tyr Ser Ile Tyr Gln Gly Gly Ile Phe
        115                 120                 125

Glu Leu Lys Glu Asn Asp Arg Ile Phe Val Ser Val Thr Asn Glu His
    130                 135                 140

Leu Ile Asp Met Asp His Glu Ala Ser Phe Phe Gly Ala Phe Leu Val
145                 150                 155                 160

Gly Gly Gly Gly Gly Ser Gly Gly Gly Gly Pro Leu Gly Leu Ala Gly
                165                 170                 175

Arg Val Val Arg Tyr Ala Arg Ala Ala Ala Arg Gln Ala Arg Ala Gly
            180                 185                 190

Gly Arg Trp Gly Lys Trp Phe Lys Lys Ala Thr His Val Gly Lys His
        195                 200                 205

Val Gly Lys Ala Ala Leu Thr Ala Tyr Leu
    210                 215
```

<210> 96
<211> 219
<212> PRT
<213> Artificial Sequence

<220>
<223> -

<400> 96

```
Arg Val Ala Ala His Ile Thr Gly Thr Arg Gly Arg Ser Asn Thr Leu
1               5                   10                  15

Ser Ser Pro Asn Ser Lys Asn Glu Lys Ala Leu Gly Arg Lys Ile Asn
            20                  25                  30

Ser Trp Glu Ser Ser Arg Ser Gly His Ser Phe Leu Ser Asn Leu His
        35                  40                  45

Leu Arg Asn Gly Glu Leu Val Ile His Glu Lys Gly Phe Tyr Tyr Ile
    50                  55                  60

Tyr Ser Gln Thr Tyr Phe Arg Phe Gln Glu Glu Ile Lys Glu Asn Thr
65                  70                  75                  80

Lys Asn Asp Lys Gln Met Val Gln Tyr Ile Tyr Lys Tyr Thr Ser Tyr
                85                  90                  95

Pro Asp Pro Ile Leu Leu Met Lys Ser Ala Arg Asn Ser Cys Trp Ser
            100                 105                 110

Lys Asp Ala Glu Tyr Gly Leu Tyr Ser Ile Tyr Gln Gly Gly Ile Phe
        115                 120                 125

Glu Leu Lys Glu Asn Asp Arg Ile Phe Val Ser Val Thr Asn Glu His
    130                 135                 140

Leu Ile Asp Met Asp His Glu Ala Ser Phe Phe Gly Ala Phe Leu Val
145                 150                 155                 160

Gly Gly Gly Gly Gly Ser Gly Gly Gly Gly Pro Leu Gly Leu Ala Gly
                165                 170                 175

Arg Val Val Arg Tyr Ala Arg Ala Ala Ala Arg Gln Ala Arg Ala Gly
            180                 185                 190
```

```
Gly Gly Arg Arg Lys Arg Lys Trp Leu Arg Arg Ile Gly Lys Gly Val
        195                 200                 205

Lys Ile Ile Gly Gly Ala Ala Leu Asp His Leu
    210                 215
```

<210> 97
<211> 212
<212> PRT
<213> Artificial Sequence

<220>
<223> -

<400> 97

```
Arg Val Ala Ala His Ile Thr Gly Thr Arg Gly Arg Ser Asn Thr Leu
1               5                   10                  15

Ser Ser Pro Asn Ser Lys Asn Glu Lys Ala Leu Gly Arg Lys Ile Asn
            20                  25                  30

Ser Trp Glu Ser Ser Arg Ser Gly His Ser Phe Leu Ser Asn Leu His
        35                  40                  45

Leu Arg Asn Gly Glu Leu Val Ile His Glu Lys Gly Phe Tyr Tyr Ile
    50                  55                  60

Tyr Ser Gln Thr Tyr Phe Arg Phe Gln Glu Glu Ile Lys Glu Asn Thr
65                  70                  75                  80

Lys Asn Asp Lys Gln Met Val Gln Tyr Ile Tyr Lys Tyr Thr Ser Tyr
                85                  90                  95

Pro Asp Pro Ile Leu Leu Met Lys Ser Ala Arg Asn Ser Cys Trp Ser
            100                 105                 110

Lys Asp Ala Glu Tyr Gly Leu Tyr Ser Ile Tyr Gln Gly Gly Ile Phe
        115                 120                 125

Glu Leu Lys Glu Asn Asp Arg Ile Phe Val Ser Val Thr Asn Glu His
    130                 135                 140
```

Leu Ile Asp Met Asp His Glu Ala Ser Phe Phe Gly Ala Phe Leu Val
145 150 155 160

Gly Gly Gly Gly Gly Ser Gly Gly Gly Gly Pro Leu Gly Leu Ala Gly
165 170 175

Arg Val Val Arg Thr His Arg Pro Pro Met Trp Ser Pro Val Trp Pro
180 185 190

Gly Gly Gly Lys Leu Leu Leu Lys Leu Leu Lys Lys Leu Leu Lys Leu
195 200 205

Leu Lys Lys Lys
210

<210> 98
<211> 207
<212> PRT
<213> Artificial Sequence

<220>
<223> -

<400> 98

Arg Val Ala Ala His Ile Thr Gly Thr Arg Gly Arg Ser Asn Thr Leu
1 5 10 15

Ser Ser Pro Asn Ser Lys Asn Glu Lys Ala Leu Gly Arg Lys Ile Asn
20 25 30

Ser Trp Glu Ser Ser Arg Ser Gly His Ser Phe Leu Ser Asn Leu His
35 40 45

Leu Arg Asn Gly Glu Leu Val Ile His Glu Lys Gly Phe Tyr Tyr Ile
50 55 60

Tyr Ser Gln Thr Tyr Phe Arg Phe Gln Glu Glu Ile Lys Glu Asn Thr
65 70 75 80

Lys Asn Asp Lys Gln Met Val Gln Tyr Ile Tyr Lys Tyr Thr Ser Tyr
85 90 95

Pro Asp Pro Ile Leu Leu Met Lys Ser Ala Arg Asn Ser Cys Trp Ser
100 105 110

Lys Asp Ala Glu Tyr Gly Leu Tyr Ser Ile Tyr Gln Gly Gly Ile Phe
115 120 125

Glu Leu Lys Glu Asn Asp Arg Ile Phe Val Ser Val Thr Asn Glu His
130 135 140

Leu Ile Asp Met Asp His Glu Ala Ser Phe Phe Gly Ala Phe Leu Val
145 150 155 160

Gly Gly Gly Gly Gly Ser Gly Gly Gly Gly Pro Leu Gly Leu Ala Gly
165 170 175

Arg Val Val Arg Tyr Gly Arg Lys Lys Arg Arg Gln Arg Arg Arg Gly
180 185 190

Gly Lys Leu Ala Lys Leu Ala Lys Lys Leu Ala Lys Leu Ala Lys
195 200 205

<210> 102
<211> 234
<212> PRT
<213> Artificial Sequence

<220>
<223> -

<400> 102

Arg Val Ala Ala His Ile Thr Gly Thr Arg Gly Arg Ser Asn Thr Leu
1 5 10 15

Ser Ser Pro Asn Ser Lys Asn Glu Lys Ala Leu Gly Arg Lys Ile Asn
20 25 30

Ser Trp Glu Ser Ser Arg Ser Gly His Ser Phe Leu Ser Asn Leu His
35 40 45

Leu Arg Asn Gly Glu Leu Val Ile His Glu Lys Gly Phe Tyr Tyr Ile
50 55 60

```
Tyr Ser Gln Thr Tyr Phe Arg Phe Gln Glu Glu Ile Lys Glu Asn Thr
65                  70                  75                  80

Lys Asn Asp Lys Gln Met Val Gln Tyr Ile Tyr Lys Tyr Thr Ser Tyr
                85                  90                  95

Pro Asp Pro Ile Leu Leu Met Lys Ser Ala Arg Asn Ser Cys Trp Ser
            100                 105                 110

Lys Asp Ala Glu Tyr Gly Leu Tyr Ser Ile Tyr Gln Gly Gly Ile Phe
        115                 120                 125

Glu Leu Lys Glu Asn Asp Arg Ile Phe Val Ser Val Thr Asn Glu His
    130                 135                 140

Leu Ile Asp Met Asp His Glu Ala Ser Phe Phe Gly Ala Phe Leu Val
145                 150                 155                 160

Gly Gly Gly Gly Gly Ser Gly Gly Gly Gly Pro Leu Gly Leu Ala Gly
                165                 170                 175

Arg Val Val Arg Gly Gly Gly Ile Gly Ala Arg Leu Lys Val Leu Thr
            180                 185                 190

Thr Gly Leu Pro Arg Ile Ser Trp Ile Lys Arg Lys Arg Gln Gln Gly
        195                 200                 205

Gly Gly Gly Ser Lys Leu Ala Lys Leu Ala Lys Lys Leu Ala Lys Leu
    210                 215                 220

Ala Lys Arg Arg Arg Arg Arg Arg Arg Arg
225                 230
```

<210> 108
<211> 669
<212> DNA
<213> Artificial Sequence

<220>
<223> -

<400> 108

aaactgctgc tgaaactgct gaaaaaactg ctgaaactgc tgaaaaaaaa aggtggtggt 60
tatggtcgtc cgcgtcagag cggtaaaaaa cgtaaacgca aacgtctgaa accgacccgt 120
gttgttcgtc cgctgggtct ggcaggcggt ggttgtgcag cagcatgtgc agcctgtagc 180
ggtggtcgtg ttgcagcaca tattaccggc acccgtggtc gtagcaatac cctgagcagc 240
ccgaatagca aaaatgaaaa agcactgggt cgcaaaatta acagctggga aagcagccgt 300
agtggtcata gctttctgag caatctgcat ctgcgtaatg gtgaactggt gattcatgaa 360
aaaggcttct actatatcta cagccagacc tattttcgct tccaagaaga gattaaagaa 420
aacaccaaaa acgataaaca aatggtgcag tacatctata aatacaccag ctatccggat 480
ccgattctgc tgatgaaaag cgcacgtaat agctgttgga gcaaagatgc agaatatggc 540
ctgtatagca tttatcaggg tggcatcttt gaactgaaag aaaacgatcg tattttcgtg 600
agcgtgacca atgaacatct gatcgatatg gatcatgaag ccagcttttt tggtgcattt 660
ctggtgggt 669

<210> 109
<211> 669
<212> DNA
<213> Artificial Sequence

<220>
<223> -

<400> 109

aaactggcaa aactggccaa aaaactggct aaactggcga aacgtcgtcg tcgccgtcgt 60
cggcgtaaaa aacgtggtgg tggttgtgca gcagcatgtg cagcctgtac cagcgaagaa 120
accattagca ccgttcaaga aaaacagcag aatattagtc cgctggttcg tgaacgtggt 180
ccgcagcgtg ttgcagcaca tattaccggc acccgtggtc gtagcaatac cctgagcagc 240
ccgaatagca aaaatgaaaa agcactgggt cgcaaaatta acagctggga aagcagccgt 300
agcggtcata gctttctgag caatctgcat ctgcgtaatg gtgaactggt gattcatgaa 360
aaaggcttct actatatcta cagccagacc tattttcgct tccaagaaga gattaaagaa 420
aacaccaaaa acgataaaca aatggtgcag tacatctata aatacaccag ctatccggat 480
ccgattctgc tgatgaaaag cgcacgtaat agctgttgga gcaaagatgc agaatatggc 540
ctgtatagca tttatcaggg tggcatcttt gaactgaaag aaaacgatcg tattttcgtg 600

agcgtgacca atgaacatct gatcgatatg gatcatgaag cgagcttttt tggtgcattt 660

ctggtgggt 669

<210> 110
<211> 696
<212> DNA
<213> Artificial Sequence

<220>
<223> -

<400> 110

aaactggcaa aactggccaa aaaactggct aaactggcga aacgtcgtcg tcgccgtcgt 60

cggcgtaaaa aacgtcatcg tcagccacgt ggttgggaac agggtggtgg ttgtgcagca 120

gcatgtgcag cctgtaccag cgaagaaacc attagcaccg ttcaagaaaa acagcagaat 180

attagtccgc tggttcgtga acgtggtccg cagcgtgttg cagcacatat taccggcacc 240

cgtggtcgta gcaataccct gagcagcccg aatagcaaaa atgaaaaagc actgggtcgc 300

aaaattaaca gctgggaaag cagccgtagc ggtcatagct ttctgagcaa tctgcatctg 360

cgtaatggtg aactggtgat tcatgaaaaa ggcttctact atatctacag ccagacctat 420

tttcgcttcc aagaagagat taaagaaaac accaaaaacg ataaacaaat ggtgcagtac 480

atctataaat acaccagcta tccggatccg attctgctga tgaaaagcgc acgtaatagc 540

tgttggagca aagatgcaga atatggcctg tatagcattt atcagggtgg catctttgaa 600

ctgaaagaaa acgatcgtat tttcgtgagc gtgaccaatg aacatctgat cgatatggat 660

catgaagcga gctttttttgg tgcatttctg gtgggt 696

<210> 111
<211> 621
<212> DNA
<213> Artificial Sequence

<220>
<223> -

<400> 111

cgtgtggcgg cgcatattac cggcacccgt ggccgtagca acaccctgag cagcccgaac 60

agcaaaaacg aaaaagcgct gggccgtaaa attaacagct gggaaagcag ccgtagcggc 120

catagctttc tgagcaacct gcatctgcgt aacggcgaac tggtgattca tgaaaaaggc 180

ttttattata tttatagcca gacctatttt cgttttcagg aagaaattaa agaaaacacc 240

aaaaacgata aacagatggt gcagtatatt tataaatata ccagctatcc ggatccgatt 300

ctgctgatga aaagcgcgcg taacagctgc tggagcaaag atgcggaata tggcctgtat 360

agcatttatc agggcggcat ttttgaactg aaagaaaacg atcgtatttt tgtgagcgtg 420

accaacgaac atctgattga tatggatcat gaagcgagct tttttggcgc gtttctggtg 480

ggcggcggcg gcggcagcgg cggcggcggc ccgctgggcc tggcgggccg tgtggtgcgt 540

tatgcgcgtg cggcggcgcg tcaggcgcgt gcgggcggca aactggcgaa actggcgaaa 600

aaactggcga aactggcgaa a 621

<210> 112
<211> 654
<212> DNA
<213> Artificial Sequence

<220>
<223> -

<400> 112

cgtgtggcgg cgcatattac cggcacccgt ggccgtagca acaccctgag cagcccgaac 60

agcaaaaacg aaaaagcgct gggccgtaaa attaacagct gggaaagcag ccgtagcggc 120

catagctttc tgagcaacct gcatctgcgt aacggcgaac tggtgattca tgaaaaaggc 180

ttttattata tttatagcca gacctatttt cgttttcagg aagaaattaa agaaaacacc 240

aaaaacgata aacagatggt gcagtatatt tataaatata ccagctatcc ggatccgatt 300

ctgctgatga aaagcgcgcg taacagctgc tggagcaaag atgcggaata tggcctgtat 360

agcatttatc agggcggcat ttttgaactg aaagaaaacg atcgtatttt tgtgagcgtg 420

accaacgaac atctgattga tatggatcat gaagcgagct tttttggcgc gtttctggtg 480

ggcggcggcg gcggcagcgg cggcggcggc ccgctgggcc tggcgggccg tgtggtgcgt 540

tatgcgcgtg cggcggcgcg tcaggcgcgt gcgggcggcc gttggggcaa atggtttaaa 600

aaagcgaccc atgtgggcaa acatgtgggc aaagcggcgc tgaccgcgta tctg 654

<210> 113
<211> 657
<212> DNA
<213> Artificial Sequence

<220>

<223> -

<400> 113

```
cgtgtggcgg cgcatattac cggcacccgt ggccgtagca acaccctgag cagcccgaac      60
agcaaaaacg aaaaagcgct gggccgtaaa attaacagct gggaaagcag ccgtagcggc     120
catagctttc tgagcaacct gcatctgcgt aacggcgaac tggtgattca tgaaaaaggc     180
ttttattata tttatagcca gacctatttt cgttttcagg aagaaattaa agaaaacacc     240
aaaaacgata aacagatggt gcagtatatt tataaatata ccagctatcc ggatccgatt     300
ctgctgatga aaagcgcgcg taacagctgc tggagcaaag atgcggaata tggcctgtat     360
agcatttatc agggcggcat ttttgaactg aaagaaaacg atcgtatttt tgtgagcgtg     420
accaacgaac atctgattga tatggatcat gaagcgagct tttttggcgc gtttctggtg     480
ggcggcggcg gcggcagcgg cggcggcggc ccgctgggcc tggcgggccg tgtggtgcgt     540
tatgcgcgtg cggcggcgcg tcaggcgcgt gcgggcggcg gccgtcgtaa acgtaaatgg     600
ctgcgtcgta ttggcaaagg cgtgaaaatt attggcggcg cggcgctgga tcatctg        657
```

<210> 114
<211> 636
<212> DNA
<213> Artificial Sequence

<220>
<223> -

<400> 114

```
cgtgtggcgg cgcatattac cggcacccgt ggccgtagca acaccctgag cagcccgaac      60
agcaaaaacg aaaaagcgct gggccgtaaa attaacagct gggaaagcag ccgtagcggc     120
catagctttc tgagcaacct gcatctgcgt aacggcgaac tggtgattca tgaaaaaggc     180
ttttattata tttatagcca gacctatttt cgttttcagg aagaaattaa agaaaacacc     240
aaaaacgata aacagatggt gcagtatatt tataaatata ccagctatcc ggatccgatt     300
ctgctgatga aaagcgcgcg taacagctgc tggagcaaag atgcggaata tggcctgtat     360
agcatttatc agggcggcat ttttgaactg aaagaaaacg atcgtatttt tgtgagcgtg     420
accaacgaac atctgattga tatggatcat gaagcgagct tttttggcgc gtttctggtg     480
ggcggcggcg gcggcagcgg cggcggcggc ccgctgggcc tggcgggccg tgtggtgcgt     540
```

acccatcgtc cgccgatgtg gagcccggtg tggccgggcg gcggcaaact gctgctgaaa 600

ctgctgaaaa aactgctgaa actgctgaaa aaaaaa 636

<210> 115
<211> 621
<212> DNA
<213> Artificial Sequence

<220>
<223> -

<400> 115

cgcgtggcgg cgcatattac cggcacccgc ggccgcagca acaccctgag cagcccgaac 60

agcaaaaacg aaaaagcgct gggccgcaaa attaacagct gggaaagcag ccgcagcggc 120

catagctttc tgagcaacct gcatctgcgc aacggcgaac tggtgattca tgaaaaaggc 180

ttttattata tttatagcca gacctatttt cgctttcagg aagaaattaa agaaaacacc 240

aaaaacgata aacagatggt gcagtatatt tataaatata ccagctatcc ggatccgatt 300

ctgctgatga aaagcgcgcg caacagctgc tggagcaaag atgcggaata tggcctgtat 360

agcatttatc agggcggcat ttttgaactg aaagaaaacg atcgcatttt tgtgagcgtg 420

accaacgaac atctgattga tatggatcat gaagcgagct tttttggcgc gtttctggtg 480

ggcggcggcg gcggcagcgg cggcggcggc ccgctgggcc tggcgggccg cgtggtgcgc 540

tatggccgca aaaaacgccg ccagcgccgc cgcggcggca aactggcgaa actggcgaaa 600

aaactggcga aactggcgaa a 621

<210> 119
<211> 702
<212> DNA
<213> Artificial Sequence

<220>
<223> -

<400> 119

cgtgttgcag cacatattac cggcacccgt ggtcgtagca ataccctgag cagcccgaat 60

agcaaaaatg aaaaagcact gggtcgcaaa attaacagct gggaaagcag ccgtagcggt 120

catagctttc tgagcaatct gcatctgcgt aatggtgaac tggtgattca tgaaaaaggc 180

ttctactata tctacagcca gacctatttt cgcttccaag aagagattaa agaaaacacc 240

```
aaaaacgata aacaaatggt gcagtacatc tataaataca ccagctatcc ggatccgatt      300

ctgctgatga aaagcgcacg taatagctgt tggagcaaag atgcagaata tggcctgtat      360

agcatttatc agggtggcat ctttgaactg aaagaaaacg atcgtatttt cgtgagcgtg      420

accaatgaac atctgatcga tatggatcat gaagccagct tttttggtgc atttctggtg      480

ggtggtggtg gcggtagtgg cggtggtggt cctctgggtc tggcaggtcg tgttgttcgc      540

ggaggtggta ttggtgcacg tctgaaagtt ctgaccaccg gtctgcctcg tattagctgg      600

attaaacgta aacgtcagca gggtgggggt ggtagcaaac tggcaaaact ggcgaaaaaa      660

ctggctaaac tggccaaacg tcgtcgtcgc cgtcgtcggc gt                         702
```

<210> 125
<211> 39
<212> PRT
<213> Artificial Sequence

<220>
<223> -

<400> 125

```
Lys Leu Leu Leu Lys Leu Leu Lys Lys Leu Leu Lys Leu Leu Lys Lys
1               5                   10                  15

Lys Gly Gly Gly Tyr Gly Arg Pro Arg Gln Ser Gly Lys Lys Arg Lys
            20                  25                  30

Arg Lys Arg Leu Lys Pro Thr
        35
```

<210> 126
<211> 25
<212> PRT
<213> Pleuronectes americanus

<300>
<301> Hilchie AL, Doucette CD, Pinto DM, Patrzykat A, Douglas S, Hoskin DW. <302> Pleurocidin-family cationic antimicrobial peptides are cytolytic for breast carcinoma cells and prevent growth of tumor xenografts <303> Breast Cancer Res.<304> 13<305> 5<306> R102<307> 2011-10-24

<400> 126

```
Arg Trp Gly Lys Trp Phe Lys Lys Ala Thr His Val Gly Lys His Val
1               5                   10                  15

Gly Lys Ala Ala Leu Thr Ala Tyr Leu
            20                  25
```

<210> 127
<211> 26
<212> PRT
<213> Pleuronectes americanus

<300>
<301> Hilchie AL, Doucette CD, Pinto DM, Patrzykat A, Douglas S, Hoskin DW. <302> Pleurocidin-family cationic antimicrobial peptides are cytolytic for breast carcinoma cells and prevent growth of tumor xenografts.<303> Breast Cancer Res.<304> 13<305> 5<306> R102<307> 2011-10-24

<400> 127

```
Gly Arg Arg Lys Arg Lys Trp Leu Arg Arg Ile Gly Lys Gly Val Lys
1               5                   10                  15

Ile Ile Gly Gly Ala Ala Leu Asp His Leu
            20                  25
```

<210> 128
<211> 17
<212> PRT
<213> Artificial Sequence

<220>
<223> -

<300>
<301> Papo N, Shai Y. <302> New lytic peptides based on the D,L-amphipathic helix motif preferentially kill tumor cells compared to normal cells.<303> Biochemistry<304> 42<305> 31<306> 9346-54<307> 2003-08-12

<400> 128

```
Lys Leu Leu Leu Lys Leu Leu Lys Lys Leu Leu Lys Leu Leu Lys Lys
1               5                   10                  15

Lys
```